# EUROPEAN PATENT APPLICATION

(11) **EP 4 625 046 A2**
(43) Date of publication of application: **01.10.2025**
(21) Application number: 25165259.0
(22) Date of filing: 21.03.2025
(51) Int. Cl.: G03F 7/09

(54) **COMPOSITION FOR FORMING ORGANIC FILM, METHOD FOR FORMING ORGANIC FILM, PATTERNING PROCESS, MONOMER, AND POLYMER**

(30) Priority: 25.03.2024 JP 2024048143
(71) Applicant: Shin-Etsu Chemical Co., Ltd., Tokyo 100-0005 (JP)
(72) Inventor: KORI, Daisuke, Niigata (JP); YAMAMOTO, Yasuyuki, Niigata (JP); HATAKEYAMA, Jun, Niigata (JP)
(74) Representative: Röthinger, Rainer

(57) **Abstract**

The present invention is a composition for forming an organic film, containing: a resin and/or compound (A) for forming an organic film; a polymer (B) having a repeating unit represented by the following general formula (1) and/or (2); and a solvent (C). This can provide: a composition for forming an organic film which is excellent in film-formability (in-plane uniformity) on a substrate (wafer) and filling property, can suppress humps in an EBR process, and allows an excellent process margin when used for an organic underlayer film for a multilayer resist; a method for forming an organic film, using the composition; a patterning process using the composition; a monomer; and a polymer.

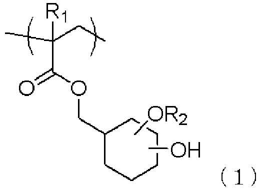

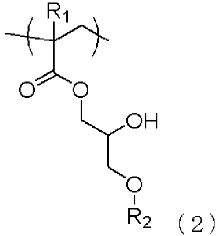

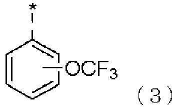

## Description

### TECHNICAL FIELD

The present invention relates to: a composition for forming an organic film for a multilayer resist for fine processing in manufacturing a semiconductor device etc. or an organic film for planarization in manufacturing a semiconductor device etc.; a method for forming an organic film by using the composition; a patterning process using the composition; a monomer; and a polymer.

### BACKGROUND ART

Recently, along with advancements toward higher integration and higher processing speed of semiconductor devices, a finer pattern rule has been required. In this situation, various techniques have been developed in regard to how patterning process can be performed more finely and precisely depending on light sources used in lithography with light exposure, which is a commonly-employed technique at present.

As the light source for lithography employed in resist pattern formation, light exposure using a g-line (436 nm) or an i-line (365 nm) of a mercury lamp as a light source is widely adopted for portions where the degree of integration is low. Meanwhile, for portions where the degree of integration is high and finer patterning is required, lithography using a KrF excimer laser (248 nm) or an ArF excimer laser (193 nm) with shorter wavelengths has also been practically used. Moreover, for the most-advanced generation requiring further finer patterning, lithography with extreme ultraviolet ray (EUV, 13.5 nm) is about to be put to practical use.

It is well known that in a monolayer resist method, which is employed as a typical resist patterning process, as the thinning of resist patterns progresses as described above, the ratio of a pattern height to a pattern line width (aspect ratio) is increased, and pattern collapse occurs due to the surface tension of a developer during development. It is known that, in this situation, a multilayer resist method, in which a pattern is formed by laminating films having different dry etching properties, is excellent in forming a pattern with a high aspect ratio on an uneven substrate. There have been developed: a two-layer resist method in which a photoresist layer made of a silicon-containing photosensitive polymer is combined with an underlayer made of an organic polymer containing carbon, hydrogen, and oxygen as main constituent elements, for example, a novolak polymer (Patent Document 1); and a three-layer resist method in which a photoresist layer made of an organic photosensitive polymer used in a monolayer resist method is combined with a middle layer made of a silicon-based polymer or a silicon-based CVD film, and an underlayer made of an organic polymer (Patent Document 2).

In the three-layer resist method, for example, an organic film made of a novolak or the like is formed uniformly as a resist underlayer film on a substrate to be processed; a silicon-containing film is formed thereon as a resist middle layer film; and an ordinary organic photoresist film is formed thereon as a resist upper layer film. In dry etching using a fluorine-based gas plasma, an organic resist upper layer film has favorable etching selectivity to a silicon-containing resist middle layer film, and therefore, a resist pattern is transferred to the silicon-containing resist middle layer film by dry etching using a fluorine-based gas plasma. According to this method, the pattern can be transferred to the silicon-containing film even when using a resist composition that causes difficulties in forming a pattern having a sufficient film thickness for directly processing the substrate to be processed or when using a resist composition that does not have sufficient dry etching resistance for processing the substrate. In addition, by subsequently transferring the pattern by dry etching with an oxygen-based gas plasma, it is possible to obtain a pattern in a novolak film having sufficient dry etching resistance for processing.

Many techniques are already known (e.g. Patent Document 3) regarding organic underlayer films like the organic underlayer film described above. However, in association with recent progress in miniaturization, the need for excellent filling property in addition to dry etching property is rising. There is a demand for an organic underlayer film material that enables uniform film formation even on an underlying substrate to be processed having a complex form or any material, and that has a filling property that allows a required pattern to be filled without gaps.

When a semiconductor substrate or the like is manufactured, the above-described organic underlayer film is formed using a coater/developer that can perform treatments such as spin-coating process, EBR process, and baking process. The EBR (Edge Bead Removal) process is a process of removing, after forming a film on a substrate (wafer) by spin-coating, the film on the edge of the substrate with a remover, for the purpose of preventing the contamination of a substrate-conveying arm of the coater/developer. A mixed solution of propylene glycol monomethyl ether acetate and propylene glycol monomethyl ether (30 mass% :70 mass%) is a remover used in EBR processes, and such removers are widely used in EBR processes of resist films and resist underlayer films (silicon-containing middle layer films and organic underlayer films).

Due to the effect of a remover in an EBR process, a state where a peripheral portion of an organic underlayer film has a thick film thickness (a hump) occurs in some cases. In the above-described dry etching step at the time of substrate processing, a hump causes defects, and therefore, an organic underlayer film in which a hump is suppressed is desired.

Furthermore, after forming the spin-coating film, the organic underlayer film is baked to form a cured film in order to use in a multilayer resist process. This is because it is necessary to achieve an insoluble and infusible organic underlayer film for applying a silicon-containing resist middle layer film thereon. On the surface of an organic film formed by baking, a hydrophobic surface caused by a surfactant contained in the composition for forming an organic film is formed, and induces coating abnormality of the silicon-containing resist middle layer film in some cases. To improve the coating property of the silicon-containing resist middle layer film and widen the process margin, the contact angle of the surface of the organic underlayer film is required to be controlled.

As stated above, surfactants greatly contribute to the guarantee of film-formability of materials for forming an organic film. A surfactant containing, for exerting the surface active effect of the surfactant sufficiently, an organic group substituted with a fluorine atom exemplified by a perfluoroalkyl group is generally applied. However, in recent years, the effect of this perfluoroalkyl compound (PFAS) on health has been pointed out, and there is a movement in the European REACH to restrict the production and sale of PFAS compounds. A demand for the development of a surfactant not having a PFAS structure has become urgent.

As examples of such a surfactant, surfactants having a trifluoromethoxy group or a pentafluorosulfanyl group and the use of the surfactants are proposed (Patent Document 4).

### CITATION LIST

### PATENT LITERATURE

Patent Document 1: JP H6-118651 A
Patent Document 2: JP 4355943 B2
Patent Document 3: JP 2004-205685 A
Patent Document 4: JP 2008-526792 A

### SUMMARY OF INVENTION

### TECHNICAL PROBLEM

The present invention has been made in view of the above-described circumstances. An object of the present invention is to provide: a composition for forming an organic film which is excellent in film-formability (in-plane uniformity) on a substrate (wafer) and filling property, can suppress humps in an EBR process, and allows an excellent process margin when used for an organic underlayer film for a multilayer resist; a method for forming an organic film, using the composition; a patterning process using the composition; a monomer; and a polymer.

### SOLUTION TO PROBLEM

To achieve the object, the present invention provides a composition for forming an organic film, comprising: a resin and/or compound (A) for forming an organic film; a polymer (B) having a repeating unit represented by the following general formula (1) and/or (2); and a solvent (C), 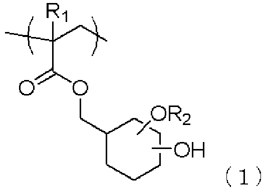 wherein R₁ represents a hydrogen atom or a methyl group, R₂ represents a saturated or unsaturated monovalent organic group having 7 to 50 carbon atoms and at least has at least one fluorine-containing structure represented by the following formula (3), and the substituents OR₂ and OH group on the cyclohexane ring are on adjacent carbon atoms on the cyclohexane ring, 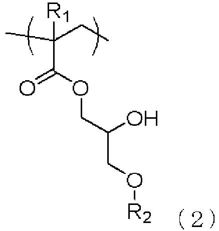 wherein R₁ and R₂ are as defined above, 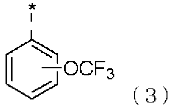 wherein (3) is a partial structure contained in the R₂, "*" represents an attachment point in a structure in the R₂, and two or more kinds of structures represented by (3) or two or more identical structures represented by (3) are optionally contained in the R₂.

A composition for forming an organic film containing a polymer containing such a partial structure has a suitable fluorine structure and a suitable polar structure, that is, has a trifluoromethoxy group on an aromatic ring and has, as a substituent, an OH group on a carbon atom adjacent to the carbon atom to which the OR₂ is bonded, thus having both a hydrophilic group and a hydrophobic group introduced, and therefore, it is possible to impart the surface active effect necessary when forming an organic film and film-formability at the time of application can be improved. Note that the trifluoromethoxybenzene structure represented by the R₂ of the present invention does not belong to the category of PFAS in REACH, and therefore, is also advantageous from the viewpoint of preventing environmental pollution and can be expected to be a highly versatile material as a surfactant of an organic film.

The R₂ in the general formulae (1) and (2) preferably contains a partial structure represented by one of the following general formulae (4), 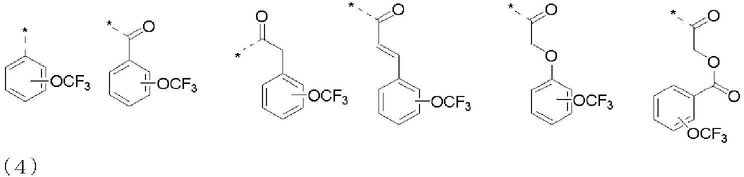 wherein "*" represents an attachment point.

In a composition for forming an organic film containing a polymer having such a structure, the trifluoromethoxybenzene structure is not directly bonded to the main chain but is introduced in a position separated from the main chain by a linker of a few carbon atoms. It is conjectured that, thus, an agglomerate structure is easily formed by the interaction between the trifluoromethoxy group and the benzene ring and behavior as a hydrophobic group is emphasized, and as a result, an excellent surface active effect is exhibited. Therefore, the material for forming an organic film of the present invention can provide a material for forming an organic film that has excellent film-formability and can be applied to various polymers and compounds.

The polymer (B) is preferably a copolymer having repeating units of one or both of the general formulae (1) and (2) and the following general formula (5), 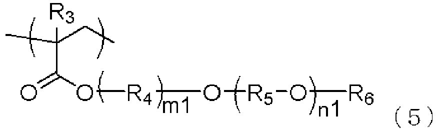 wherein R₃ represents a hydrogen atom or a methyl group; R₄ and R₅ each represent a linear or branched divalent alkylene group having 1 to 4 carbon atoms; R₆ represents a hydrogen atom, an alkyl group having 1 to 4 carbon atoms, or a phenyl group; "m1" represents 0 to 23; "n1" represents 0 to 23; and "m1" and "n1" satisfy 2 ≤ m1+n1 ≤ 23.

As stated above, a material for forming an organic film excellent in film-formability can be achieved, and when the polymer is a copolymer containing a repeating unit having a linking group like that shown in (5), the repeating unit of (5) contained contributes as an appropriate alleviation unit of the agglomerate structure, and thus, the surface activating ability can be adjusted. Therefore, it is possible to provide a material for forming an organic film adapted to various film thicknesses (not dependent on the solution concentration) and having both filling property and film-formability.

The polymer (B) preferably has a weight-average molecular weight of 1500 to 30000.

When the weight-average molecular weight is in such a range, it is possible to form an organic film excellent in film-formability and filling property. Furthermore, the contact angle on the surface of a film after film formation can be controlled to be in an appropriate range, and it is possible to form an organic underlayer film suitable for use in a multilayer resist process.

The polymer (B) is preferably contained in an amount of 0.01 parts by mass to 5 parts by mass based on 100 parts by mass of the resin and/or compound (A) for forming an organic film.

When the composition for forming an organic film contains the polymer in such an amount, better in-plane uniformity of a formed organic film can be achieved, and therefore, such a composition is preferable.

The present invention also provides a method for forming an organic film to be used in a manufacturing process of a semiconductor device, the method comprising:
spin-coating a substrate to be processed with the above-described composition for forming an organic film; and
forming an organic film by heating the substrate to be processed coated with the composition for forming an organic film at a temperature of 100°C or higher and 600°C or lower for 10 to 600 seconds for curing.

The inventive composition for forming an organic film can fill, by spin-coating, a pattern having a complicated shape on a substrate to be processed, makes it possible to form an organic film having excellent in-plane uniformity, and is particularly useful when an edge portion of an organic film is to be removed while suppressing humps in an EBR process.

The present invention also provides a patterning process comprising:
forming an organic film on a body to be processed by using the above-described composition for forming an organic film;
forming a silicon-containing resist middle layer film on the organic film by using a silicon-containing resist middle layer film material;
forming a resist upper layer film on the silicon-containing resist middle layer film by using a resist upper layer film material comprising a photoresist composition;
forming a circuit pattern in the resist upper layer film;
transferring the pattern to the silicon-containing resist middle layer film by etching while using the resist upper layer film having the formed circuit pattern as a mask;
transferring the pattern to the organic film by etching while using the silicon-containing resist middle layer film having the transferred pattern as a mask; and
further transferring the pattern to the body to be processed by etching while using the organic film having the transferred pattern as a mask.

The present invention also provides a patterning process comprising:
forming an organic film on a body to be processed by using the above-described composition for forming an organic film;
forming a silicon-containing resist middle layer film on the organic film by using a silicon-containing resist middle layer film material;
forming an organic antireflective film or an adhesive film on the silicon-containing resist middle layer film;
forming a resist upper layer film on the organic antireflective film or the adhesive film by using a resist upper layer film material comprising a photoresist composition;
forming a circuit pattern in the resist upper layer film;
transferring the pattern to the organic antireflective film or the adhesive film and to the silicon-containing resist middle layer film by etching while using the resist upper layer film having the formed circuit pattern as a mask;
transferring the pattern to the organic film by etching while using the silicon-containing resist middle layer film having the transferred pattern as a mask; and
further transferring the pattern to the body to be processed by etching while using the organic film having the transferred pattern as a mask.

The present invention also provides a patterning process comprising:
forming an organic film on a body to be processed by using the above-described composition for forming an organic film;
forming an inorganic hard mask middle layer film selected from the group consisting of a silicon oxide film, a silicon nitride film, and a silicon oxynitride film on the organic film;
forming a resist upper layer film on the inorganic hard mask middle layer film by using a resist upper layer film material comprising a photoresist composition;
forming a circuit pattern in the resist upper layer film;
transferring the pattern to the inorganic hard mask middle layer film by etching while using the resist upper layer film having the formed circuit pattern as a mask;
transferring the pattern to the organic film by etching while using the inorganic hard mask middle layer film having the transferred pattern as a mask; and
further transferring the pattern to the body to be processed by etching while using the organic film having the transferred pattern as a mask.

The present invention also provides a patterning process comprising:
forming an organic film on a body to be processed by using the above-described composition for forming an organic film;
forming an inorganic hard mask middle layer film selected from the group consisting of a silicon oxide film, a silicon nitride film, and a silicon oxynitride film on the organic film;
forming an organic antireflective film or an adhesive film on the inorganic hard mask middle layer film;
forming a resist upper layer film on the organic antireflective film or the adhesive film by using a resist upper layer film material comprising a photoresist composition;
forming a circuit pattern in the resist upper layer film;
transferring the pattern to the organic antireflective film or the adhesive film and to the inorganic hard mask middle layer film by etching while using the resist upper layer film having the formed circuit pattern as a mask;
transferring the pattern to the organic film by etching while using the inorganic hard mask middle layer film having the transferred pattern as a mask; and
further transferring the pattern to the body to be processed by etching while using the organic film having the transferred pattern as a mask.

As described, the inventive composition for forming an organic film can be used suitably in various patterning processes such as a three-layer resist process using a silicon-containing resist middle layer film or an inorganic hard mask middle layer film and a four-layer resist process additionally using an organic antireflective film. According to such patterning processes of the present invention, it is possible to transfer and form a circuit pattern of a resist upper layer film in a body to be processed with high accuracy.

The inorganic hard mask middle layer film is preferably formed by a CVD method or an ALD method.

In the inventive patterning process, the inorganic hard mask middle layer film can be formed by such methods, for example.

The circuit pattern is preferably formed by a lithography using light having a wavelength of 10 nm or more and 300 nm or less, a direct writing with electron beam, nanoimprinting, or a combination thereof.

Furthermore, the circuit pattern is preferably developed with an alkaline development or an organic solvent.

In the inventive patterning process, such means of forming and developing the circuit pattern can be used suitably.

The body to be processed is preferably a semiconductor device substrate or the semiconductor device substrate coated with any of a metal film, a metal carbide film, a metal oxide film, a metal nitride film, a metal oxycarbide film, and a metal oxynitride film.

The metal constituting the body to be processed is preferably silicon, titanium, tungsten, hafnium, zirconium, chromium, germanium, copper, silver, gold, aluminum, indium, gallium, arsenic, palladium, iron, tantalum, iridium, molybdenum, or an alloy thereof.

According to the inventive patterning process, such a body to be processed can be processed to form a pattern.

The present invention also provides a monomer represented by the following general formula (6) or (7), 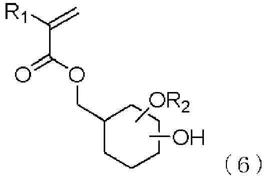 wherein R₁ represents a hydrogen atom or a methyl group, R₂ represents a saturated or unsaturated monovalent organic group having 7 to 50 carbon atoms and at least has at least one fluorine-containing structure represented by the following formula (3), and the substituents OR₂ and OH group on the cyclohexane ring are on adjacent carbon atoms on the cyclohexane ring, 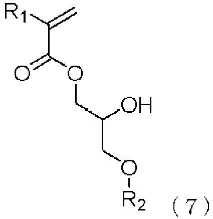 wherein R₁ and R₂ are as defined above, 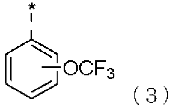 wherein (3) is a partial structure contained in the R₂, "*" represents an attachment point in a structure in the R₂, and two or more kinds of structures represented by (3) or two or more identical structures represented by (3) are optionally contained in the R₂.

Such a monomer having trifluoromethoxybenzene in a terminal structure and having, between a polymerizable functional group and the trifluoromethoxybenzene, a linker having a hydroxy group structure is useful when manufacturing a polymer for a surfactant. Furthermore, it is also possible to reduce environmental load, and the monomer can be used not only for a surfactant but also as a candidate for an industrially useful monomer.

The R₂ in the general formulae (6) and (7) preferably contains a partial structure represented by one of the following general formulae (4), 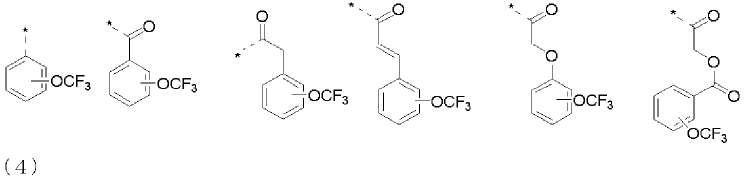 wherein "*" represents an attachment point.

When the structure is as described above, the monomer can be used as a monomer capable of controlling a high surface active effect by making use of the interaction between the trifluoromethoxy group and the benzene ring.

The present invention also provides a polymer having a repeating unit represented by the following general formula (1) and/or (2), 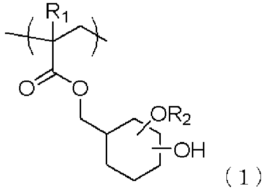 wherein R₁ represents a hydrogen atom or a methyl group, R₂ represents a saturated or unsaturated monovalent organic group having 7 to 50 carbon atoms and at least has at least one fluorine-containing structure represented by the following formula (3), and the substituents OR₂ and OH group on the cyclohexane ring are on adjacent carbon atoms on the cyclohexane ring, 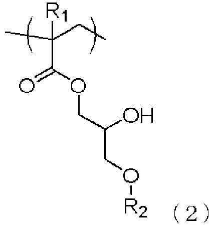 wherein R₁ and R₂ are as defined above, 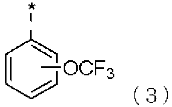 wherein (3) is a partial structure contained in the R₂, "*" represents an attachment point in a structure in the R₂, and two or more kinds of structures represented by (3) or two or more identical structures represented by (3) are optionally contained in the R₂.

The above-described polymer has an appropriate linking group and terminal structure in the side chain, and therefore, the polymer is not only useful for a surfactant of a composition for forming an organic film, but can also be expected to be an environment-conscious material.

The R₂ in the general formulae (1) and (2) preferably contains a partial structure represented by one of the following general formulae (4), 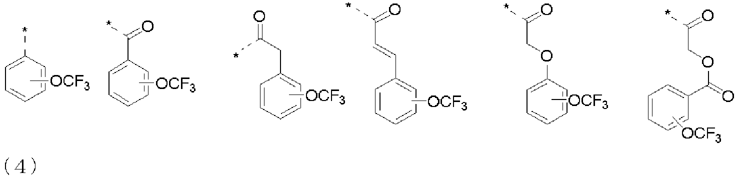 wherein "*" represents an attachment point.

When the polymer has a partial structure shown above, the polymer is useful for a surfactant that can realize high film-formability.

The polymer is preferably a copolymer having repeating units of one or both of the general formulae (1) and (2) and the following general formula (5), 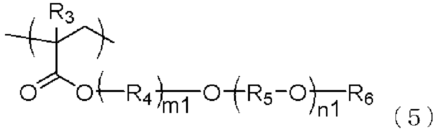 wherein R₃ represents a hydrogen atom or a methyl group; R₄ and R₅ each represent a linear or branched divalent alkylene group having 1 to 4 carbon atoms; R₆ represents a hydrogen atom, an alkyl group having 1 to 4 carbon atoms, or a phenyl group; "m1" represents 0 to 23; "n1" represents 0 to 23; and "m1" and "n1" satisfy 2 ≤ m1+n1 ≤ 23.

When the polymer is a copolymer having the above-described partial structure, the polymer can be used as a surfactant that makes it possible to form a film without various kinds of dependence on film thickness.

The polymer preferably has a weight-average molecular weight of 1500 to 30000.

When the weight-average molecular weight is in the above-described range, the polymer is suitable as a surfactant that can realize film-formability with an excellent process margin.

### ADVANTAGEOUS EFFECTS OF INVENTION

As described above, according to the present invention, it is possible to provide a composition for forming an organic film having excellent film-formability (in-plane uniformity) on a substrate (wafer) and excellent filling property, having film-formability on the organic film when used for an organic underlayer film, and in which humps are suppressed at the time of an EBR process. Furthermore, by providing a copolymer having a combination of particular units, application to various materials for forming an organic film can be realized. In particular, the inventive composition for forming an organic film is excellent in film-formability, filling property, and the property of suppressing humps that are generated during an EBR process, and therefore, is extremely useful as: for example, an organic film material used in multilayer resist processes, such as a two-layer resist process, a three-layer resist process using a silicon-containing resist middle layer film or an inorganic hard mask middle layer film, or a four-layer resist process using a silicon-containing resist middle layer film or an inorganic hard mask middle layer film and an organic antireflective film; or a material for forming a film for manufacturing a semiconductor device, such as a photoresist material and a silicon-containing resist middle layer film material. Thus, according to the inventive method for forming an organic film, it is possible to form an organic film in which humps have been suppressed, and therefore, semiconductor devices and so forth can be manufactured efficiently. In addition, the present invention can provide a monomer and a polymer that are useful as surfactants necessary for realizing high film-formability.

### BRIEF DESCRIPTION OF DRAWINGS

FIG. 1 is an example of a graph on measuring the height of a hump by using a stylus profiler in the case of a composition for forming an organic film in which humps have been suppressed.
FIG. 2 is an example of a graph on measuring the height of a hump by using a stylus profiler in the case of a composition for forming an organic film in which humps have not been suppressed.
FIG. 3 is an explanatory diagram of an example of the inventive patterning process according to a three-layer resist process.
FIG. 4 is an explanatory diagram of a filling property evaluation method in the Examples.

### DESCRIPTION OF EMBODIMENTS

As described above, in particular, there has been required a material having a surface active effect that not only provides film-formability as an organic film material but also has a low environmental load. In addition, there has been required the development of a composition for forming an organic film that has excellent film-formability (in-plane uniformity) on a substrate (wafer) when the organic film material is used as an organic underlayer film material and excellent filling property, and that can suppress humps at the time of an EBR process.

Usually, when an organic film is to be formed, a resin for forming an organic film and additives are dissolved in an organic solvent to form a composition, the composition is applied with a coater and a developer onto a substrate on which a structure, a circuit, etc. is formed, the composition is spread by the substrate rotating, the composition on the edge is removed in an EBR process, and then the composition is baked to form an organic film.

It is thought that if the surface active effect of the composition is insufficient, gaps are generated when a hole or trench having a very high aspect ratio is filled due to uneven distribution of the surfactant in the film occurring, and in addition, a hump is generated in the periphery of the organic film if the resin for forming an organic film or the additives have poor solubility in the remover used in the EBR process.

The present inventors have studied earnestly the above-described problems and found out that when a polymer having a repeating unit having a particular substituent is contained in a composition for forming an organic film, the composition has excellent film-formability and high filling property and is excellent in hump suppression during an EBR process. Thus, the present invention has been completed. Furthermore, by reducing environmental load by applying trifluoromethoxybenzene, development not only as a surfactant but also as an industrially useful monomer and polymer can also be expected.

That is, the present invention is a composition for forming an organic film, comprising: a resin and/or compound (A) for forming an organic film; a polymer (B) having a repeating unit represented by the following general formula (1) and/or (2); and a solvent (C), 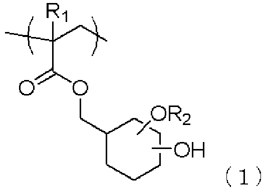 wherein R₁ represents a hydrogen atom or a methyl group, R₂ represents a saturated or unsaturated monovalent organic group having 7 to 50 carbon atoms and at least has at least one fluorine-containing structure represented by the following formula (3), and the substituents OR₂ and OH group on the cyclohexane ring are on adjacent carbon atoms on the cyclohexane ring, 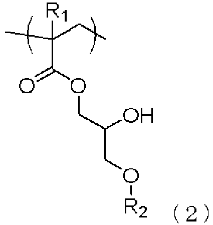 wherein R₁ and R₂ are as defined above, 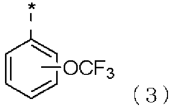 wherein (3) is a partial structure contained in the R₂, "*" represents an attachment point in a structure in the R₂, and two or more kinds of structures represented by (3) or two or more identical structures represented by (3) are optionally contained in the R₂.

Hereinafter, the present invention will be described in detail, but the present invention is not limited thereto.

### [Monomer]

The monomer to be used for manufacturing the inventive polymer for forming an organic film is represented by the following general formula (6) or (7).

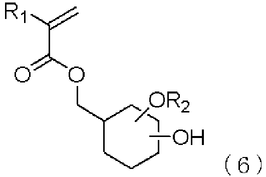

In the formula (6), R₁ represents a hydrogen atom or a methyl group, R₂ represents a saturated or unsaturated monovalent organic group having 7 to 50 carbon atoms and at least has at least one fluorine-containing structure represented by the following formula (3), and the substituents OR₂ and OH group on the cyclohexane ring are on adjacent carbon atoms on the cyclohexane ring.

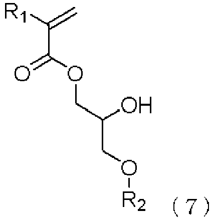

In the formula (7), R₁ and R₂ are as defined above. 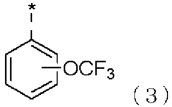 (3) is a partial structure contained in the R₂, "*" represents an attachment point in a structure in the R₂, and two or more kinds of structures represented by (3) or two or more identical structures represented by (3) are optionally contained in the R₂.

Preferable examples of the monomer represented by the general formula (6) include the following.

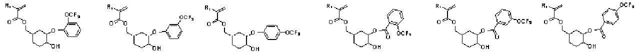

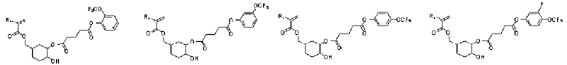

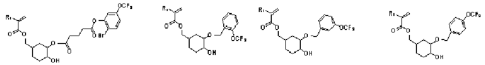

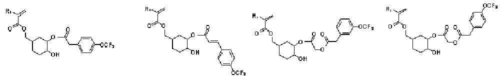

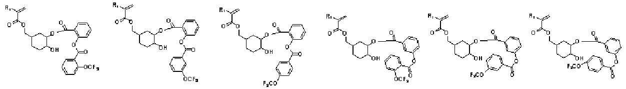

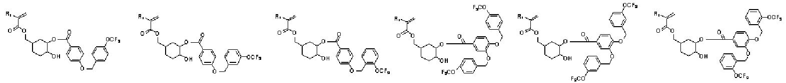

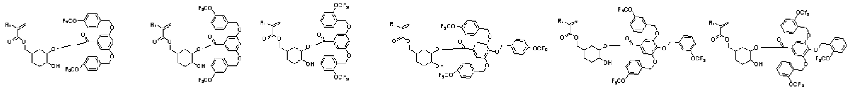

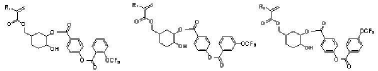

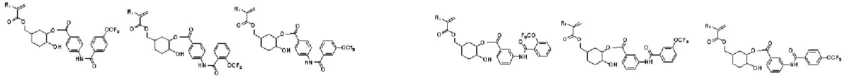

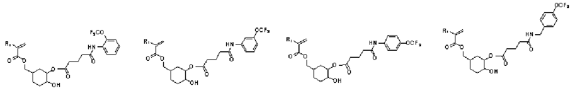

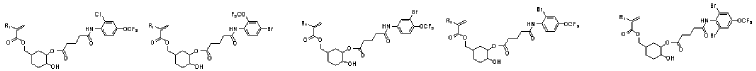

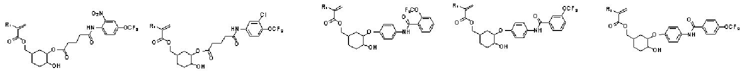

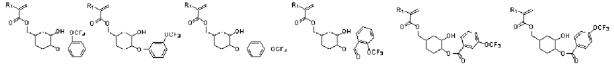

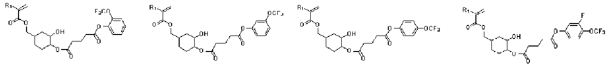

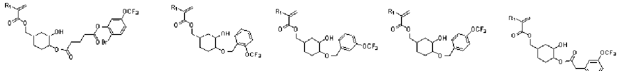

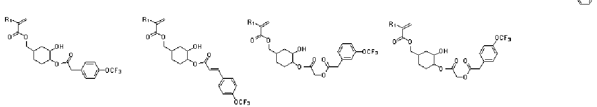

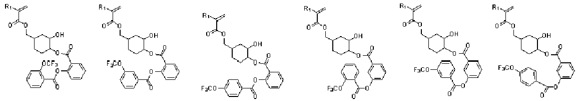

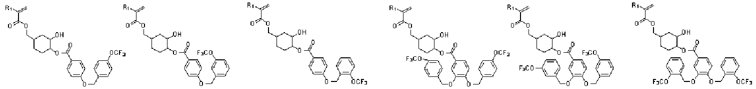

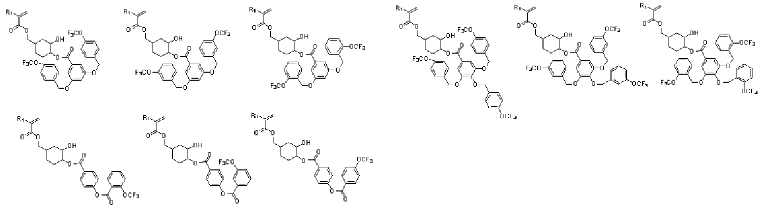

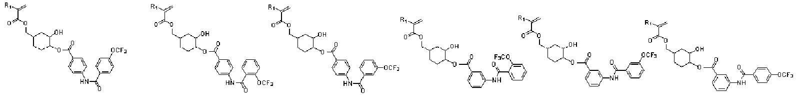

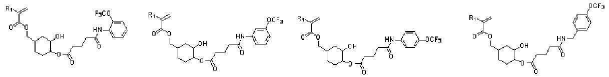

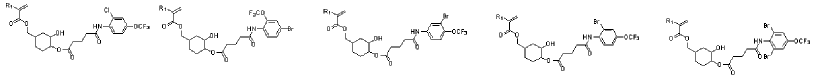

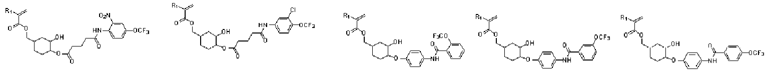

Preferable examples of the monomer represented by the general formula (7) include the following.

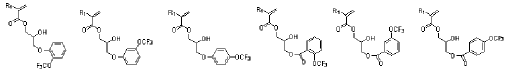

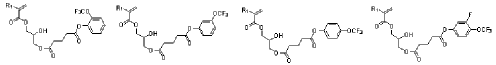

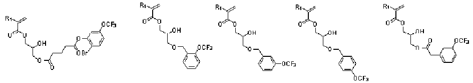

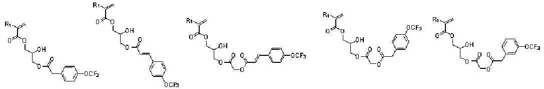

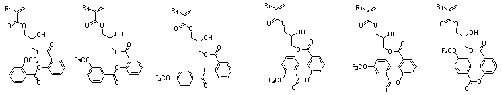

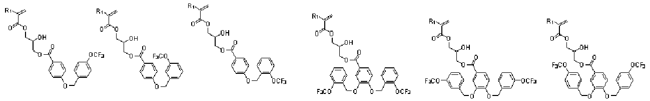

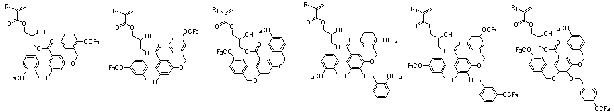

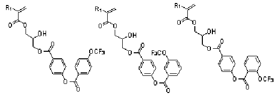

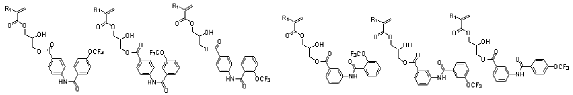

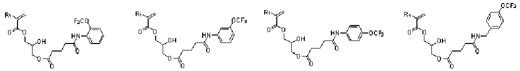

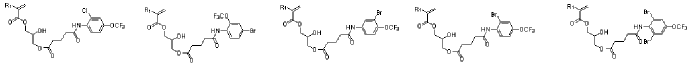

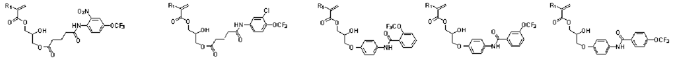

The monomers represented by the general formulae (6) and (7) are extremely useful as starting materials, that is, monomers for polymers to be used as surfactants for forming an organic film. These monomers allow high surface tension control by a hydroxy group, being a polar group, and trifluoromethoxybenzene, being a hydrophobic group, being positioned in a linking group and a terminal group of the monomer.

The R₂ in the general formulae (6) and (7) preferably contains a partial structure represented by one of the following general formulae (4). 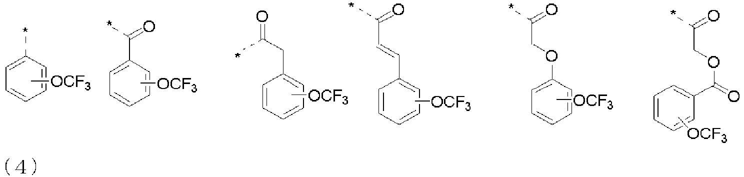 "*" represents an attachment point.

Among the above, partial structures in which the trifluoromethoxy group is a substituent in the para position relative to the substituent having the attachment point at "*" are particularly preferable from the viewpoint of surface active effect. As monomers having a trifluoromethoxy group in the para position, monomers in which the R₂ in the general formulae (6) and (7) contains a partial structure represented by the following general formula (8) are preferable, for example.

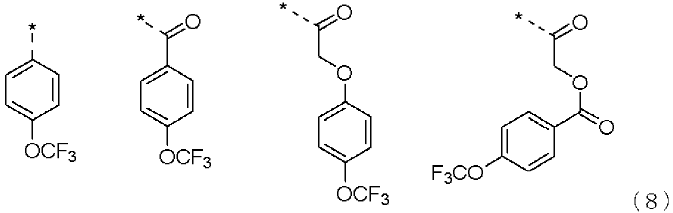

As monomers having a structure shown above, the following can be given as preferable examples. Among these, monomers in which, in the R₂, the trifluoromethoxybenzene and the side chain having the polymerizable group are bonded by an ester bond are preferable. The interaction between hydroxy groups or between the aromatic ring and the trifluoromethoxy group of the trifluoromethoxybenzene in the polymer becomes efficient, so that surface tension can be reduced efficiently and the surface active effect is enhanced, and improvement in film-formability can be expected. Among these examples, those having the partial structure represented by the general formula (2) are more preferable from the viewpoint of hump suppression, and furthermore, among these, the R₁ is further preferably a methyl group from the viewpoint of the toxicity of starting materials.

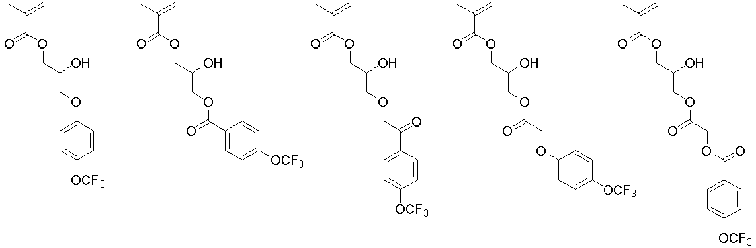

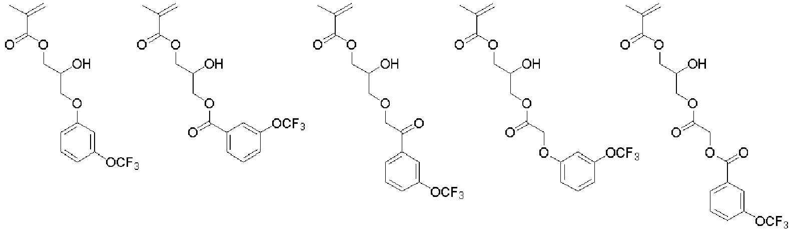

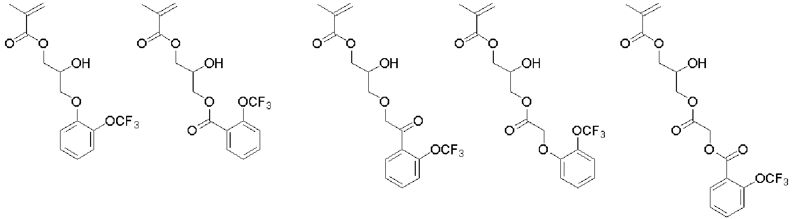

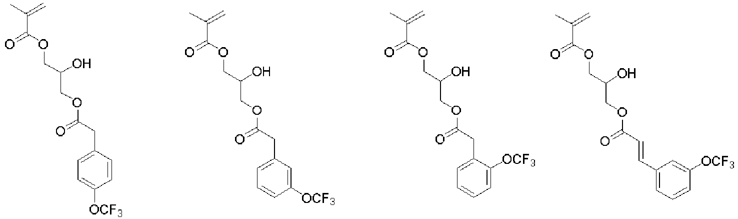

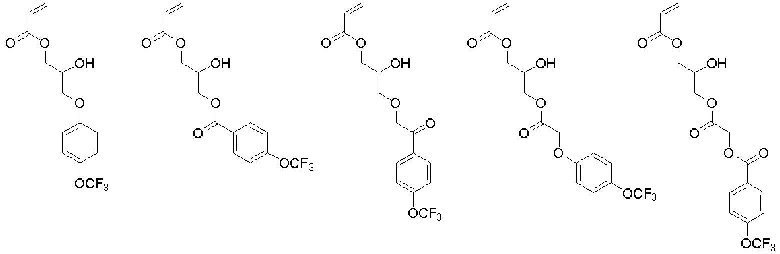

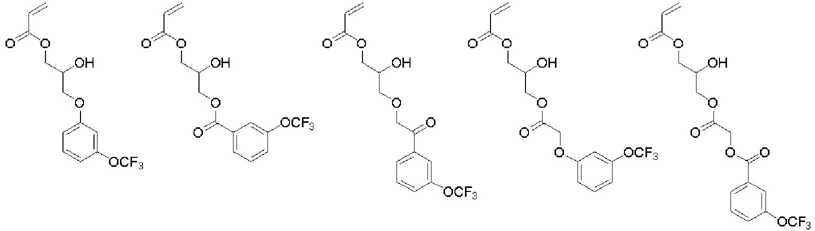

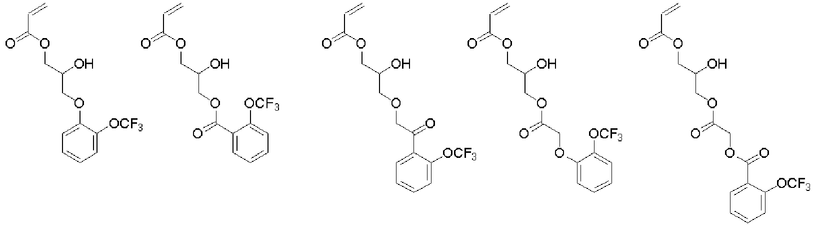

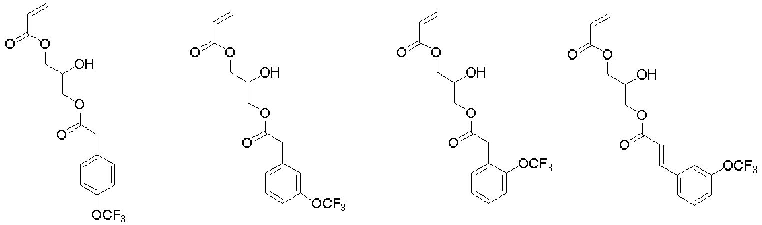

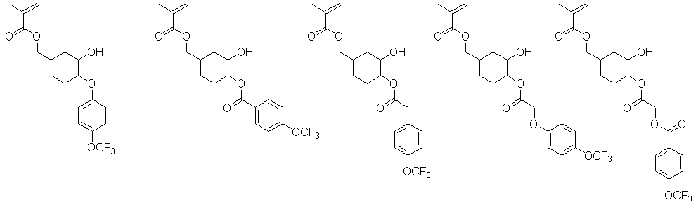

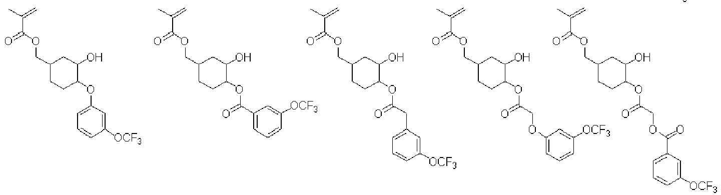

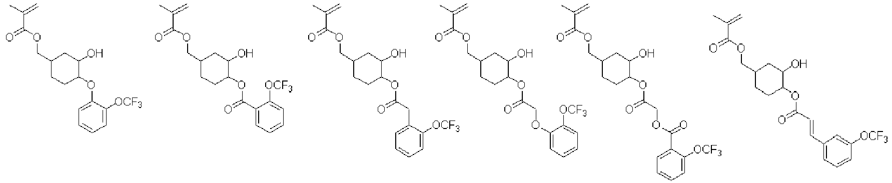

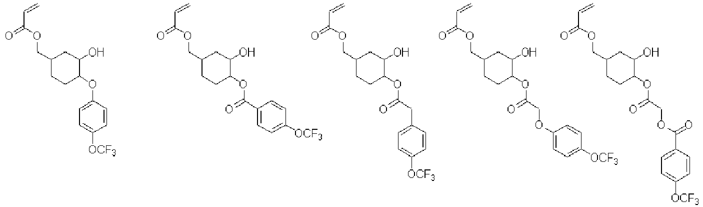

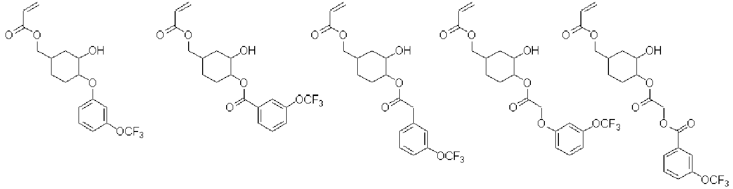

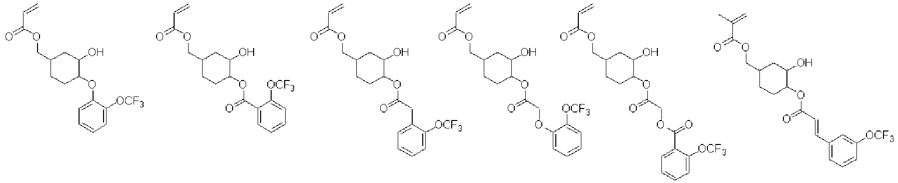

In the inventive monomer, a hydroxy group is introduced together with a substituent containing an appropriate fluorine structure, and therefore, a polymer synthesized using the inventive monomer functions as a surfactant and has the ability to reduce surface tension and thus, makes it possible to realize excellent uniform coatability (levelling property) of an organic film.

### [Method for Manufacturing Monomer]

Means for obtaining the monomer represented by the general formula (6) or (7) used in the present invention are not particularly limited. However, the monomer can be obtained by an addition reaction between an epoxy compound as shown in the following reaction formulae and a compound (carboxylic acid, alcohol, phenol, etc.) having R₂ as a substituent and having a hydroxy group. Note that, in this case, the monomer having the cyclohexane structure represented by (6) is obtained as a mixture of two kinds, since there is no reaction selectivity with the epoxycyclohexane as shown in the following reaction formula.

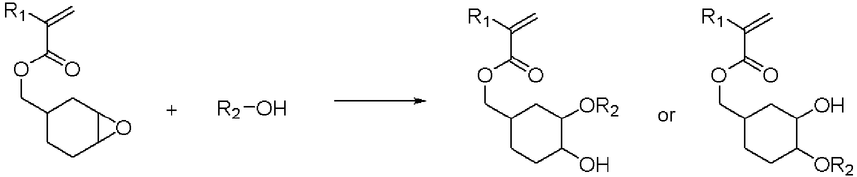

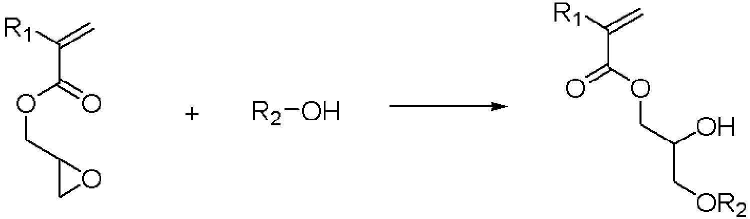

In the reaction between the epoxy compound and the compound having a hydroxy group shown above, the used amount of the compound having a hydroxy group is preferably 0.3 to 2.0 mol, more preferably 0.5 to 1.5 mol, and further preferably 0.75 to 1.25 mol based on 1 mol of the epoxy in the epoxy compound.

When the used amount of the compound having a hydroxy group relative to the epoxy unit is not insufficient, unreacted epoxy groups do not remain, and there is no influence on storage stability. When the used amount of the compound having a hydroxy group relative to the epoxy unit is not in large excess, an unreacted compound having a hydroxy group does not remain, and outgassing does not occur.

A compound synthesized from starting materials like those shown above can usually be obtained by allowing a reaction between an epoxy compound and a compound having a hydroxy group in a solvent or in a solvent and in the presence of a reaction catalyst at room temperature or under cooling or heating as necessary.

Examples of the solvent used include: alcohols, such as methanol, ethanol, isopropyl alcohol, butanol, ethylene glycol, propylene glycol, diethylene glycol, glycerol, methyl cellosolve, ethyl cellosolve, butyl cellosolve, and propylene glycol monomethyl ether; ethers, such as diethyl ether, dibutyl ether, diethylene glycol diethyl ether, diethylene glycol dimethyl ether, tetrahydrofuran, and 1,4-dioxane; chlorine-based solvents, such as methylene chloride, chloroform, dichloroethane, and trichloroethylene; hydrocarbons, such as hexane, heptane, benzene, toluene, xylene, and cumene; nitriles, such as acetonitrile; ketones, such as acetone, ethyl methyl ketone, and isobutyl methyl ketone; esters, such as ethyl acetate, n-butyl acetate, and propylene glycol methyl ether acetate; lactones, such as γ-butyrolactone; and aprotic polar solvents, such as dimethyl sulfoxide, N,N-dimethylformamide, and hexamethylphosphoric triamide. One of these solvents may be used, or two or more kinds thereof may be used in mixture. These solvents can be used in an amount of 0 to 2000 parts by mass based on 100 parts by mass of the reaction raw materials.

Examples of the reaction catalyst include quaternary ammonium salts, such as benzyltriethylammonium chloride, benzyltriethylammonium bromide, benzyltrimethylammonium chloride, tetramethylammonium chloride, tetramethylammonium bromide, tetramethylammonium iodide, tetramethylammonium hydroxide, tetraethylammonium bromide, tetrabutylammonium chloride, tetrabutylammonium bromide, tetrabutylammonium iodide, tetrabutylammonium hydrogen sulfate, trioctylmethylammonium chloride, tributylbenzylammonium chloride, trimethylbenzylammonium chloride, trimethylbenzylammonium hydroxide, N-laurylpyridinium chloride, N-lauryl-4-picolinium chloride, N-laurylpicolinium chloride, trimethylphenylammonium bromide, and N-benzylpicolinium chloride; quaternary phosphonium salts, such as tetrabutylphosphonium chloride, tetrabutylphosphonium bromide, and tetraphenylphosphonium chloride; tertiary amines, such as tris[2-(2-methoxyethoxy)ethyl]amine, tris(3,6-dioxaheptyl)amine, and tris(3,6-dioxaoctyl)amine; etc. The amount of the catalyst to be used can be 0.001 to 100 mass%, preferably 0.005 to 50 mass%, relative to the raw materials.

The reaction temperature is preferably about -50°C to the boiling point of the solvent, further preferably room temperature to 150°C. The reaction time is appropriately selected within a range of 0.1 to 100 hours.

Examples of methods for the reaction include: a method in which an epoxy compound, a compound having a hydroxy group, and a catalyst are charged at once; a method in which an epoxy compound and a compound having a hydroxy group are prepared into a form of dispersion or solution, and then a catalyst is added at once thereto or diluted with a solvent and added dropwise thereto; and a method in which a catalyst is prepared into a form of dispersion or solution, and then an epoxy compound and a compound having a hydroxy group are added at once thereto or diluted with a solvent and added dropwise thereto. After completion of the reaction, the resultant may be directly used without purification etc., or may be diluted with an organic solvent and then subjected to liquid-liquid separation washing to remove unreacted raw materials, the catalyst, and so forth present in the system, and to collect the reaction product.

The organic solvent used in this event is not particularly limited, as long as it is capable of dissolving the reaction product and being separated into two layers when mixed with water. Examples of the organic solvent include: hydrocarbons, such as hexane, heptane, benzene, toluene, and xylene; esters, such as ethyl acetate, n-butyl acetate, and propylene glycol methyl ether acetate; ketones, such as methyl ethyl ketone, methyl amyl ketone, cyclohexanone, and methyl isobutyl ketone; ethers, such as diethyl ether, diisopropyl ether, methyl-tert-butyl ether, and ethylcyclopentylmethyl ether; chlorinated solvents, such as methylene chloride, chloroform, dichloroethane, and trichloroethylene; mixtures thereof; and the like. As the water used for washing in this event, generally, what is called deionized water or ultrapure water may be used. The washing may be performed once or more, preferably approximately once to five times, because washing ten times or more does not always produce the full washing effects thereof.

In the liquid-liquid separation washing, the washing may be performed with a basic aqueous solution to remove unreacted compounds having a hydroxy group or acidic components. Examples of the base include hydroxides of alkaline metals, carbonates of alkaline metals, hydroxides of alkali earth metals, carbonates of alkali earth metals, ammonia, organic ammonium, etc.

Further, in the liquid-liquid separation washing, the washing may be performed with an acidic aqueous solution to remove metal impurities or basic components in the system. Examples of the acid include: inorganic acids, such as hydrochloric acid, hydrobromic acid, sulfuric acid, nitric acid, phosphoric acid, and heteropoly acid; organic acids, such as oxalic acid, trifluoroacetic acid, methanesulfonic acid, benzenesulfonic acid, p-toluenesulfonic acid, and trifluoromethanesulfonic acid; etc.

The liquid-liquid separation washing with water may be performed with any one of the basic aqueous solution and the acidic aqueous solution, or can be performed with a combination of the two. The liquid-liquid separation washing with water is preferably performed with the basic aqueous solution and the acidic aqueous solution in this order from the viewpoint of removing the metal impurities.

After the liquid-liquid separation washing with water with the basic aqueous solution and/or acidic aqueous solution, washing with neutral water may be successively performed. The washing may be performed once or more, preferably about once to five times. As the neutral water, deionized water, ultrapure water, or the like mentioned above may be used. The washing may be performed once or more, preferably approximately once to five times because washing ten times or more does not always produce the full washing effects.

Further, the reaction product after the liquid-liquid separation operation can also be collected as a powder by concentration and drying of the solvent under reduced pressure or normal pressure or by crystallizing the reaction product. In a case where there is a boiling point, the reaction product can also be generated by distillation under reduced pressure. The reaction product can also be retained in a solution state with an appropriate concentration to improve the workability when the reaction product is used for polymerization as a monomer that is a highly viscous liquid or the like. When the concentration in this event is not excessively high, the viscosity is not too high, and there is no risk of workability being degraded. When the concentration is not excessively low, the amount of the solvent is not excessive, and the solution can be prepared economically. The concentration in this event is preferably 0.1 to 50 mass%, further preferably 0.5 to 30 mass%.

The solvent in this event is not particularly limited, as long as it is capable of dissolving the obtained monomer and does not have an adverse effect on storage stability or when polymerizing the monomer. Specific examples of the solvent include: ketones, such as cyclohexanone and methyl-2-amyl ketone; alcohols, such as 3-methoxybutanol, 3-methyl-3-methoxybutanol, 1-methoxy-2-propanol, and 1-ethoxy-2-propanol; ethers, such as propylene glycol monomethyl ether, ethylene glycol monomethyl ether, propylene glycol monoethyl ether, ethylene glycol monoethyl ether, propylene glycol dimethyl ether, and diethylene glycol dimethyl ether; and esters such as propylene glycol monomethyl ether acetate, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate. One of these can be used or a mixture of two or more thereof can be used.

### [Polymer]

The inventive polymer for forming an organic film has a repeating unit represented by the following general formula (1) and/or (2).

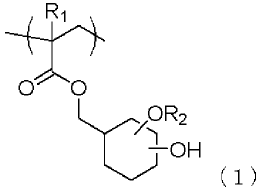

In the formula (1), R₁ represents a hydrogen atom or a methyl group, R₂ represents a saturated or unsaturated monovalent organic group having 7 to 50 carbon atoms and at least has at least one fluorine-containing structure represented by the following formula (3), and the substituents OR₂ and OH group on the cyclohexane ring are on adjacent carbon atoms on the cyclohexane ring.

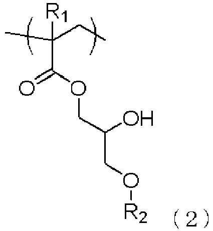

In the formula (2), R₁ and R₂ are as defined above. 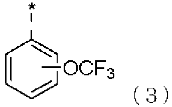 (3) is a partial structure contained in the R₂, "*" represents an attachment point in a structure in the R₂, and two or more kinds of structures represented by (3) or two or more identical structures represented by (3) are optionally contained in the R₂.

The above-described polymer has, in the side chain, a hydroxy group, being a hydrophilic group, on the linking group and a trifluoromethoxybenzene structure, being a hydrophobic group, on the end. Therefore, when the polymer is used as a surfactant, appropriate control of surface tension is possible, and when used as a surfactant for forming an organic film, the polymer can provide excellent film-formability.

Preferable examples of the polymer having the repeating unit represented by the general formula (1) include a polymer obtained using a monomer represented by the general formula (6). Similarly, preferable examples of the polymer having the repeating unit represented by the general formula (2) include a polymer obtained using a monomer represented by the general formula (7). The specific reaction formula is as shown below, and the polymer can be obtained by a polymerization method such as the radical polymerization of the unsaturated bond in the monomers shown in paragraphs [0058] to [0060]. From the viewpoint of exhibiting a surface active effect, the structure represented by the general formula (2) is preferable.

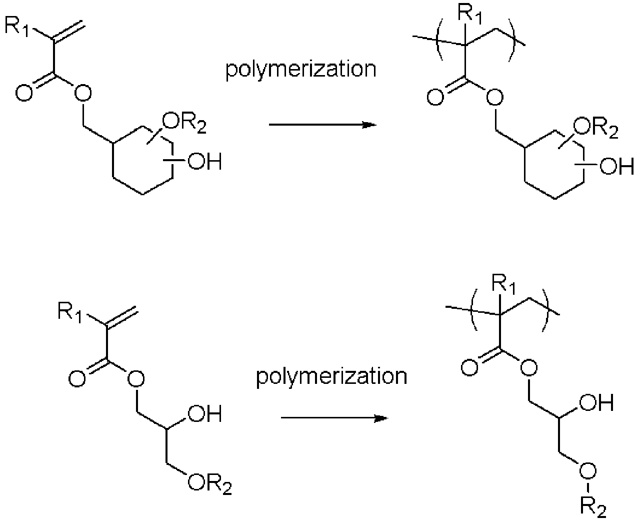

Furthermore, the R₂ in the general formulae (1) and (2) of the present invention preferably contains a partial structure represented by one of the following general formulae (4). 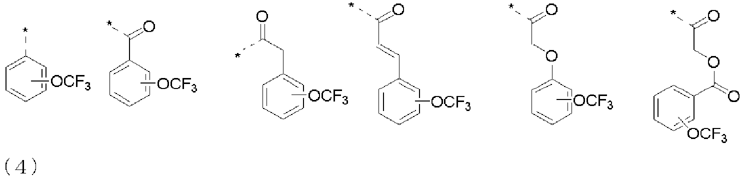 "*" represents an attachment point.

Preferable examples of the polymer having a partial structure represented by one of the general formulae (4) include polymers obtained by polymerization using a monomer shown in paragraph [0064]. In particular, a polymer having a repeating unit shown below is more preferable from the viewpoint of surface active effect. To allow a fluorophilic effect, which is the interaction between compounds having a fluorine substituent, to be exhibited efficiently, the trifluoromethoxy group is preferably in the para position on the aromatic group, and furthermore, it is more preferable that R₁ is a methyl group from the availability of raw materials.

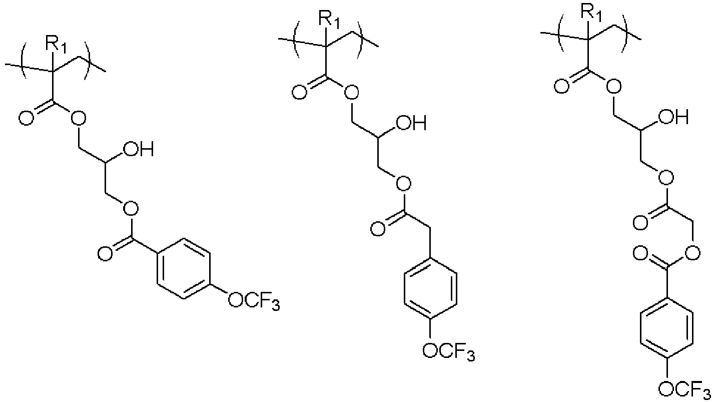

Furthermore, the polymer is preferably a copolymer having repeating units of one or both of the general formulae (1) and (2) and the following general formula (5).

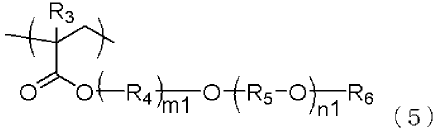

In the formula, R₃ represents a hydrogen atom or a methyl group; R₄ and R₅ each represent a linear or branched divalent alkylene group having 1 to 4 carbon atoms; R₆ represents a hydrogen atom, an alkyl group having 1 to 4 carbon atoms, or a phenyl group; "m1" represents 0 to 23; "n1" represents 0 to 23; and "m1" and "n1" satisfy 2 ≤ m1+n1 ≤ 23.

When the polymer is a copolymer having the above-described partial structure, the polymer can be used as a surfactant that makes it possible to form a film without various kinds of dependence on film thickness.

The above-described R₄ and R₅ each represent a linear or branched divalent alkylene group having 1 to 4 carbon atoms. Specific examples of the linear or branched divalent alkylene group having 1 to 4 carbon atoms include a methylene group, an ethylene group, a propylene group, a butylene group, a trimethylene group, and a tetramethylene group. Among these, an ethylene group is more preferable from the viewpoint of void-elimination in film-formability. The array of (R₄O) and (R₅O) may be any of random, block, or multiblock.

The above-described R₆ represents a hydrogen atom, an alkyl group having 1 to 4 carbon atoms, or a phenyl group. Specific examples of the alkyl group having 1 to 4 carbon atoms include a methyl group, an ethyl group, a propyl group, an isopropyl group, an n-butyl group, an s-butyl group, a t-butyl group, and an isobutyl group. In particular, a hydrogen atom, a methyl group, an ethyl group, and a phenyl group are more preferable. From the availability of raw materials, a hydrogen atom or a methyl group is preferable.

The above-described "m1" represents 0 to 23, "n1" represents 0 to 23, and "m1" and "n1" satisfy 2 ≤ m1+n1 ≤ 23. Here, "m1" and "n1" each represent an average repeating number. The upper limit of m1+n1 is 23, and from the viewpoint of storage stability at low temperature, "m1" and "n1" more preferable satisfy 9 ≤ m1+n1 ≤ 13.

Further specific examples of the repeating unit represented by the general formula (5) include the following.

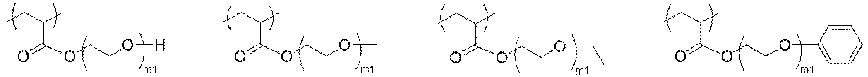

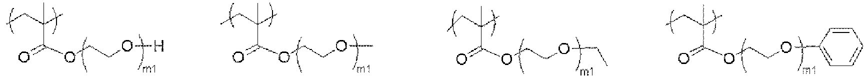

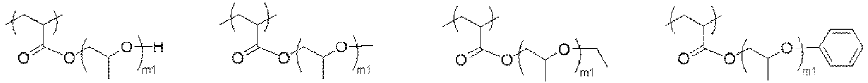

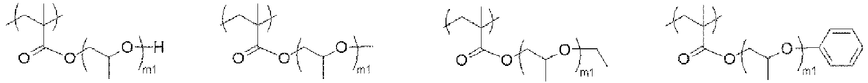

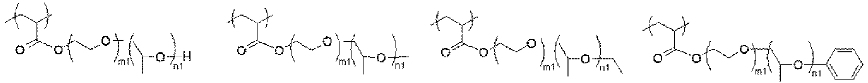

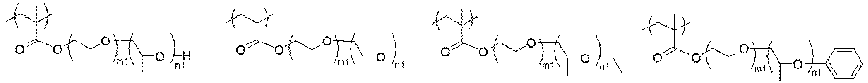

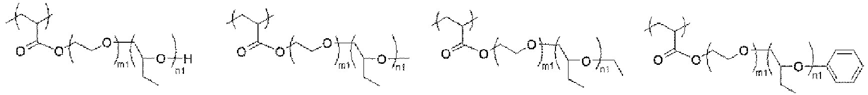

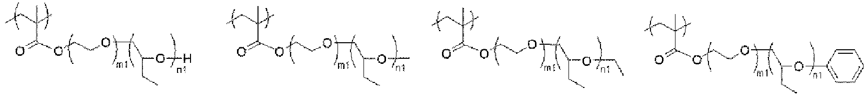

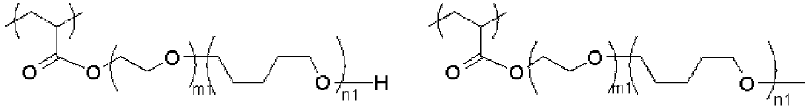

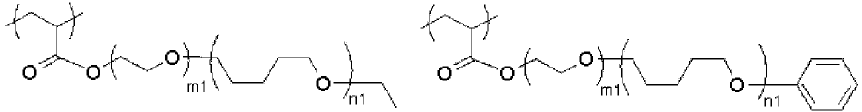

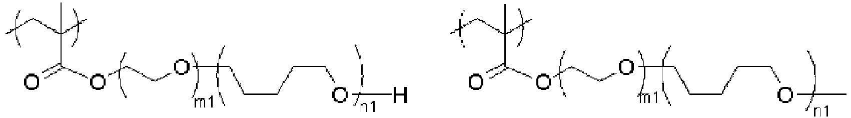

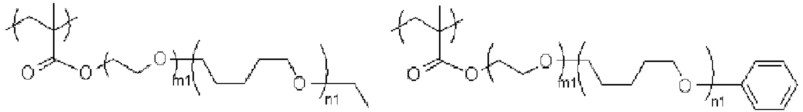

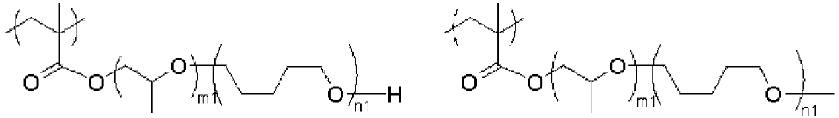

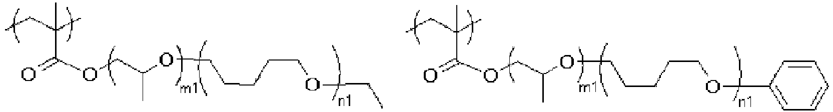

Furthermore, the above-described polymer may be achieved by a combination of one or more kinds of each of the monomers to give the structural units represented by the general formulae (1) and (2) and the monomer to give the structural unit represented by the general formula (5). For example, for the improvement of film-formability, it is possible to use a combination of monomers that can achieve multiple repeating units of (1) and (2), a combination of multiple monomers that can achieve repeating units represented by (5), and multiple monomers of each of monomers that can achieve repeating units of (1), (2), and (5). Thus, the combination can be selected appropriately in accordance with the properties of the resin, compound, or the like to be used for the organic film to be adopted. In this manner, it is possible to adjust the performance for achieving the desired film-formability depending on the resin or compound contained in the material for forming an organic film even in the case of a phenolic resin, a polymethacrylate or polyacrylate obtained by radical polymerization, polystyrene, polyimide, polyimine, polycarbonate, etc., or a single compound such as a polyfunctional phenolic compound.

In a case where a copolymer is used, when the proportion of repeating units represented by the general formula (1) and/or (2) is X and the proportion of repeating units represented by (5) is Y, and X+Y=1, it is preferable that X is 0.5 or more and Y is less than 0.5, and it is more preferable that X is 0.6 or more. When the proportions are as described, the copolymer sufficiently exhibits a function as a surfactant and can be applied to a material for forming an organic film regardless of resins and compounds and without dependence on film thickness, and the material for forming an organic film is applicable as a highly versatile material.

The polymer preferably has a weight-average molecular weight of 1500 to 30000, more preferably 3000 to 25000, and further preferably 5000 to 23000. When the weight-average molecular weight is controlled in such ranges, sufficient surface active effect for a material for forming an organic film can be exhibited stably. Furthermore, it is possible to prevent film-formability failure caused by degradation in affinity to the solvent used in the material for forming an organic film.

Meanwhile, the molecular weight distribution (Mw/Mn) is preferably 1.0 to 5.0, more preferably 1.1 to 3.0. It is possible to prevent the degradation of the surface active effect due to a wide range of ultra-high-molecular-weight polymers and low-molecular-weight compounds, being similar to monomers, being present in the polymer in mixture caused by the Mw/Mn (molecular weight distribution) being wide. Note that, specifically, the measurement of the molecular weight was performed in the following manner. Weight-average molecular weight (Mw) and number-average molecular weight (Mn) on polystyrene basis were measured by gel permeation chromatography (GPC) using tetrahydrofuran as an eluent (solvent), and dispersity (Mw/Mn) was calculated therefrom.

Furthermore, in the present invention, a monomer to give a repeating unit other than (1), (2), and (5) can be additionally used in accordance with the resin or compound contained in the material for forming an organic film. Thus, by introducing not only the particular repeating units of the present invention but also other repeating units, the range of application as a material for forming an organic film can be further widened.

### [Method for Manufacturing Polymer]

Examples of methods for manufacturing the above-described polymer include a method of polymerizing the monomer by adding a radical polymerization initiator and heating in an organic solvent.

Examples of the organic solvent used at the time of the polymerization include toluene, benzene, tetrahydrofuran (THF), diethyl ether, dioxane, cyclohexane, cyclopentane, methyl ethyl ketone (MEK), propylene glycol monomethyl ether acetate (PGMEA), and γ-butyrolactone (GBL). Examples of the polymerization initiator include 2,2'-azobisisobutyronitrile (AIBN), 2,2'-azobis(2,4-dimethylvaleronitrile), dimethyl-2,2'-azobis(2-methylpropionate), 1,1'-azobis (1-acetoxy-1-phenylethane), benzoyl peroxide, and lauroyl peroxide. The amount of these initiators to be contained is preferably 0.01 to 25 mol% relative to the total amount of the monomer to be polymerized. The reaction temperature is preferably 50 to 150°C, more preferably 60 to 100°C. The reaction time is preferably 2 to 24 hours, and from the viewpoint of production efficiency, more preferably 2 to 18 hours.

The polymerization initiator may be added to the monomer solution and supplied to a reaction vessel, or an initiator solution may be prepared separately from the monomer solution, and each solution may be supplied to the reaction vessel independently. The polymerization reaction may progress due to radicals generated from the initiator during waiting time and an ultra-high-molecular-weight polymer may be generated, and therefore, from the viewpoint of quality control, the monomer solution and the initiator solution are preferably each prepared independently and added dropwise. In addition, for adjusting the molecular weight, a known chain transfer agent, such as dodecyl mercaptan and 2-mercaptoethanol, may also be used. In this case, the amount of these chain transfer agents to be added is preferably 0.01 to 20 mol% relative to the total amount of the monomer to be polymerized.

Incidentally, the amount of each monomer in the monomer solution can be set appropriately, for example, in such a manner as to achieve a favorable content ratio of the repeating units in accordance with the required performance of the above-described polymer.

As for the polymer obtained in the manufacturing method, a reaction solution obtained by the polymerization reaction may be a final product. Alternatively, a powder obtained via a purification step, such as a reprecipitation method in which the polymerization solution is added into a poor solvent to obtain a powder, may be treated as a final product.
From the viewpoints of operation efficiency and quality stabilization, the powder obtained in the purification step is preferably dissolved in a solvent to form a polymer solution to be operated as a final product.

Specific examples of the solvent used in this case include solvents described in paragraphs [0144] to [0145] in JP 2008-111103 A, and specifically include: ketones, such as cyclohexanone and methyl-2-n-pentylketone; alcohols, such as 3-methoxybutanol, 3-methyl-3-methoxybutanol, 1-methoxy-2-propanol, and 1-ethoxy-2-propanol; ethers, such as propylene glycol monomethyl ether (PGME), ethylene glycol monomethyl ether, propylene glycol monoethyl ether, ethylene glycol monoethyl ether, propylene glycol dimethyl ether, and diethylene glycol dimethyl ether; esters, such as PGMEA, propylene glycol monoethyl ether acetate, ethyl lactate, ethyl pyruvate, butyl acetate, methyl 3-methoxypropionate, ethyl 3-ethoxypropionate, tert-butyl acetate, tert-butyl propionate, and propylene glycol mono-tert-butyl ether acetate; lactones, such as GBL; alcohols, such as diacetone alcohol (DAA); alcoholic solvents having a high boiling point, such as diethylene glycol, propylene glycol, glycerin, 1,4-butanediol, and 1,3-butanediol; and mixed solvents thereof.

The concentration of the polymer in the solution when the polymer is operated as a polymer solution is preferably 0.1 to 60 mass%, more preferably 1 to 50 mass%. When the concentration is in this range, degradation of handling property due to increased viscosity caused by excessively high concentration does not occur, and no inconvenience occurs where the concentration is so low that the dilution amount of a polymer and compound solution is too large to add a predetermined amount of surfactant to a product when the polymer and compound are operated as a solution when a material for forming an organic film is formed, resulting in a narrowed range of thicknesses with which a film can be manufactured.

The polymer solution is preferably filtered with a filter. The filtration can remove a foreign matter and gel, which may cause film-formation failure, and is effective in terms of quality stabilization.

Examples of a material of the filter used for the filtration include a fluorocarbon, cellulose, nylon, a polyester, and a hydrocarbon. In the step of filtering the chemically-amplified resist composition, the filter is preferably formed with a fluorocarbon, so-called Teflon(R), a hydrocarbon, such as polyethylene and polypropylene, or nylon. A pore size of the filter can be appropriately selected depending on target cleanliness, and is preferably 100 nm or smaller, and more preferably 20 nm or smaller. One of these filters may be used alone, or a plurality of these filters may be used in combination. As for the filtration method, the solution may be passed through the filter once, but the solution is preferably circulated to be filtered a plurality of times. In the step for producing the polymer solution, the filtration step may be performed in any order any number of times, but the reaction solution after the polymerization reaction, the polymer solution, or both thereof are preferably filtered.

### [Different Method for Manufacturing Polymer]

Examples of different methods for manufacturing the above-described polymer include, as shown below, a method of polymerizing a monomer having an epoxycyclohexane group or an epoxy group by adding a radical polymerization initiator and heating in an organic solvent (Step 1), and then obtaining the polymer by an addition reaction with a compound (carboxylic acid, alcohol, phenol, etc.) having R₂ as a substituent and having a hydroxy group (Step 2). When a polymer having an epoxycyclohexane structure is used as a starting material in the same manner as in paragraph [0068], there is no reaction selectivity, and therefore, the structure of the polymer after the substitution with the R₂ has repeating units having two kinds of partial structures.

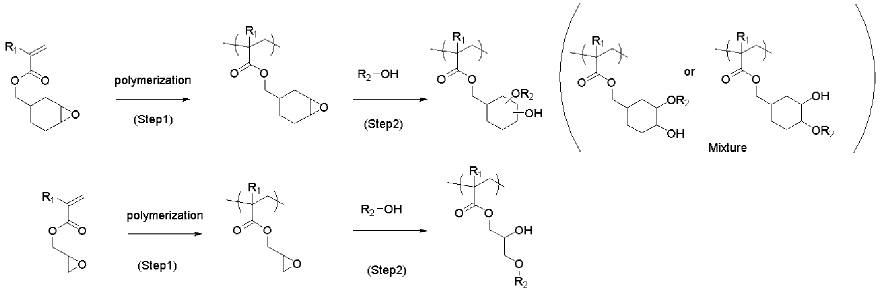

As the method for obtaining the polymer, the polymer can be obtained by the method described in [0100] to [0108], only changing the monomer to a compound having an epoxycyclohexane structure or an epoxy structure regarding the polymerization of Step 1. Meanwhile, regarding the addition reaction of Step 2, the polymer can be manufactured by the method described in [0068] to [0082], only changing the used starting material from the monomer having the epoxycyclohexane structure or the epoxy structure to the polymer obtained in Step 1. Furthermore, the obtained polymer may be used after filtering the obtained polymerization solution as described in [0107] and [0108].

In the production of the polymer obtained by these methods to be contained in a composition for forming an organic film, it is possible to adjust appropriately the structure and proportions of terminal-group structures of the compound in accordance with the required performance by using various compounds having a hydroxy group. It is possible to use any combination of, for example, compounds having a side chain structure that contributes to the improvement of planarizing property or compounds having a fluorine-containing substituent etc. for controlling surface tension or the like to change the surface activating ability. Therefore, these polymers not only have an excellent surface active effect that contributes to the improvement of film-formability, the polymers also make it possible to achieve high levels of various properties, such as film-formability and filling property, when a composition for forming an organic film is formed and used for an underlayer film.

### [Composition for Forming Organic Film]

The inventive composition for forming an organic film contains: a resin and/or compound (A) for forming an organic film; a polymer (B) having a repeating unit represented by the following general formula (1) and/or (2); and a solvent (C).

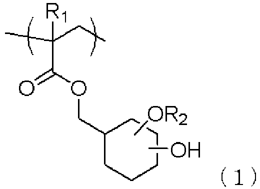

In the formula (1), R₁ represents a hydrogen atom or a methyl group, R₂ represents a saturated or unsaturated monovalent organic group having 7 to 50 carbon atoms and at least has at least one fluorine-containing structure represented by the following formula (3), and the substituents OR₂ and OH group on the cyclohexane ring are on adjacent carbon atoms on the cyclohexane ring.

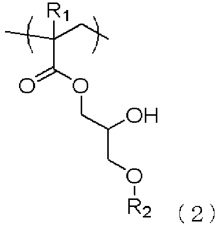

In the formula (2), R₁ and R₂ are as defined above. 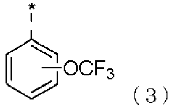 (3) is a partial structure contained in the R₂, "*" represents an attachment point in a structure in the R₂, and two or more kinds of structures represented by (3) or two or more identical structures represented by (3) are optionally contained in the R₂.

Note that, in the inventive composition for forming an organic film, one kind of each of the polymer (B), the resin and/or compound (A) for forming an organic film, and the solvent (C) may be used, or two or more kinds thereof may be used in combination. The inventive polymer (B) functions as a surfactant that can impart excellent film-formability and high levelling property. Uses for the polymer are not limited to organic underlayer films, and the polymer can be used for coating materials for photolithography in general. Examples include photosensitive resist materials and materials for forming a top coat to be formed on a resist film. Moreover, the polymer is applicable not only to materials for forming an organic film but also to silicon-containing resist middle layer films, and can be used as a suitable surfactant for realizing highly versatile film-formability that is applicable to various materials for forming a film.

Note that one kind of the polymer (B) of the present invention having a repeating unit represented by (1) and/or (2) may be used, or two or more kinds thereof may be used in combination. Regarding the contained amounts of these compounds, it is preferable that the polymer (B) is contained in an amount of 0.01 parts by mass to 5 parts by mass when the amount of the resin and/or compound (A) for forming an organic film is regarded as 100 parts by mass.

### [Resin and/or Compound (A) For Forming Organic Film]

The resin and/or compound (A) for forming an organic film contained in the inventive composition for forming an organic film is not particularly limited as long as the resin and/or compound has sufficient film-formability in spin-coating and curability. From the viewpoints of etching resistance, optical characteristics, heat resistance, etc., a resin and/or compound including an aromatic skeleton is preferable.

Examples of the aromatic skeleton include benzene, naphthalene, anthracene, pyrene, indene, fluorene, furan, pyrrole, thiophene, phosphole, pyrazole, oxazole, isoxazole, thiazole, pyridine, pyrazine, pyrimidine, pyridazine, triazine, carbazole, etc. Among these, benzene, naphthalene, fluorene, and carbazole are particularly preferable.

Examples of the resin and/or compound (A) for forming an organic film used in the present invention include resins containing the following structures, disclosed in JP 2012-001687 A and JP 2012-077295 A.

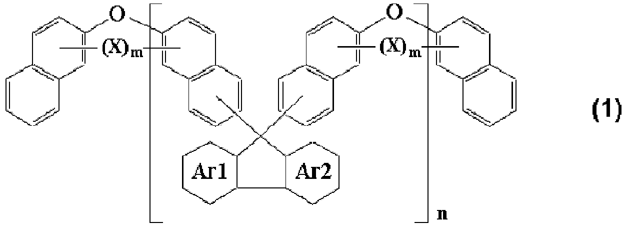

In the formula (1), the cyclic structures Ar1 and Ar2 each represent a benzene ring or a naphthalene ring. X represents a single bond or an alkylene group having 1 to 20 carbon atoms. "m" represents 0 or 1. "n" represents any natural number that provides a molecular weight of 100,000 or less. Note that the symbols in the formula apply only in this formula.

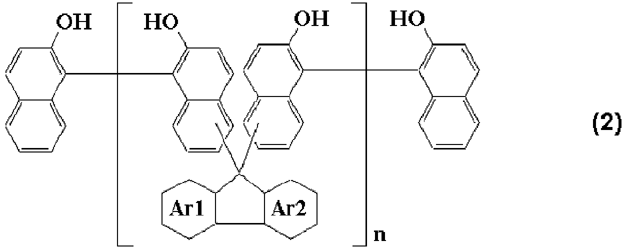

In the formula (2), the cyclic structures Ar1 and Ar2 each represent a benzene ring or a naphthalene ring. "n" represents any natural number that provides a weight-average molecular weight of 100,000 or less as measured by gel permeation chromatography in terms of polystyrene. Note that the symbols in the formula apply only in this formula.

Further examples of the resin and/or compound (A) for forming an organic film used in the present invention include resins containing the following structures, disclosed in JP 2004-264710 A, JP 2005-043471 A, JP 2005-250434 A, JP 2007-293294 A, and JP 2008-065303 A.

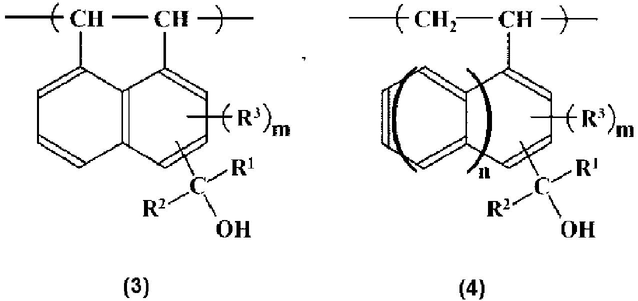

In the formula (3) and the formula (4), R¹ and R² each represent a hydrogen atom, an alkyl group having 1 to 3 carbon atoms, or an aryl group; R³ represents an alkyl group having 1 to 3 carbon atoms, a vinyl group, an allyl group, or an aryl group that is optionally substituted; "n" represents 0 or 1; and "m" represents 0, 1, or 2. Note that the symbols in the formulae apply only in these formulae.

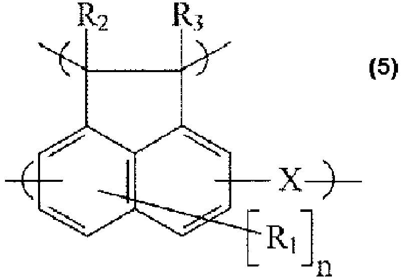

In the formula (5), R₁ represents a monovalent atom other than a hydrogen atom or a monovalent group; and "n" represents an integer of 0 to 4. Here, when "n" is 2 to 4, the multiple R₁'s may be identical to or different from each other. R₂ and R₃ each independently represent a monovalent atom or group. X represents a divalent group. Note that the symbols in the formula apply only in this formula.

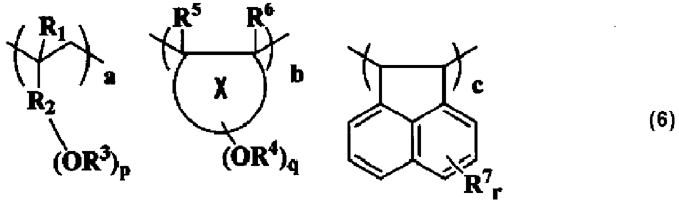

In the formula (6), R₁ represents a hydrogen atom or a methyl group. R₂ represents a single bond, a linear, branched, or cyclic alkylene group having 1 to 20 carbon atoms, or an arylene group having 6 to 10 carbon atoms, and may have any of ether, ester, lactone, and amide. R³ and R⁴ each represent a hydrogen atom or a glycidyl group. X represents any polymer of any of a hydrocarbon including an indene skeleton, a cycloolefin having 3 to 10 carbon atoms, and maleimide, and may have any of ether, ester, lactone, and carboxylic anhydride. R⁵ and R⁶ each represent a hydrogen atom, a fluorine atom, a methyl group, or a trifluoromethyl group. R⁷ represents a hydrogen atom, a linear, branched, or cyclic alkyl group having 1 to 6 carbon atoms, a hydroxy group, or an alkoxycarbonyl group. "p" and "q" each represent an integer of 1 to 4. "r" represents an integer of 0 to 4. "a", "b", and "c" each satisfy 0.5 ≤ a+b+c ≤ 1, 0 ≤ a ≤ 0.8, 0 ≤ b ≤ 0.8, 0.1 ≤ a+b ≤ 0.8, and 0.1 ≤ c ≤ 0.8. Note that the symbols in the formula apply only in this formula.

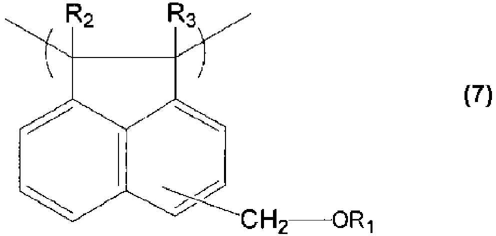

In the formula (7), R₁ represents a hydrogen atom or a monovalent organic group; and R₂ and R₃ each independently represent a monovalent atom or a monovalent organic group. Note that the symbols in the formula apply only in this formula.

Further specific examples of the resin and/or compound (A) for forming an organic film used in the present invention include resins containing the following structures, disclosed in JP 2004-205685 A, JP 2007-171895 A, and JP 2009-014816 A.

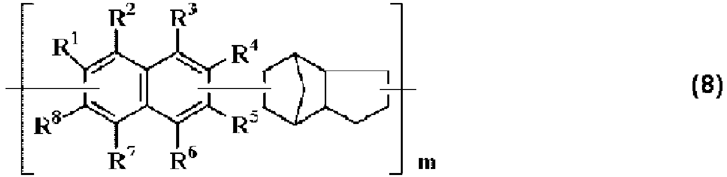

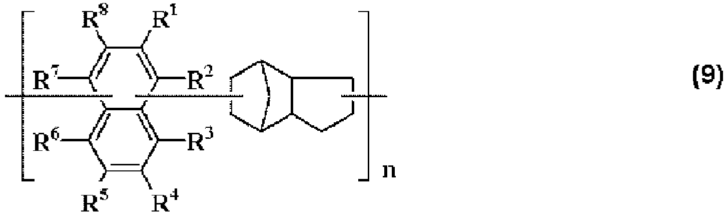

In the formula (8) and the formula (9), R¹ to R⁸ each independently represent a hydrogen atom, a hydroxy group, an alkyl group having 1 to 6 carbon atoms and optionally being substituted, an alkoxy group having 1 to 6 carbon atoms and optionally being substituted, an alkoxycarboxy group having 2 to 6 carbon atoms and optionally being substituted, an aryl group having 6 to 10 carbon atoms and optionally being substituted, a hydroxyalkyl group having 1 to 6 carbon atoms, an isocyanate group, or a glycidyl group. "m" and "n" each represent a positive integer. Note that the symbols in the formulae apply only in these formulae.

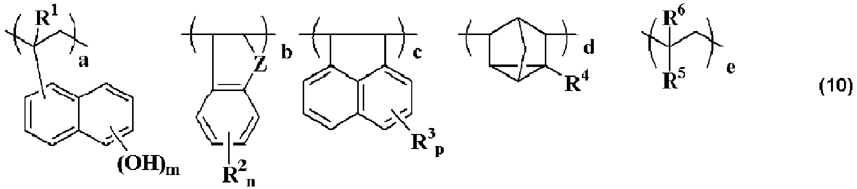

In the formula (10), R¹ and R⁶ each represent a hydrogen atom or a methyl group. R², R³, and R⁴ each represent a hydrogen atom, an alkyl group having 1 to 4 carbon atoms, an alkoxy group, a hydroxy group, an acetoxy group, an alkoxycarbonyl group, or an aryl group having 6 to 10 carbon atoms; R⁵ represents a condensed polycyclic hydrocarbon group having 13 to 30 carbon atoms, -O-R⁷, -C(=O)-O-R⁷, -O-C(=O)-R⁷, or -C(=O)-NR⁸-R⁷; "m" represents 1 or 2; "n" represents an integer of 0 to 4; and "p" represents an integer of 0 to 6. R⁷ represents an organic group having 7 to 30 carbon atoms; R⁸ represents a hydrogen atom or a hydrocarbon group having 1 to 6 carbon atoms. Z represents a methylene group, -O-, -S-, or -NH-. "a", "b", "c", "d", and "e" each satisfy 0 < a < 1.0, 0 ≤ b ≤ 0.8, 0 ≤ c ≤ 0.8, 0 ≤ d ≤ 0.8, 0 ≤ e ≤ 0.8, and 0 < b+c+d+e < 1.0. Note that the symbols in the formula apply only in this formula.

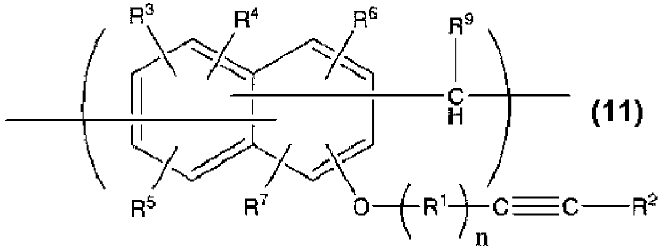

In the formula (11), "n" represents 0 or 1. R¹ represents a methylene group that is optionally substituted, an alkylene group having 2 to 20 carbon atoms and optionally being substituted, or an arylene group having 6 to 20 carbon atoms and optionally being substituted. R² represents a hydrogen atom, an alkyl group having 1 to 20 carbon atoms and optionally being substituted, or an aryl group having 6 to 20 carbon atoms and optionally being substituted. R³ to R⁷ each represent a hydroxy group, an alkyl group having 1 to 6 carbon atoms and optionally being substituted, an alkoxy group having 1 to 6 carbon atoms and optionally being substituted, an alkoxycarbonyl group having 2 to 10 carbon atoms and optionally being substituted, an aryl group having 6 to 14 carbon atoms and optionally being substituted, or a glycidyl ether group having 2 to 6 carbon atoms and optionally being substituted. R⁹ represents a hydrogen atom, a linear, branched, or cyclic alkyl group having 1 to 10 carbon atoms, a linear, branched, or cyclic alkyl ether group having 1 to 10 carbon atoms, or an aryl group having 6 to 10 carbon atoms. Note that the symbols in the formula apply only in this formula.

Examples of the formula (11) include the following resins.

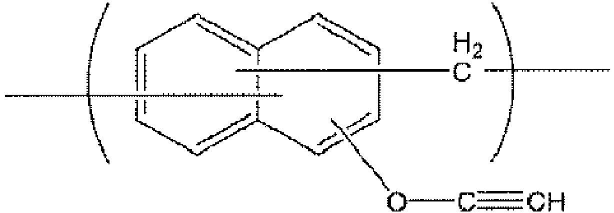

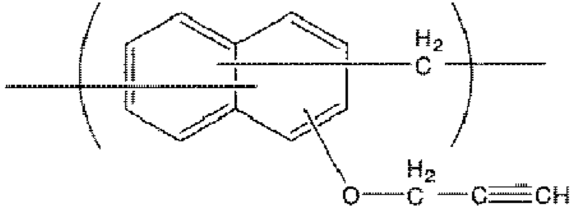

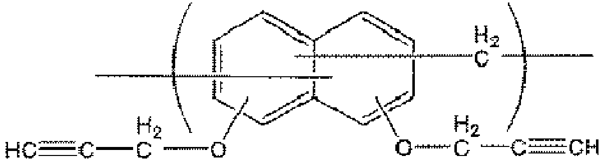

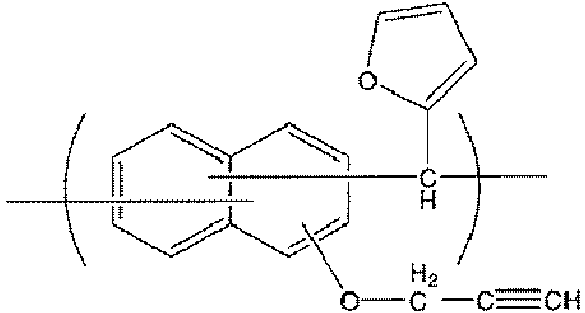

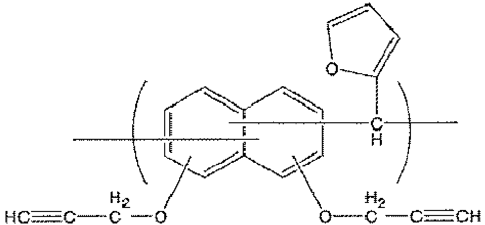

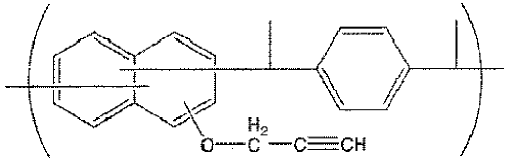

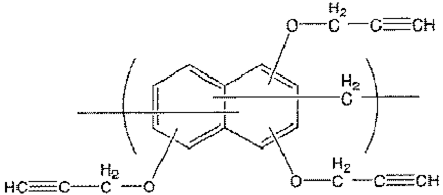

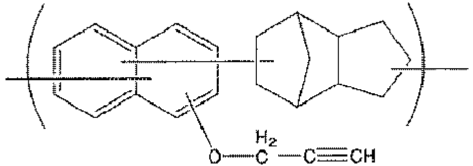

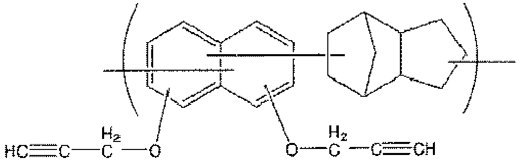

Further examples of the resin and/or compound (A) for forming an organic film used in the present invention include resins containing the following structures, disclosed in JP 2007-199653 A, JP 2008-274250 A, and JP 2010-122656 A.

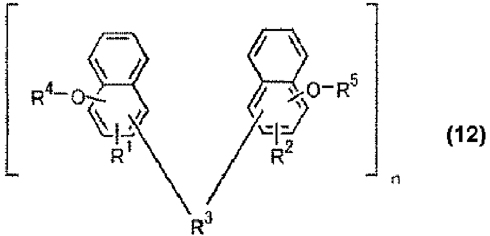

In the formula (12), R¹ and R² are identical to or different from each other and each independently represent a hydrogen atom, a linear, branched, or cyclic alkyl group having 1 to 10 carbon atoms, an aryl group having 6 to 10 carbon atoms, or an alkenyl group having 2 to 10 carbon atoms; R³ represents a single bond or a linear, branched, or cyclic alkylene group having 1 to 30 carbon atoms, and optionally has a bridged cyclic hydrocarbon group, a double bond, a heteroatom, or an aromatic group having 6 to 30 carbon atoms; R⁴ and R⁵ each independently represent a hydrogen atom or a glycidyl group; and "n" represents an integer of 1 to 4. Note that the symbols in the formula apply only in this formula.

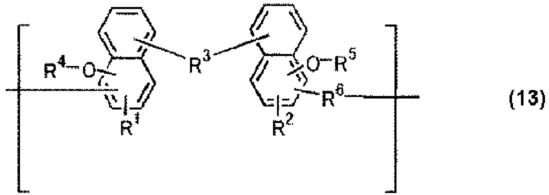

In the formula (13), R¹ and R² are identical to or different from each other and each independently represent a hydrogen atom, a linear, branched, or cyclic alkyl group having 1 to 10 carbon atoms, an aryl group having 6 to 10 carbon atoms, or an alkenyl group having 2 to 10 carbon atoms; R³ represents a single bond or a linear, branched, or cyclic alkylene group having 1 to 30 carbon atoms, and optionally has a bridged cyclic hydrocarbon group, a double bond, a heteroatom, or an aromatic group having 6 to 30 carbon atoms; R⁴ and R⁵ each independently represent a hydrogen atom or a glycidyl group; and R⁶ represents a single bond or a linear or branched alkylene group having 1 to 10 carbon atoms. Note that the symbols in the formula apply only in this formula.

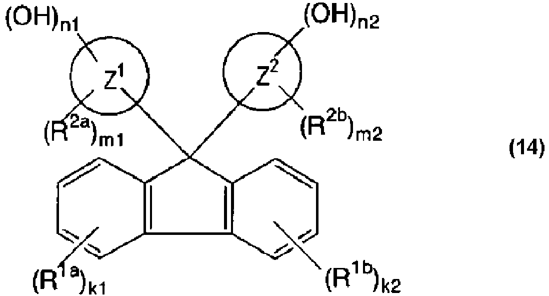

In the formula (14), the ring Z¹ and the ring Z² each represent a condensed polycyclic aromatic hydrocarbon ring; and R^{1a}, R^{1b}, R^{2a}, and R^{2b} are identical to or different from each other and each represent a substituent. "k1" and "k2" may be identical to or different from each other and each represent 0 or an integer of 1 to 4; "m1" and "m2" each represent 0 or an integer of 1 or more; and "n1" and "n2" each represent 0 or an integer of 1 or more. Here, 1 ≤ n1+n2. Note that the symbols in the formula apply only in this formula.

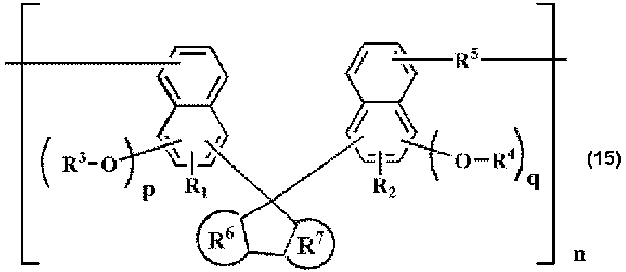

In the formula (15), R₁ and R₂ are identical to or different from each other, and each represent a hydrogen atom, a linear, branched, or cyclic alkyl group having 1 to 10 carbon atoms, an aryl group having 6 to 10 carbon atoms, or an alkenyl group having 2 to 10 carbon atoms. R³ and R⁴ each represent a hydrogen atom or a glycidyl group; R⁵ represents a single bond or a linear or branched alkylene group having 1 to 10 carbon atoms; and R⁶ and R⁷ each represent a benzene ring or a naphthalene ring. "p" and "q" each represent 1 or 2. "n" satisfies 0 < n ≤ 1. Note that the symbols in the formula apply only in this formula.

Examples of the formula (15) include the following resins.

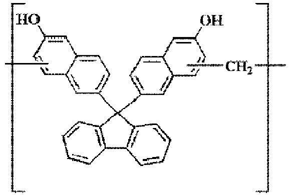

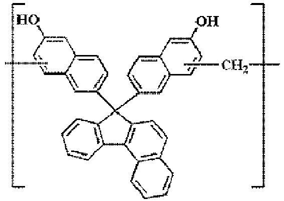

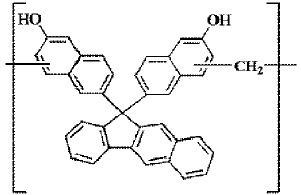

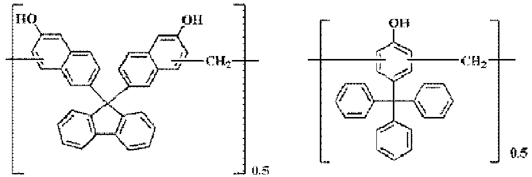

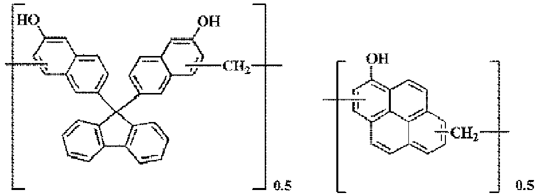

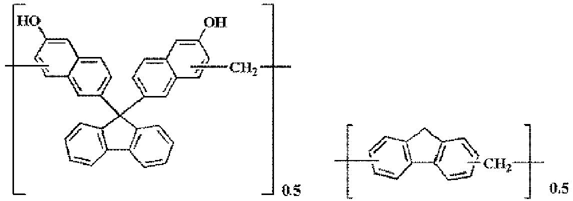

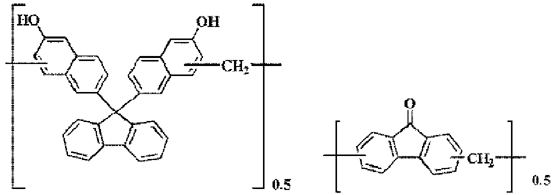

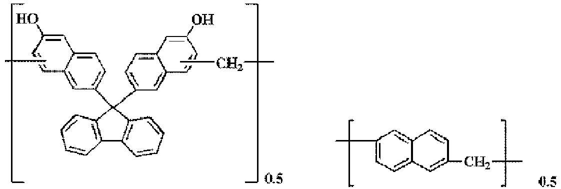

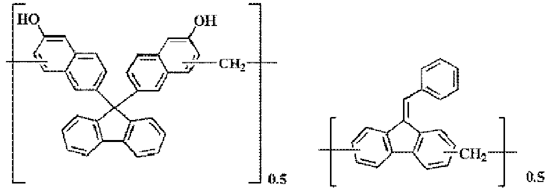

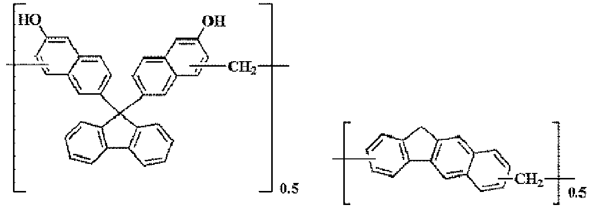

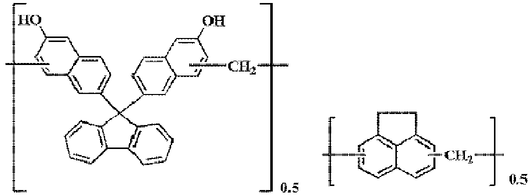

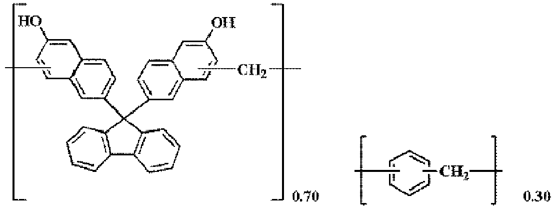

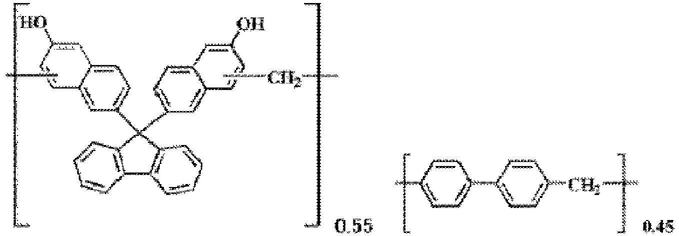

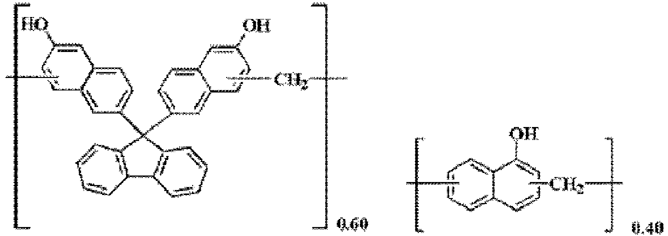

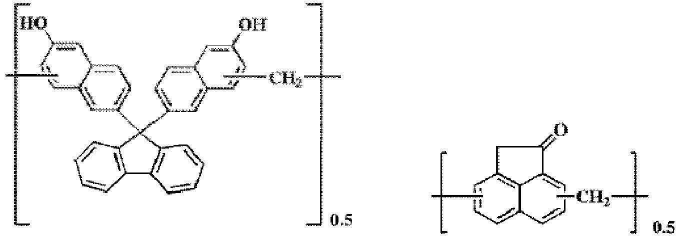

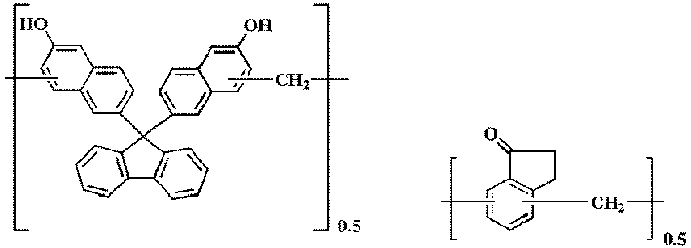

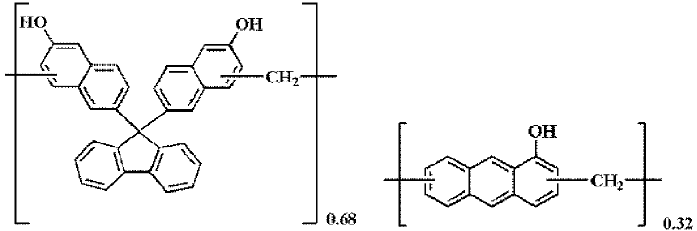

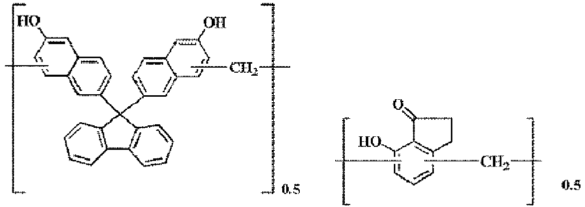

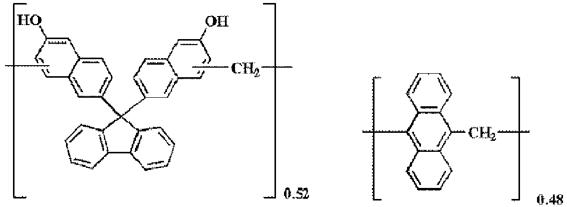

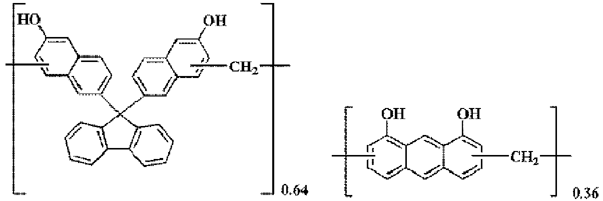

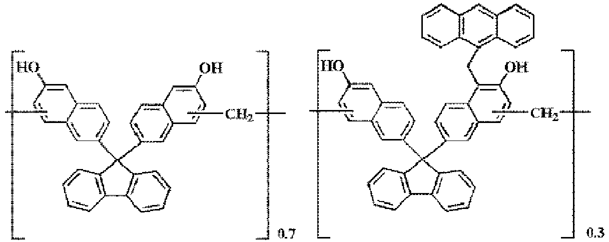

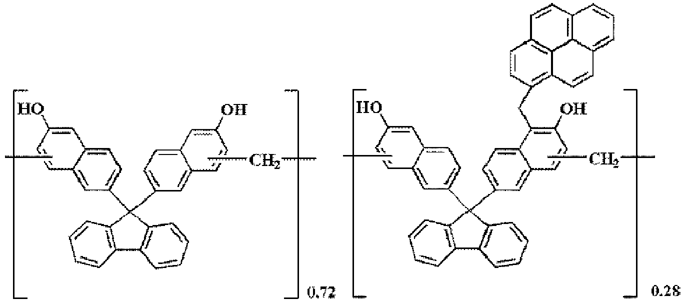

Further examples of the resin and/or compound (A) for forming an organic film used in the present invention include resins containing the following structure, disclosed in JP 2012-214720 A.

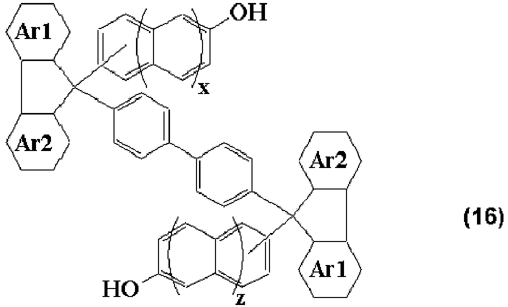

In the formula (16), the cyclic structures Ar1 and Ar2 each represent a benzene ring or a naphthalene ring. "x" and "z" each independently represent 0 or 1. Note that the symbols in the formula apply only in this formula.

Further examples of the resin and/or compound (A) for forming an organic film used in the present invention include resins disclosed in JP 2014-029435 A.

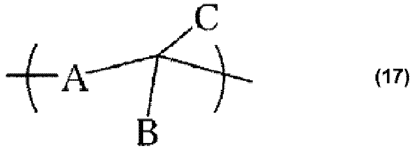

In the formula (17), A represents a structure having carbazole, B represents a structure having an aromatic ring, and C represents a hydrogen atom, an alkyl group, or a structure having an aromatic ring, B and C optionally constituting a ring with each other. One to four carboxy groups, a salt thereof, or a carboxylic acid ester group is contained in the combined structure of A, B, and C. Note that the symbols in the formula apply only in this formula.

Further examples of the resin and/or compound (A) for forming an organic film used in the present invention include polymers disclosed in WO 2012/077640 A1, the polymers containing a unit structure represented by the following formula (18) and a unit structure represented by the following formula (19), where the ratio of the unit structure represented by the formula (18) to the unit structure represented by the formula (19) is 3 to 97 : 97 to 3 by molar ratio.

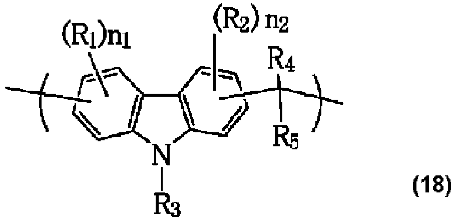

In the formula (18), R₁ and R₂ each independently represent a hydrogen atom, a halogen atom, a nitro group, an amino group, a hydroxy group, an alkyl group having 1 to 10 carbon atoms, an alkenyl group having 2 to 10 carbon atoms, an aryl group having 6 to 40 carbon atoms, or a combination of the groups, optionally including an ether bond, a ketone bond, or an ester bond. R₃ represents a hydrogen atom, an alkyl group having 1 to 10 carbon atoms, an alkenyl group having 2 to 10 carbon atoms, an aryl group having 6 to 40 carbon atoms, or a combination of the groups, optionally including an ether bond, a ketone bond, or an ester bond. R₄ represents a hydrogen atom, or an aryl group or heterocyclic group having 6 to 40 carbon atoms and optionally being substituted with a halogen atom, a nitro group, an amino group, or a hydroxy group; R₅ represents a hydrogen atom, or an alkyl group having 1 to 10 carbon atoms, aryl group having 6 to 40 carbon atoms, or heterocyclic group optionally substituted with a halogen atom, a nitro group, an amino group or a hydroxy group; and R₄ and R₅ may form a ring with each other. "n1" and "n2" each represent an integer of 1 to 3. Note that the symbols in the formula apply only in this formula.

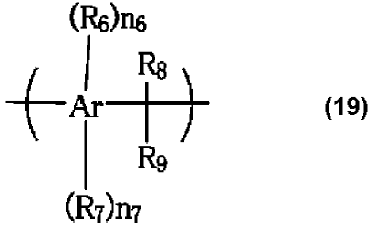

In the formula (19), Ar represents an aromatic ring group having 6 to 20 carbon atoms; R₆ represents a hydroxy group; and R₇ represents a hydrogen atom, a halogen atom, a nitro group, an amino group, an alkyl group having 1 to 10 carbon atoms, an alkenyl group having 2 to 10 carbon atoms, an aryl group having 6 to 40 carbon atoms, or a combination of the groups, optionally including an ether bond, a ketone bond, or an ester bond. R₈ represents a hydrogen atom, or an aryl group or heterocyclic group having 6 to 40 carbon atoms and optionally substituted with a halogen atom, a nitro group, an amino group, or a hydroxy group; R₉ represents a hydrogen atom, or an alkyl group having 1 to 10 carbon atoms optionally substituted with a halogen atom, a nitro group, an amino group, or a hydroxy group, an aryl group having 6 to 40 carbon atoms optionally substituted with a halogen atom, a nitro group, an amino group, or a hydroxy group, or a heterocyclic group optionally substituted with a halogen atom, a nitro group, an amino group, or a hydroxy group; and R₈ and R₉ may form a ring with each other. "n6" represents an integer of 1 to "p", "n7" represents an integer of p-n6. Here, "p" is the maximum number of substituents with which the aromatic ring group Ar can be substituted. Note that the symbols in the formula apply only in this formula.

Further examples of the resin and/or compound (A) for forming an organic film used in the present invention include polymers containing a unit structure represented by the following formula (20), disclosed in WO 2010/147155 A1.

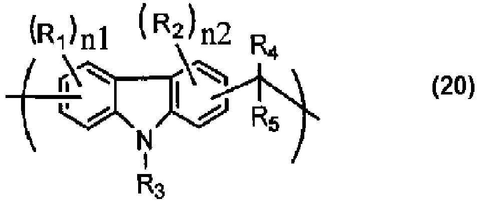

In the formula (20), R₁ and R₂ are each selected from the group consisting of a hydrogen atom, a halogen group, a nitro group, an amino group, a hydroxy group, alkyl groups having 1 to 10 carbon atoms, alkenyl groups having 2 to 10 carbon atoms, aryl groups having 6 to 40 carbon atoms, and combinations thereof, the alkyl groups, the alkenyl groups, or the aryl groups optionally containing an ether bond, a ketone bond, or an ester bond; R₃ is selected from the group consisting of a hydrogen atom, alkyl groups having 1 to 10 carbon atoms, alkenyl groups having 2 to 10 carbon atoms, aryl groups having 6 to 40 carbon atoms, and combinations thereof, the alkyl groups, the alkenyl groups, or the aryl groups optionally containing an ether bond, a ketone bond, or an ester bond; R₄ represents an aryl group or heterocyclic group having 6 to 40 carbon atoms and optionally substituted with a halogen group, a nitro group, an amino group, or a hydroxy group; R₅ represents a hydrogen atom or an alkyl group having 1 to 10 carbon atoms or an aryl group or heterocyclic group having 6 to 40 carbon atoms and optionally substituted with a halogen group, a nitro group, an amino group, or a hydroxy group; R₄ and R₅ optionally form a ring together with the carbon atom bonded to R₄ and R₅; and "n1" and "n2" each represent an integer of 1 to 3. Note that the symbols in the formula apply only in this formula.

Further examples of the resin and/or compound (A) for forming an organic film used in the present invention include: novolak resins obtained by a reaction between one or more phenols, such as phenol, cresol, xylenol, catechol, resorcinol, hydroquinone, pyrogallol, hydroxyquinol, and phloroglucinol, and one or more aldehyde sources, such as formaldehyde, paraformaldehyde, and trioxane, in the presence of an acid catalyst; and resins containing a repeating unit structure represented by the following formula (21), disclosed in WO 2012/176767 A1.

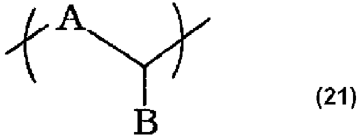

In the formula (21), A represents a hydroxy group-substituted phenylene group derived from polyhydroxybenzene; and B represents a monovalent condensed aromatic hydrocarbon ring group in which two to six benzene rings are condensed. Note that the symbols in the formula apply only in this formula.

Further examples of the resin and/or compound (A) for forming an organic film used in the present invention include novolak resins having a fluorene or tetrahydrospirobiindene structure, disclosed in JP 2005-128509 A, JP 2006-259249 A, JP 2006-259482 A, JP 2006-293298 A, and JP 2007-316282 A, the novolak resins containing a repeating unit structure represented by the following formula (22-1) or (22-2).

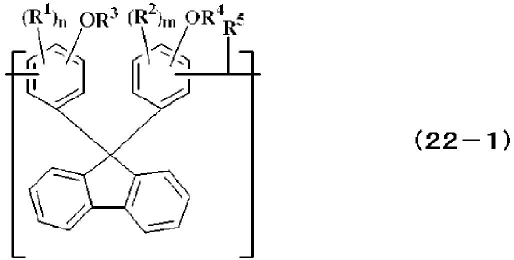

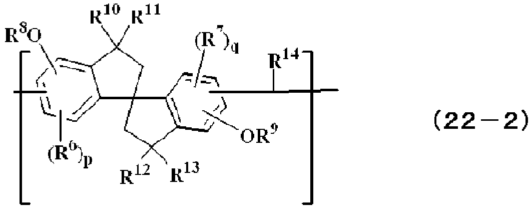

In the formula (22-1) and the formula (22-2), R¹, R², R⁶, and R⁷ each independently represent a hydrogen atom, a linear, branched, or cyclic alkyl group having 1 to 10 carbon atoms, an aryl group having 6 to 10 carbon atoms, an allyl group, or a halogen atom; R³, R⁴, R⁸, and R⁹ each independently represent a hydrogen atom, a linear, branched, or cyclic alkyl group having 1 to 6 carbon atoms, a linear, branched, or cyclic alkenyl group having 2 to 6 carbon atoms, an aryl group having 6 to 10 carbon atoms, or a glycidyl group; and R⁵ and R¹⁴ each independently represent a hydrogen atom, a linear, branched, or cyclic alkyl group having 1 to 10 carbon atoms, or an aryl group having 6 to 10 carbon atoms. "n", "m" , "p", and "q" each represent an integer of 1 to 3. R¹⁰ to R¹³ each independently represent a hydrogen atom, a halogen atom, a hydroxy group, a linear, branched, or cyclic alkyl group having 1 to 6 carbon atoms, or a linear, branched, or cyclic alkoxy group having 1 to 6 carbon atoms. Note that the symbols in the formulae apply only in these formulae.

Further examples of the resin and/or compound (A) for forming an organic film used in the present invention include reaction products obtained by a method disclosed in JP 2012-145897 A. More specific examples include polymers obtained by condensing one or more compounds represented by the following general formula (23-1) and/or (23-2) and one or more compounds represented by the following general formula (24-1) and/or (24-2) and/or an equivalent thereof.

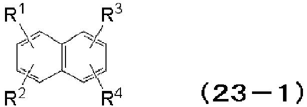

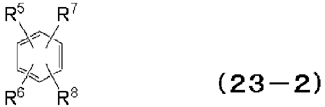

In the general formula (23-1) and the general formula (23-2), R¹ to R⁸ each independently represent a hydrogen atom, a halogen atom, a hydroxy group, an isocyanato group, a glycidyloxy group, a carboxy group, an amino group, an alkoxy group having 1 to 30 carbon atoms, an alkoxycarbonyl group having 1 to 30 carbon atoms, an alkanoyloxy group having 1 to 30 carbon atoms, or a saturated or unsaturated organic group having 1 to 30 carbon atoms and optionally being substituted. Furthermore, any two substituents selected from each of R¹ to R⁴ or R⁵ to R⁸ may be bonded to each other within a molecule to form a cyclic substituent. Note that the symbols in the formulae apply only in these formulae.

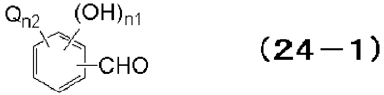

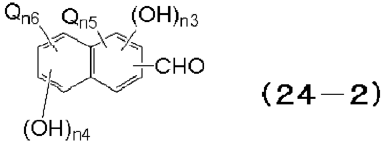

In the general formula (24-1) and the general formula (24-2), Q represents an organic group having 1 to 30 carbon atoms and optionally being substituted, and furthermore, any two Q's selected therefrom are optionally bonded with each other within a molecule to form a cyclic substituent. "n1" to "n6" each represent the number of each substituent, "n1" to "n6" are each independently 0, 1, or 2, and in the formula (24-1), hydroxybenzaldehyde is excluded. Meanwhile, in the formula (24-2), the relationships 0 ≤ n3+n5 ≤ 3, 0 ≤ n4+n6 ≤ 4, and 1 ≤ n3+n4 ≤ 4 are satisfied. Note that the symbols in the formulae apply only in these formulae.

Further examples also include polymers obtained by condensing one or more compounds represented by the general formula (23-1) and/or (23-2), one or more compounds represented by the general formula (24-1) and/or (24-2) and/or an equivalent thereof, and one or more compounds represented by the following general formula (25) and/or an equivalent thereof.

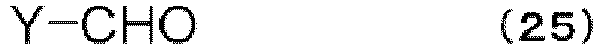

In the formula (25), Y represents a hydrogen atom or a monovalent organic group having 30 or fewer carbon atoms and optionally having a substituent, and the formula (25) is different from the general formula (24-1) and the general formula (24-2). Note that the symbol in the formula applies only in this formula.

Further examples of the resin and/or compound (A) for forming an organic film used in the present invention include compounds containing the following structure, disclosed in JP 2017-119671 A.

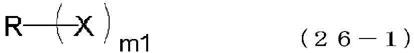

In the formula (26-1), R represents a single bond or an organic group having 1 to 50 carbon atoms; X represents a group represented by any of the following general formulae (26-2); and "m1" represents an integer that satisfies 2 ≤ m1 ≤ 10. Note that the symbols in the formula apply only in this formula.

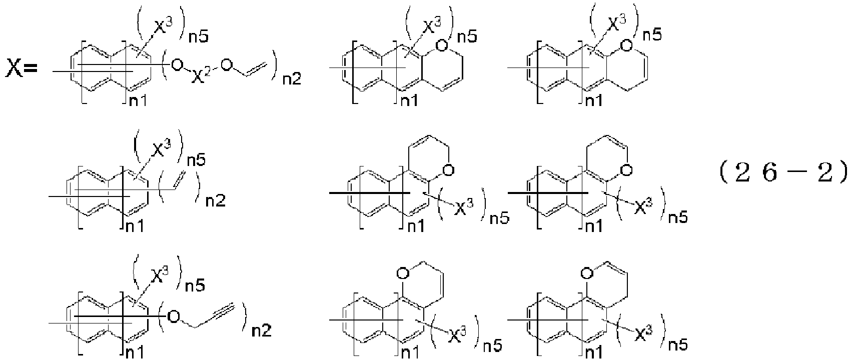

In the formulae, X² represents a divalent organic group having 1 to 10 carbon atoms; "n1" represents 0 or 1; "n2" represents 1 or 2; X³ represents a group represented by the following general formula (26-3); and "n5" represents 0, 1, or 2. Note that the symbols in the formulae apply only in these formulae.

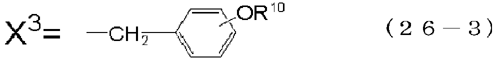

In the formula, R¹⁰ represents a hydrogen atom or a saturated or unsaturated hydrocarbon group having 1 to 10 carbon atoms, and a hydrogen atom on the benzene ring in the formula may be substituted with a methyl group or a methoxy group. Note that the symbol in the formula applies only in this formula.

Examples of the compounds containing the above-described structures include the following compounds.

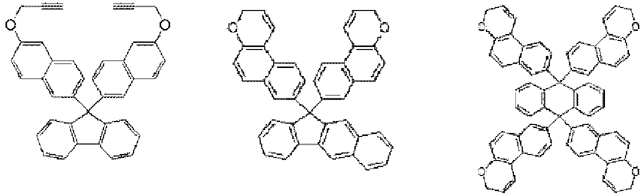

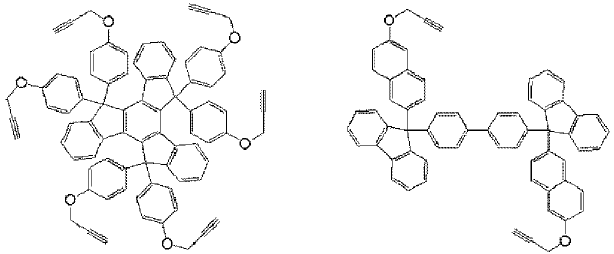

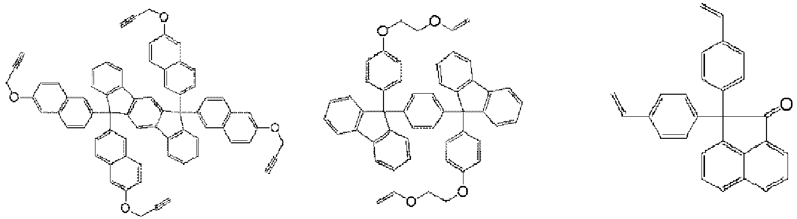

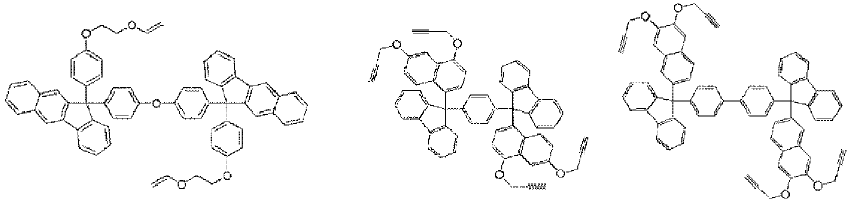

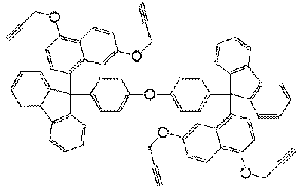

Further examples of the resin and/or compound (A) for forming an organic film used in the present invention include polymers having a repeating unit represented by the following general formula (27-1), disclosed in JP 2019-044022 A.

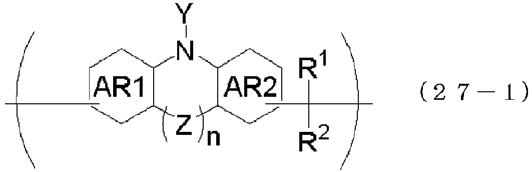

In the formula (27-1), AR1 and AR2 each represent a benzene ring or naphthalene ring optionally having a substituent. R¹ and R² each independently represent a hydrogen atom or an organic group having 1 to 30 carbon atoms, and when R¹ and R² each represent an organic group, R¹ and R² are optionally bonded to each other within a molecule to form a cyclic organic group. "n" represents 0 or 1; when n = 0, AR1 and AR2 do not form a bridged structure between the aromatic rings of AR1 and AR2 via Z; when n = 1, AR1 and AR2 form a bridged structure between the aromatic rings of AR1 and AR2 via Z; and Z represents a single bond or one of the following formulae (27-2). Y represents a group represented by the following formula (27-3). Note that the symbols in the formula apply only in this formula.

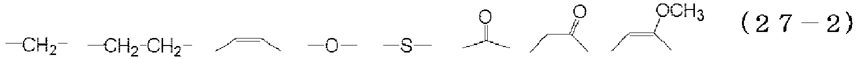

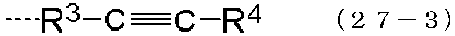

In the formula, R³ represents a single bond or a divalent organic group having 1 to 20 carbon atoms; R⁴ represents a hydrogen atom or a monovalent organic group having 1 to 20 carbon atoms; and a broken line represents an attachment point. Note that the symbols in the formula apply only in this formula.

Examples of the polymers having a repeating unit represented by the general formula (27-1) include the following polymers.

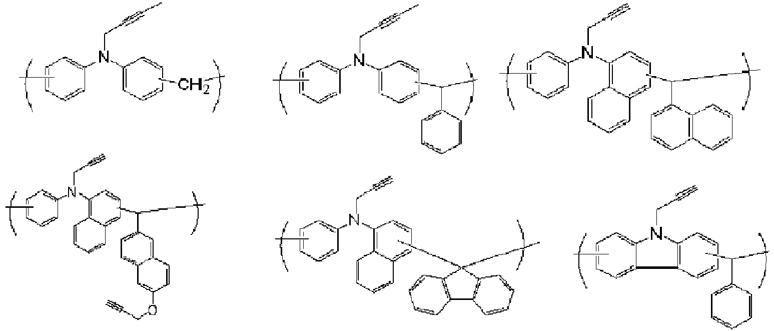

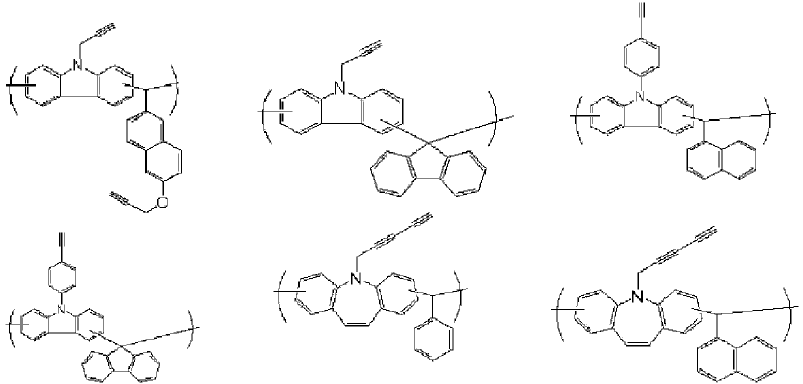

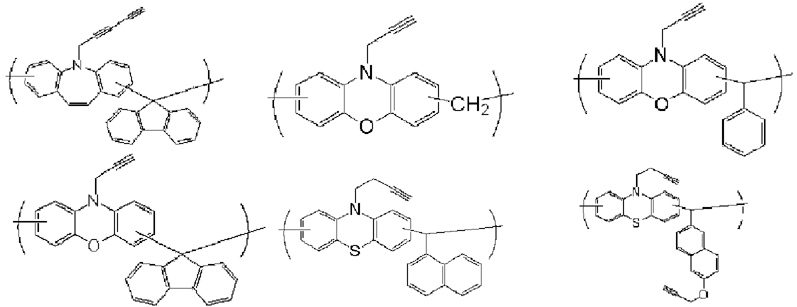

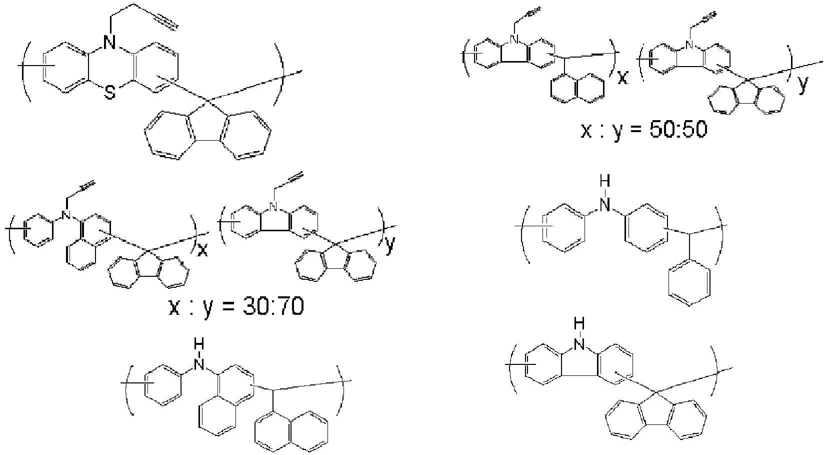

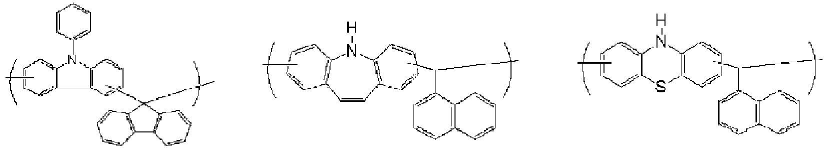

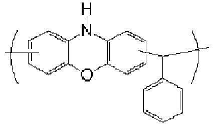

The resin and/or compound (A) for forming an organic film may be synthesized by a known method, and a commercially available product may be used.

The amount of the resin and/or compound (A) for forming an organic film to be contained is not particularly limited as long as sufficient film-formability can be achieved on spin-coating with the composition for forming an organic film, but the resin and/or compound (A) for forming an organic film is preferably contained in an amount of 10 to 40 parts by mass, more preferably 10 to 30 parts by mass, and further preferably 10 to 25 parts by mass, based on 100 parts by mass of the composition for forming an organic film. For example, when a hole or trench, having a very high aspect ratio, of a 3D NAND memory architecture is to be filled with the composition for forming an organic film, the contained amount of the resin for forming an organic film needs to be large. However, on the other hand, such a composition for forming an organic film is highly viscous, and in-plane uniformity after spin-coating and filling property are degraded. Even when the resin (A) for forming an organic film is contained in such a proportion, the inventive composition for forming an organic film makes it possible to form an organic film having excellent in-plane uniformity and excellent filling property, and can therefore be applied suitably.

Meanwhile, the polymer (B) is preferably contained in an amount of 0.01 parts by mass to 5 parts by mass based on 100 parts by mass of the resin and/or compound (A) for forming an organic film. A composition for forming an organic film containing the polymer in such an amount makes it possible to form an organic film with better in-plane uniformity.

The solvent (C) usable for the inventive composition for forming an organic film is not particularly limited as long as the solvent can dissolve the resin and/or compound (A) for forming an organic film and the polymer (B), and the solvent can preferably also dissolve the acid generator, crosslinking agent, surfactant, etc. described later. Specifically, it is possible to use a solvent having a boiling point lower than 180°C, such as the solvents disclosed in paragraphs

(0091) and (0092) of JP 2007-199653 A. In particular, preferable solvents are propylene glycol monomethyl ether acetate, propylene glycol monomethyl ether, 2-heptanone, cyclopentanone, cyclohexanone, and mixtures of two or more thereof.

The solvent (C) is preferably contained in an amount of 200 to 10,000 parts by mass, more preferably 300 to 5,000 parts based on 100 parts by mass of the resin and/or compound (A) for forming an organic film. When the amount is in such a range, the concentration is adjustable in accordance with the desired film thickness.

Furthermore, the inventive composition for forming an organic film may also contain, as an organic solvent, a high-boiling-point solvent, having a boiling point of 180°C or higher, in addition to the above-described solvent having a boiling point of lower than 180°C (a mixture of a solvent having a boiling point of lower than 180°C and a solvent having a boiling point of 180°C or higher). The high-boiling-point organic solvent is not particularly limited to hydrocarbons, alcohols, ketones, esters, ethers, or chlorine-based solvents as long as the solvent is capable of dissolving the compound for forming an organic film. Specific examples include 1-octanol, 2-ethylhexanol, 1-nonanol, 1-decanol, 1-undecanol, ethylene glycol, 1,2-propylene glycol, 1,3-butylene glycol, 2,4-pentanediol, 2-methyl-2,4-pentanediol, 2,5-hexanediol, 2,4-heptanediol, 2-ethyl-1,3-hexanediol, diethylene glycol, dipropylene glycol, triethylene glycol, tripropylene glycol, glycerin, n-nonyl acetate, ethylene glycol monohexyl ether, ethylene glycol mono-2-ethylhexyl ether, ethylene glycol monophenyl ether, ethylene glycol monobenzyl ether, diethylene glycol monoethyl ether, diethylene glycol monoisopropyl ether, diethylene glycol mono-n-butyl ether, diethylene glycol monoisobutyl ether, diethylene glycol monohexyl ether, diethylene glycol monophenyl ether, diethylene glycol monobenzyl ether, diethylene glycol diethyl ether, diethylene glycol dibutyl ether, diethylene glycol butyl methyl ether, triethylene glycol dimethyl ether, triethylene glycol monomethyl ether, triethylene glycol-n-butyl ether, triethylene glycol butyl methyl ether, triethylene glycol diacetate, tetraethylene glycol dimethyl ether, dipropylene glycol monomethyl ether, dipropylene glycol mono-n-propyl ether, dipropylene glycol mono-n-butyl ether, tripropylene glycol dimethyl ether, tripropylene glycol monomethyl ether, tripropylene glycol mono-n-propyl ether, tripropylene glycol mono-n-butyl ether, ethylene glycol monoethyl ether acetate, ethylene glycol monobutyl ether acetate, diethylene glycol monomethyl ether acetate, diethylene glycol monoethyl ether acetate, diethylene glycol monobutyl ether acetate, triacetin, propylene glycol diacetate, dipropylene glycol monomethyl ether acetate, dipropylene glycol methyl-n-propyl ether, dipropylene glycol methyl ether acetate, 1,4-butanediol diacetate, 1,3-butylene glycol diacetate, 1,6-hexanediol diacetate, γ-butyrolactone, dihexyl malonate, diethyl succinate, dipropyl succinate, dibutyl succinate, dihexyl succinate, dimethyl adipate, diethyl adipate, dibutyl adipate, and the like. One of the solvents may be used or a mixture of two or more kinds may be used.

The boiling point of the high-boiling-point solvent can be selected appropriately in accordance with the temperature at which the material for forming an organic film is heated, and the boiling point of the high-boiling-point solvent to be contained is preferably 180°C to 300°C, further preferably 200°C to 300°C. When the boiling point is as described, sufficient thermal flowability can be achieved, since there is no risk of excessive evaporation rate at the baking (heating) due to the boiling point being too low. Moreover, a solvent having such a boiling point does not remain in the film without evaporating even after the baking due to the boiling point being too high. Therefore, there is no risk of the solvent adversely affecting the physical properties, such as etching resistance, of the film.

Furthermore, when the high-boiling-point solvent is used, the amount to be blended is preferably 1 to 30 parts by mass per 100 parts by mass of the solvent having a boiling point of lower than 180°C. When the contained amount is as described, there are no risks that sufficient thermal flowability cannot be imparted at the time of baking due to the contained amount being too small, or that the solvent remains in the film and leads to degradation of physical properties, such as etching resistance, of the film due to the contained amount being too large.

The above-described composition for forming an organic film is provided with both high filling property and high planarizing property by thermal flowability being imparted to the resin and/or compound for forming an organic film by virtue of the high-boiling-point solvent being contained.

### [Other Components]

In addition, the inventive composition for forming an organic film can contain an acid generator or a crosslinking agent for further promoting the crosslinking reaction. Acid generators include materials that generate an acid by thermal decomposition and materials that generate an acid by light irradiation, and any acid generator can be contained. Specific examples of the acid generator include those disclosed in paragraphs [0061] to [0085] of JP 2007-199653 A. One kind of the acid generator may be used, or two or more kinds thereof may be used in combination. When the acid generator is contained, the contained amount is preferably 0.05 to 50 parts by mass, more preferably 0.1 to 10 parts by mass based on 100 parts by mass of the resin and/or compound (A) for forming an organic film. When the amount is as described, it is possible to promote the crosslinking reaction and form a dense film.

Specific examples of the crosslinking agent include those disclosed in paragraphs [0055] to [0060] of JP 2007-199653 A. One kind of the crosslinking agent may be used, or two or more kinds thereof may be used in combination. The crosslinking agent is preferably contained in an amount of 1 to 100 parts by mass, more preferably 5 to 50 parts by mass based on 100 parts by mass of the resin and/or compound (A) for forming an organic film. When the amount is as described, it is possible to enhance curability and further suppress intermixing with an upper layer film.

The inventive composition for forming an organic film can also contain a surfactant other than the polymer (B) of the present invention, in order to further improve in-plane uniformity in spin-coating. Specific examples of the surfactant include those disclosed in paragraphs [0142] to [0147] of JP 2009-269953 A. One kind of the surfactant may be used, or two or more kinds thereof may be used in combination. When the surfactant is contained, the contained amount is preferably 0.01 to 10 parts by mass, more preferably 0.05 to 5 parts by mass based on 100 parts by mass of the resin and/or compound for forming an organic film. When the amount is as described, it is possible to form an organic film excellent in in-plane uniformity.

The inventive composition for forming an organic film can further contain a basic compound for enhancing storage stability. The basic compound serves as a quencher to an acid to prevent a very small amount of acid generated by the acid generator from promoting the crosslinking reaction. Specific examples of such a basic compound include those disclosed in paragraphs [0086] to [0090] of JP 2007-199653 A. One kind of the basic compound may be used, or two or more kinds thereof may be used in combination. When the basic compound is contained, the contained amount is preferably 0.05 to 50 parts by mass, more preferably 0.1 to 10 parts by mass based on 100 parts by mass of the resin and/or compound (A) for forming an organic film. When the amount is as described, it is possible to improve the storage stability of the composition for forming an organic film.

As described above, the inventive composition for forming an organic film is excellent in hump suppression at the time of an EBR process. Accordingly, the inventive composition for forming an organic film is extremely useful as a resist underlayer film material (organic film material) for multilayer resist processes such as two-layer resist processes, three-layer resist processes using a silicon-containing resist middle layer film or a silicon-containing inorganic hard mask middle layer film, and four-layer resist processes using a silicon-containing resist middle layer film or silicon-containing inorganic hard mask middle layer film and an organic antireflective film or adhesive film.

### [Method for Forming Organic Film]

The present invention provides a method for forming an organic film to be used in a manufacturing process of a semiconductor device, the method including:
spin-coating a substrate to be processed with the inventive composition for forming an organic film described above; and
forming an organic film by heating the substrate to be processed coated with the composition for forming an organic film at a temperature of 100°C or higher and 600°C or lower for 10 to 600 seconds for curing.

In this method for forming an organic film, firstly, the inventive composition for forming an organic film described above is applied onto a substrate to be processed by spin-coating. An excellent filling property can be obtained by using a spin-coating method. After removing a film on an edge portion in an EBR process, baking (heating) is performed in order to promote the crosslinking reaction. Incidentally, the solvent in the composition can be evaporated by this baking, and therefore, mixing can be prevented even when a resist upper layer film or a silicon-containing resist middle layer film is formed on the organic film.

The baking is performed at a temperature of 100°C or higher and 600°C or lower for 10 to 600 seconds, preferably at a temperature of 200°C or higher and 500°C or lower for 10 to 300 seconds. Considering influence on device damage and wafer deformation, the upper limit of the heating temperature in a wafer process of lithography is preferably 600°C or lower, more preferably 500°C or lower. By performing the heat treatment under such conditions, the crosslinking reaction can be promoted, and it is possible to form an organic film that does not mix with a film formed thereon.

### [Patterning Process]

In the following, patterning processes using the inventive composition for forming an organic film will be described.

### [Three-Layer Resist Process Using Silicon-Containing Resist Middle Layer Film]

The present invention provides a patterning process including:
forming an organic film on a body to be processed by using the above-described composition for forming an organic film;
forming a silicon-containing resist middle layer film on the organic film by using a silicon-containing resist middle layer film material;
forming a resist upper layer film on the silicon-containing resist middle layer film by using a resist upper layer film material including a photoresist composition;
forming a circuit pattern in the resist upper layer film;
transferring the pattern to the silicon-containing resist middle layer film by etching while using the resist upper layer film having the formed circuit pattern as a mask;
transferring the pattern to the organic film by etching while using the silicon-containing resist middle layer film having the transferred pattern as a mask; and
further transferring the pattern to the body to be processed by etching while using the organic film having the transferred pattern as a mask.

The body to be processed is preferably a semiconductor device substrate or the semiconductor device substrate coated with any of a metal film, a metal carbide film, a metal oxide film, a metal nitride film, a metal oxycarbide film, and a metal oxynitride film. The body to be processed is not particularly limited, but more specifically, may be a substrate made of Si, α-Si, p-Si, SiO₂, SiN, SiON, W, TiN, Al, etc.; the substrate coated with the above-mentioned metal film or the like as a layer to be processed; etc.

Examples of the layer to be processed include various Low-k films made of Si, SiO₂, SiON, SiN, p-Si, α-Si, W, W-Si, Al, Cu, Al-Si, or the like; and stopper films thereof. Generally, the layer can be formed to have a thickness of 50 to 10,000 nm, particularly preferably 100 to 5,000 nm. Note that when the layer to be processed is formed, the substrate and the layer to be processed are formed from different materials.

Incidentally, the metal constituting the body to be processed is preferably silicon, titanium, tungsten, hafnium, zirconium, chromium, germanium, copper, silver, gold, aluminum, indium, gallium, arsenic, palladium, iron, tantalum, iridium, molybdenum, or an alloy thereof.

When forming an organic film on the body to be processed by using the inventive composition for forming an organic film, the inventive method for forming an organic film described above can be adopted.

Subsequently, a resist middle layer film (silicon-containing resist middle layer film) is formed on the organic film by using a resist middle layer film material containing a silicon atom. As the resist middle layer film material containing a silicon atom, a polysiloxane-based middle layer film material is preferable. By imparting an antireflective effect to the silicon-containing resist middle layer film, reflection can be suppressed. Particularly, for 193-nm light exposure, when a material containing many aromatic groups and having high etching selectivity relative to the substrate is used as a composition for forming an organic film, the k-value and thus the substrate reflection are increased; in contrast, the reflection can be suppressed by imparting absorption to the silicon-containing resist middle layer film so as to have an appropriate k-value, and the substrate reflection can be reduced to 0.5% or less. As the silicon-containing resist middle layer film having an antireflective effect, a polysiloxane is preferably used which has anthracene for 248-nm or 157-nm light exposure, or a phenyl group or a light-absorbing group having a silicon-silicon bond for 193-nm light exposure in a pendant structure, and which is to be crosslinked by an acid or heat.

Subsequently, a resist upper layer film is formed on the silicon-containing resist middle layer film by using a resist upper layer film material containing a photoresist composition. The resist upper layer film material may be a positive type or a negative type, and a photoresist composition that is normally used can be used. After the spin-coating with the resist upper layer film material, pre-baking is preferably performed at 60 to 180°C for 10 to 300 seconds. Then, light exposure, post-exposure baking (PEB), and development are performed according to conventional methods to obtain a resist upper layer film pattern. Note that the thickness of the resist upper layer film is not particularly limited, but is preferably 30 to 500 nm, particularly preferably 50 to 400 nm.

Subsequently, a circuit pattern (resist upper layer film pattern) is formed in the resist upper layer film. The circuit pattern is preferably formed by a lithography using light having a wavelength of 10 nm or more and 300 nm or less, a direct writing with electron beam, nanoimprinting, or a combination thereof.

Examples of exposure light include high-energy beams having a wavelength of 300 nm or less, specifically, deep ultraviolet ray, KrF excimer laser beam (248 nm), ArF excimer laser beam (193 nm), F₂ laser beam (157 nm), Kr₂ laser beam (146 nm), Ar₂ laser beam (126 nm), soft X-ray (EUV) of 3 to 20 nm, electron beam (EB), ion beam, X-ray, etc.

Furthermore, the circuit pattern is preferably developed with an alkaline development or an organic solvent.

Subsequently, the pattern is transferred to the silicon-containing resist middle layer film by etching while using the resist upper layer film having the formed circuit pattern as a mask. The etching of the silicon-containing resist middle layer film performed while using the resist upper layer film pattern as a mask is preferably performed by using a fluorocarbon-based gas. Thus, the pattern is transferred to the silicon-containing resist middle layer film.

Subsequently, the pattern is transferred to the organic film by etching while using the silicon-containing resist middle layer film having the transferred pattern as a mask. The silicon-containing resist middle layer film exhibits etching resistance to oxygen gas or hydrogen gas, and therefore, the etching of the organic film performed while using the silicon-containing resist middle layer film pattern as a mask is preferably performed by using an etching gas mainly containing oxygen gas or hydrogen gas. Thus, the pattern is transferred to the organic film.

Subsequently, the pattern is transferred to the body to be processed by etching while using the organic film having the transferred pattern as a mask. This etching of the body to be processed (layer to be processed) can be performed by a conventional method. For example, a body to be processed made of SiO₂, SiN, or silica low-dielectric insulating film is etched mainly with a fluorocarbon-based gas; and a body to be processed made of p-Si, Al, or W is etched mainly with a chlorine- or bromine-based gas. When the substrate is processed by etching with a fluorocarbon-based gas, the silicon-containing resist middle layer film pattern is removed when the substrate is processed. Meanwhile, when the substrate is processed by etching with a chlorine- or bromine-based gas, the silicon-containing resist middle layer film pattern needs to be removed by additional dry etching with a fluorocarbon-based gas after the processing of the substrate.

The organic film obtained by using the inventive composition for forming an organic film can be provided with excellent etching resistance when the body to be processed is etched as described above.

### [Four-Layer Resist Process Using Silicon-Containing Resist Middle Layer Film and Organic Antireflective Film or Adhesive Film]

The present invention also provides a patterning process including:
forming an organic film on a body to be processed by using the above-described composition for forming an organic film;
forming a silicon-containing resist middle layer film on the organic film by using a silicon-containing resist middle layer film material;
forming an organic antireflective film or an adhesive film on the silicon-containing resist middle layer film;
forming a resist upper layer film on the organic antireflective film or the adhesive film by using a resist upper layer film material containing a photoresist composition;
forming a circuit pattern in the resist upper layer film;
transferring the pattern to the organic antireflective film or the adhesive film and to the silicon-containing resist middle layer film by etching while using the resist upper layer film having the formed circuit pattern as a mask;
transferring the pattern to the organic film by etching while using the silicon-containing resist middle layer film having the transferred pattern as a mask; and
further transferring the pattern to the body to be processed by etching while using the organic film having the transferred pattern as a mask.

This method can be performed in the same manner as the three-layer resist process using the silicon-containing resist middle layer film, except that the organic antireflective film (BARC) or the adhesive film is formed between the silicon-containing resist middle layer film and the resist upper layer film.

The organic antireflective film and the adhesive film can be formed by spin-coating by using a known organic antireflective film material.

### [Three-Layer Resist Process Using Inorganic Hard Mask Middle Layer Film]

The present invention also provides a patterning process including:
forming an organic film on a body to be processed by using the above-described composition for forming an organic film;
forming an inorganic hard mask middle layer film selected from the group consisting of a silicon oxide film, a silicon nitride film, and a silicon oxynitride film on the organic film;
forming a resist upper layer film on the inorganic hard mask middle layer film by using a resist upper layer film material containing a photoresist composition;
forming a circuit pattern in the resist upper layer film;
transferring the pattern to the inorganic hard mask middle layer film by etching while using the resist upper layer film having the formed circuit pattern as a mask;
transferring the pattern to the organic film by etching while using the inorganic hard mask middle layer film having the transferred pattern as a mask; and
further transferring the pattern to the body to be processed by etching while using the organic film having the transferred pattern as a mask.

This method can be performed in the same manner as the three-layer resist process using the silicon-containing resist middle layer film, except that the inorganic hard mask middle layer film is formed instead of the silicon-containing resist middle layer film on the organic film.

The inorganic hard mask middle layer film selected from a silicon oxide film, a silicon nitride film, and a silicon oxynitride film (SiON film) can be formed by a CVD method, an ALD method, etc. Examples of methods for forming the silicon nitride film are disclosed in JP 2002-334869 A, WO 2004/066377 A1, etc. The inorganic hard mask middle layer film preferably has a thickness of 5 to 200 nm, more preferably 10 to 100 nm. As the inorganic hard mask middle layer film, a SiON film, which has a high function as an antireflective film, is the most preferably used.

### [Four-Layer Resist Process Using Inorganic Hard Mask Middle Layer Film and Organic Antireflective Film or Adhesive Film]

The present invention also provides a patterning process including:
forming an organic film on a body to be processed by using the above-described composition for forming an organic film;
forming an inorganic hard mask middle layer film selected from the group consisting of a silicon oxide film, a silicon nitride film, and a silicon oxynitride film on the organic film;
forming an organic antireflective film or an adhesive film on the inorganic hard mask middle layer film;
forming a resist upper layer film on the organic antireflective film or the adhesive film by using a resist upper layer film material containing a photoresist composition;
forming a circuit pattern in the resist upper layer film;
transferring the pattern to the organic antireflective film or the adhesive film and to the inorganic hard mask middle layer film by etching while using the resist upper layer film having the formed circuit pattern as a mask;
transferring the pattern to the organic film by etching while using the inorganic hard mask middle layer film having the transferred pattern as a mask; and
further transferring the pattern to the body to be processed by etching while using the organic film having the transferred pattern as a mask.

This method can be performed in the same manner as the three-layer resist process using the inorganic hard mask middle layer film, except that the organic antireflective film (BARC) or the adhesive film is formed between the inorganic hard mask middle layer film and the resist upper layer film.

Particularly, when a SiON film is used as the inorganic hard mask middle layer film, two antireflective films including the SiON film and the BARC make it possible to suppress the reflection even in liquid immersion exposure at a high NA exceeding 1.0. Another merit of forming the BARC is having an effect of reducing trailing of the resist upper layer film pattern immediately above the SiON film.

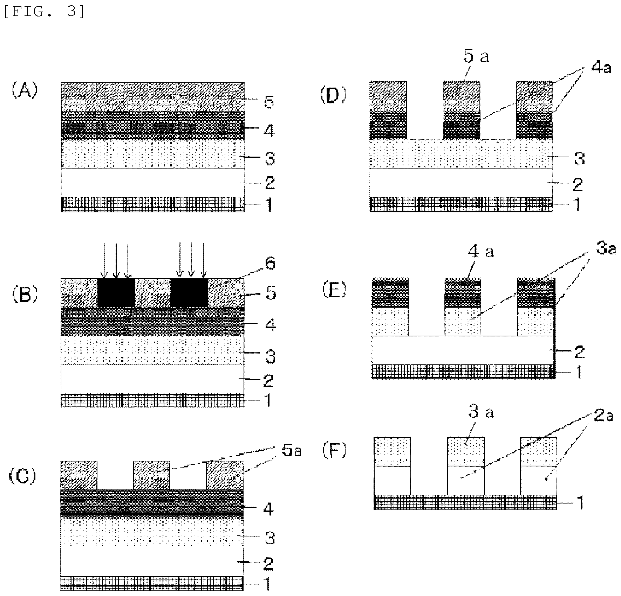

Here, an example of the inventive patterning processes according to a three-layer resist process is shown in FIG. 3 (A) to (F). In the case of a three-layer resist process, as shown in FIG. 3 (A), an organic film 3 is formed by using the inventive composition for forming an organic film on a layer 2 to be processed formed on a substrate 1; then, a silicon-containing resist middle layer film 4 is formed; and a resist upper layer film 5 is formed thereon. Next, as shown in FIG. 3 (B), an exposure portion 6 of the resist upper layer film 5 is exposed, and then PEB (post-exposure baking) is performed. Next, as shown in FIG. 3 (C), development is performed to form a resist upper layer film pattern 5a. Next, as shown in FIG. 3 (D), the silicon-containing resist middle layer film 4 is dry-etched by using a fluorocarbon-based gas while using the resist upper layer film pattern 5a as a mask, and thus, a silicon-containing resist middle layer film pattern 4a is formed. Next, as shown in FIG. 3 (E), the resist upper layer film pattern 5a is removed, then, the organic film 3 is etched with oxygen plasma while using the silicon-containing resist middle layer film pattern 4a as a mask, and thus, an organic film pattern 3a is formed. Furthermore, as shown in FIG. 3 (F), the silicon-containing resist middle layer film pattern 4a is removed, then the layer 2 to be processed is etched while using the organic film pattern 3a as a mask, and thus, a pattern 2a is formed. By humps being suppressed when the organic film is formed, during the dry etching of the drawings (D), (E), and (F), it is possible to reduce defects that are generated due to humps of the organic film.

In a case where an inorganic hard mask middle layer film is formed, the silicon-containing resist middle layer film 4 can be changed to an inorganic hard mask middle layer film, and in a case where a BARC or an adhesive film is formed, the BARC or the adhesive film can be formed between the silicon-containing resist middle layer film 4 and the resist upper layer film 5. The etching of the BARC or the adhesive film may be performed continuously before the etching of the silicon-containing resist middle layer film 4; alternatively, after the BARC or the adhesive film alone is etched, the etching apparatus may be changed and so forth, and then the etching of the silicon-containing resist middle layer film 4 may be performed.

As described above, the inventive patterning processes make it possible to form a fine pattern in a body to be processed with high precision according to a multilayer resist process, and to suppress hump formation on an organic film, thus reducing defects derived from humps of the organic film.

### EXAMPLES

Hereinafter, the present invention will be further specifically described with reference to Synthesis Examples, Comparative Synthesis Examples, Examples, and Comparative Examples. However, the present invention is not limited thereto. Note that, specifically, the molecular weight was measured in the following manner. Weight-average molecular weight (Mw) and number-average molecular weight (Mn) on polystyrene basis were measured by gel permeation chromatography (GPC) using tetrahydrofuran as an eluent (solvent), and dispersity (Mw/Mn) was calculated therefrom.

### [Synthesis of Monomers (M1) to (M12)]

For the synthesis of compounds (M1) to (M12), used for the preparation of compositions for forming an organic film, the following epoxy compounds (E1) to (E3) and hydroxy-group-containing compounds (alcohol compound or carboxylic acid compound) (F1) to (F8) were used.

### Epoxy Compounds

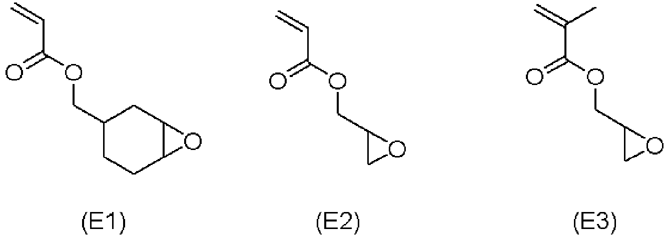

### Hydroxy-Group-Containing Compounds (Phenol or Carboxylic Acid Compounds)

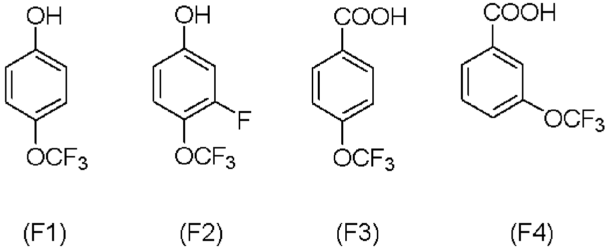

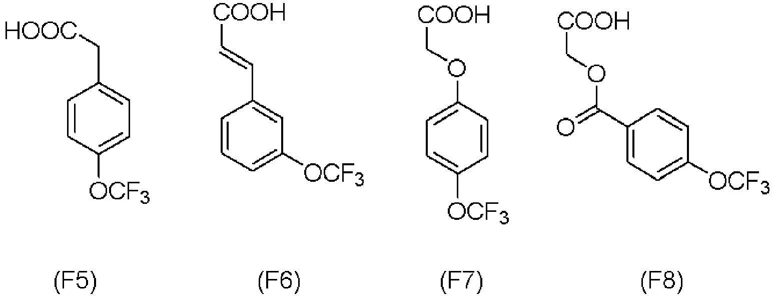

### [Synthesis Example 1] Synthesis of (M1)

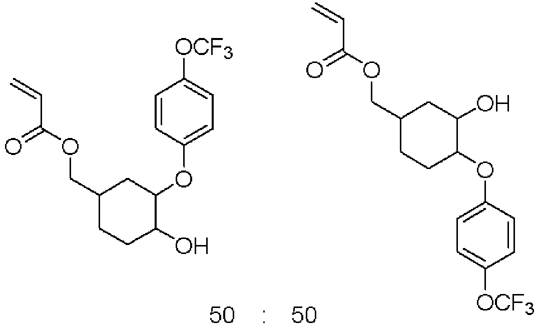

Under a nitrogen atmosphere, 50.0 g (274.4 mmol) of the epoxy compound (E1), 48.9 g (274.4 mmol) of the hydroxy-group-containing compound (F1), 2.0 g (8.8 mmol) of benzyltriethylammonium chloride, and 200 g of 2-methoxy-1-propanol were added together to form a homogeneous solution at an internal temperature of 80°C, and then the solution was further stirred at an internal temperature of 100°C for 24 hours. After cooling to room temperature, 500 ml of diisopropyl ether was added thereto, and washing was performed twice with 100 g of a 3% aqueous solution of NaHCO₃, once with 100 g of ultrapure water and 100 g of a 3% aqueous solution of nitric acid, and five times with 100 g of ultrapure water in this order. Then, the organic layer was evaporated under reduced pressure to dryness to obtain a monomer (M1). The isomer ratio calculated by LC (liquid chromatography) was 50:50.

Monomers (M2) to (M12) were obtained as products under the same reaction conditions as in Synthesis Example 1, except that the epoxy compound and the hydroxy-group-containing compound were changed to those shown in Table 1. The synthesis method (Synthesis Example 1) used for the synthesis of the compounds is also shown in the table. Note that, when a hydroxy-group-containing compound was used, the reaction was performed using a small excess of a carboxylic acid relative to the epoxy compound to improve the reaction efficiency.

**[Table 1]**

| Synthesis Example | Monomer | Epoxy compound | Used amount (g) | Hydroxy-group-containing compound | Used amount (g) |
|---|---|---|---|---|---|
| 1 | M1 | E1 | 50.0 | F1 | 48.9 |
| 2 | M2 | E1 | 50.0 | F2 | 53.8 |
| 3 | M3 | E1 | 50.0 | F3 | 65.3 |
| 4 | M4 | E1 | 30.0 | F7 | 44.9 |
| 5 | M5 | E2 | 30.0 | F3 | 55.7 |
| 6 | M6 | E2 | 30.0 | F4 | 55.7 |
| 7 | M7 | E2 | 30.0 | F6 | 62.8 |
| 8 | M8 | E2 | 30.0 | F7 | 56.6 |
| 9 | M9 | E3 | 50.0 | F1 | 62.6 |
| 10 | M10 | E3 | 30.0 | F3 | 50.2 |
| 11 | M11 | E3 | 30.0 | F5 | 53.7 |
| 12 | M12 | E3 | 30.0 | F8 | 64.4 |

The structural formula and isomer ratio of the monomers (M1) to (M12) obtained in Synthesis Examples 1 to 12 are shown below.

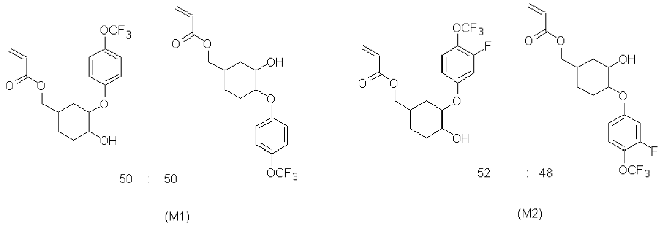

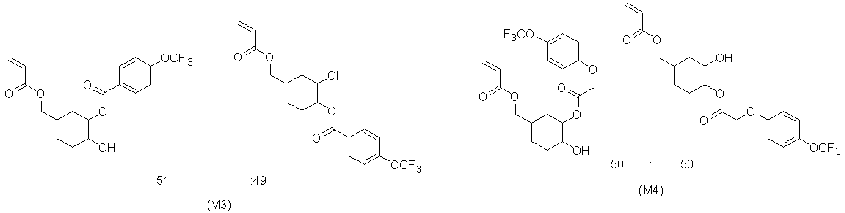

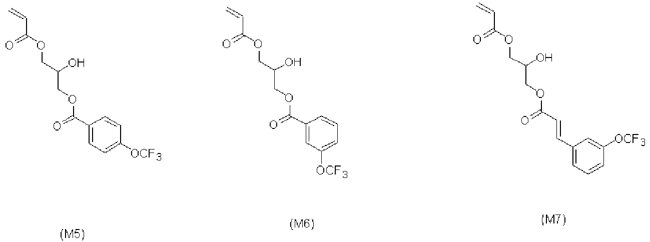

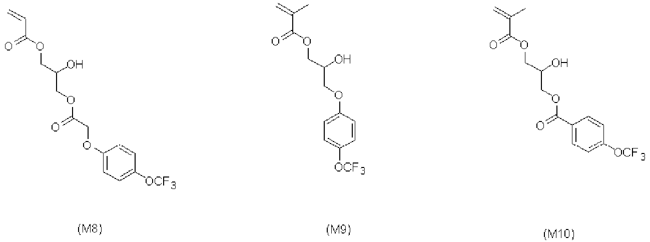

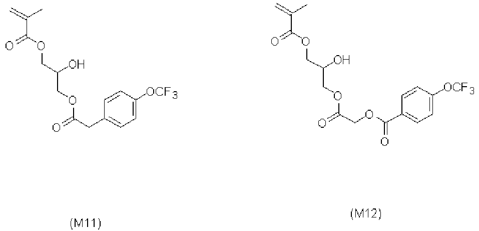

For the synthesis of polymers (A1) to (A19) to be used as polymers for materials for forming an organic film, (M1) to (M12), synthesized in the Synthesis Examples, and (m-1) to (m-3), shown below, were used as the monomers shown below.

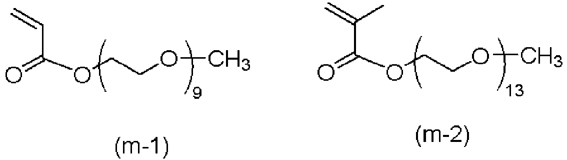

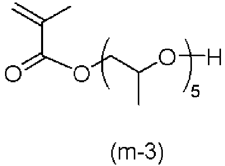

### [Synthesis Example 13] Synthesis of Polymer (A1)

10.0 g of propylene glycol monomethyl ether acetate (PGMEA) was heated and stirred under a nitrogen atmosphere at 80°C. A mixture of 3.60 g (15.0 mmol) of the monomer (M1), 0.173 g (0.75 mmol) of dimethyl 2,2-azobis(2-methylpropionate), and 15 g of PGMEA was added dropwise thereto over 4 hours. The mixture was further heated and stirred for 16 hours, and then cooled to room temperature. Thus, a solution of the target polymer (A1) in PGMEA was obtained. As a result of analysis, it was found that the polymer (A1) had a weight-average molecular weight (Mw) of 12900 and a dispersity (Mw/Mn) of 1.45.

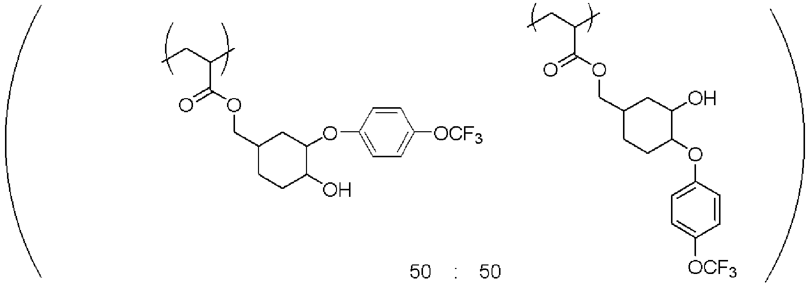

### [Synthesis Examples 14 to 31] Polymers (A2) to (A19)

The polymers (A2) to (A19) shown below were obtained in the same manner as in Synthesis Example 13, except that the kind and molar ratio of the monomers used were changed according to the structure of each polymer. In addition, the weight-average molecular weight (Mw) and dispersity (Mw/Mn) determined by GPC are also shown.

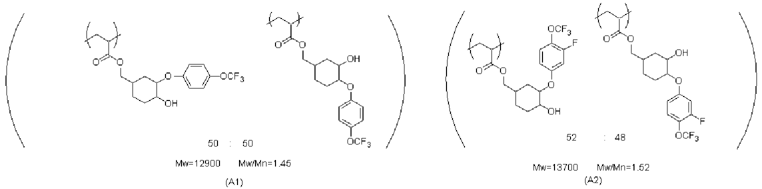

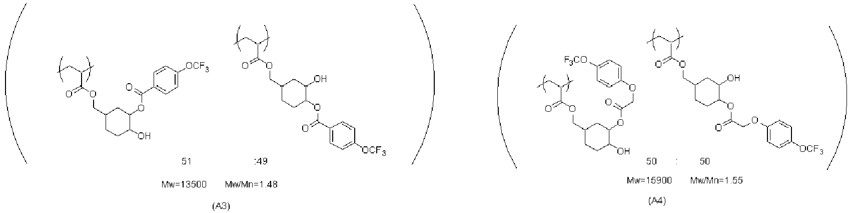

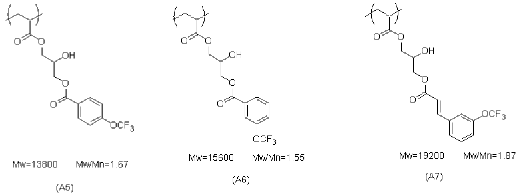

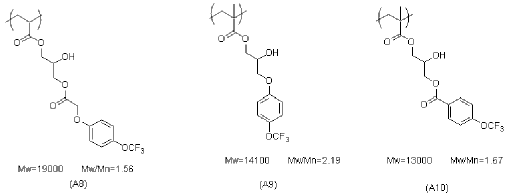

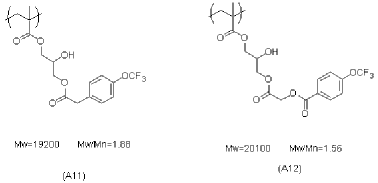

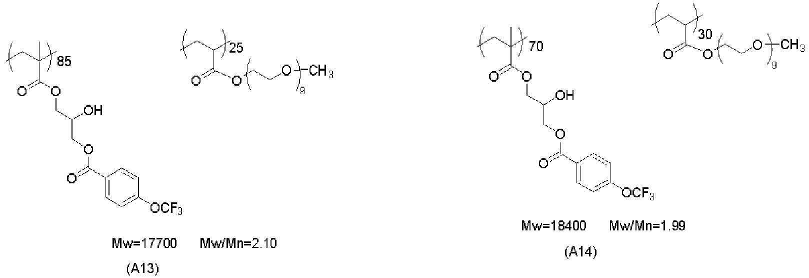

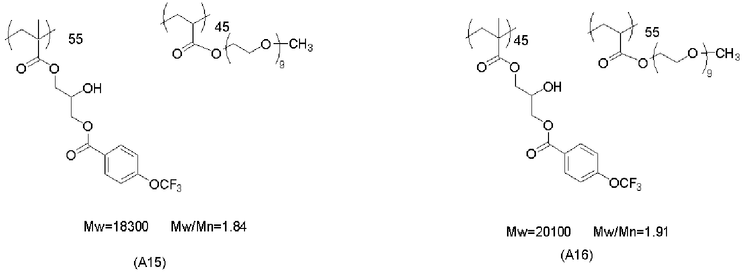

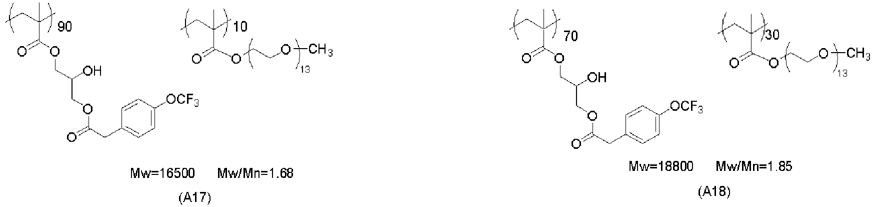

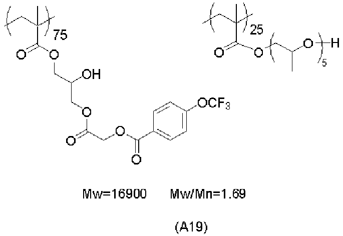

### [Synthesis Example 32] Synthesis of Polymer (A20)

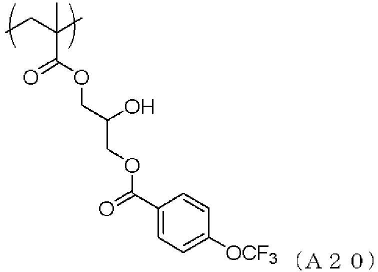

10.0 g of PGMEA was heated and stirred under a nitrogen atmosphere at 80°C. A mixture of 2.13 g (15.0 mmol) of glycidyl methacrylate, 0.103 g (0.45 mmol) of dimethyl 2,2-azobis(2-methylpropionate), and 10 g of PGMEA was added dropwise thereto over 4 hours. The mixture was further heated and stirred for 16 hours, and then cooled to room temperature. Then, 10.0 g of propylene glycol monomethyl ether (PGME), 3.24 g of (15.75 mmol) of 4-(trifluoromethoxy)benzoic acid, and 0.17 g (0.75 mmol) of benzyltriethylammonium chloride were added thereto. Under a nitrogen atmosphere, the mixture was heated at 100°C for 16 hours. After cooling to room temperature, 100 ml of methyl isobutyl ketone was added thereto, and washing was performed twice with 30 g of a 3% aqueous solution of NaHCO₃, once with 30 g of ultrapure water and 30 g of a 3% aqueous solution of nitric acid, and five times with 30 g of ultrapure water in this order. Then, the organic layer was evaporated under reduced pressure to dryness, PGMEA was added, and furthermore, concentration and solvent exchange were performed to obtain a polymer (A20) as a 30 wt% solution of the polymer. As a result of analysis, it was found that the polymer (A20) had a weight-average molecular weight (Mw) of 24100 and a dispersity (Mw/Mn) of 2.25.

### [Synthesis Example 33] Synthesis of Polymer (A21)

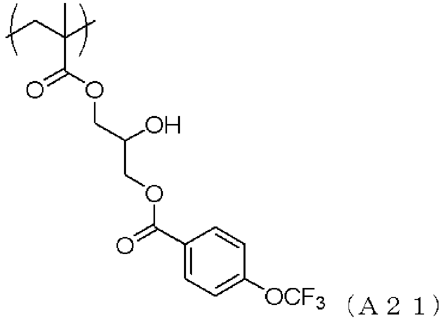

10.0 g of PGMEA was heated and stirred under a nitrogen atmosphere at 80°C. A mixture of 2.13 g (15.0 mmol) of glycidyl methacrylate, 0.069 g (0.30mmol) of dimethyl 2,2-azobis(2-methylpropionate), and 10 g of PGMEA was added dropwise thereto over 4 hours. The mixture was further heated and stirred for 16 hours, and then cooled to room temperature. Then, 10.0 g of PGME, 3.24 g (15.75 mol) of 4-(trifluoromethoxy)benzoic acid, and 0.17 g (0.75 mmol) of benzyltriethylammonium chloride were added thereto. Under a nitrogen atmosphere, the mixture was heated at 100°C for 16 hours. After cooling to room temperature, 100 ml of methyl isobutyl ketone was added thereto, and washing was performed twice with 30 g of a 3% aqueous solution of NaHCO₃, once with 30 g of ultrapure water and 30 g of a 3% aqueous solution of nitric acid, and five times with 30 g of ultrapure water in this order. Then, the organic layer was evaporated under reduced pressure to dryness, PGMEA was added, and furthermore, concentration and solvent exchange were performed to obtain a polymer (A21) as a 30 wt% solution of the polymer. As a result of analysis, it was found that the polymer (A21) had a weight-average molecular weight (Mw) of 31200 and a dispersity (Mw/Mn) of 2.87.

### [Synthesis Example 34] Synthesis of Polymer (A22)

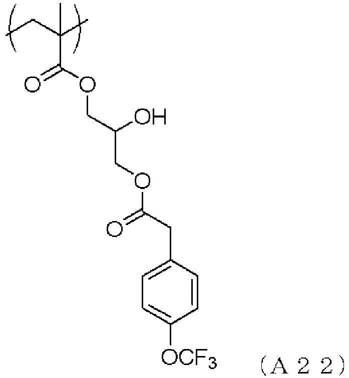

10.0 g of PGMEA was heated and stirred under a nitrogen atmosphere at 80°C. A mixture of 2.13 g (15.0 mol) of glycidyl methacrylate, 0.345 g (1.5 mmol) of dimethyl 2,2-azobis(2-methylpropionate), and 10 g of PGMEA was added dropwise thereto over 4 hours. The mixture was further heated and stirred for 16 hours, and then cooled to room temperature. Then, 10.0 g of PGME, 3.47 g (15.75 mmol) of 4-(trifluoromethoxy)phenylacetic acid, and 0.17 g (0.75 mmol) of benzyltriethylammonium chloride were added thereto. Under a nitrogen atmosphere, the mixture was heated at 100°C for 16 hours. After cooling to room temperature, 100 ml of methyl isobutyl ketone was added thereto, and washing was performed twice with 30 g of a 3% aqueous solution of NaHCO₃, once with 30 g of ultrapure water and 30 g of a 3% aqueous solution of nitric acid, and five times with 30 g of ultrapure water in this order. Then, the organic layer was evaporated under reduced pressure to dryness, PGMEA was added, and furthermore, concentration and solvent exchange were performed to obtain a polymer (A22) as a 30 wt% solution of the polymer. As a result of analysis, it was found that the polymer (A22) had a weight-average molecular weight (Mw) of 6800 and a dispersity (Mw/Mn) of 2.57.

### [Synthesis Example 35] Synthesis of Polymer (A23)

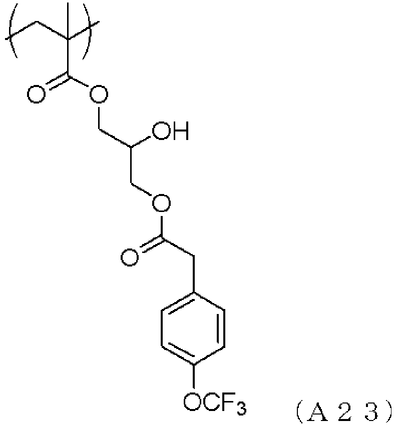

10.0 g of PGMEA was heated and stirred under a nitrogen atmosphere at 80°C. A mixture of 2.13 g (15.0 mol) of glycidyl methacrylate, 0.518 g (22.5 mmol) of dimethyl 2,2-azobis(2-methylpropionate), and 10.0 g of PGMEA was added dropwise thereto over 4 hours. The mixture was further heated and stirred for 16 hours, and then cooled to room temperature. Then, 10.0 g of PGME, 3.47 g (15.75 mmol) of 4-(trifluoromethoxy)phenylacetic acid, and 0.17 g (0.75 mmol) of benzyltriethylammonium chloride were added thereto. Under a nitrogen atmosphere, the mixture was heated at 100°C for 16 hours. After cooling to room temperature, 100 ml of methyl isobutyl ketone was added thereto, and washing was performed twice with 30 g of a 3% aqueous solution of NaHCO₃, once with 30 g of ultrapure water and 30 g of a 3% aqueous solution of nitric acid, and five times with 30 g of ultrapure water in this order. Then, the organic layer was evaporated under reduced pressure to dryness, PGMEA was added, and furthermore, concentration and solvent exchange were performed to obtain a polymer (A23) as a 30 wt% solution of the polymer. As a result of analysis, it was found that the polymer (A23) had a weight-average molecular weight (Mw) of 4800 and a dispersity (Mw/Mn) of 2.87.

### [Synthesis of Comparative Polymers (R1) to (R9)]

For the synthesis of comparative polymers (R1) to (R9), used for the preparation of compositions for forming an organic film, the following monomers (b1) to (b10) were used.

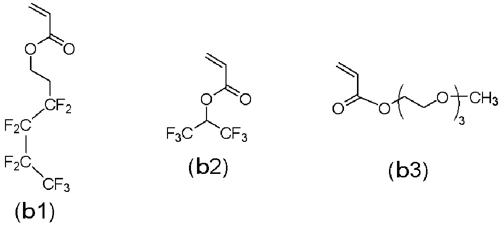

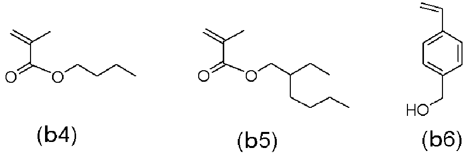

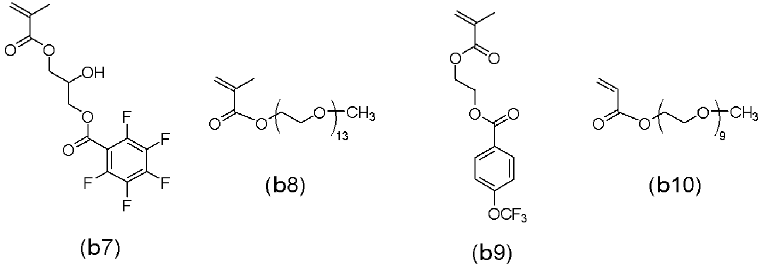

### [Comparative Synthesis Example 1] Synthesis of Comparative Polymer (R1)

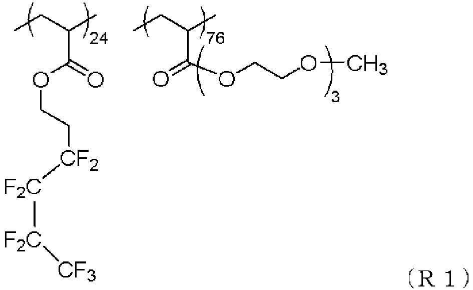

Under a nitrogen atmosphere, 6.0 g of PGMEA was heated and stirred at 80°C. A mixture of 3.44 g (0.011 mol) of the monomer (b1), 7.46 g (0.034 mol) of the monomer (b3), 0.473 g of dimethyl 2,2-azobis(2-methylpropionate), and 34 g of PGMEA was added dropwise thereto over 4 hours. The mixture was further heated and stirred for 16 hours, and then cooled to room temperature to obtain a solution of the target polymer (R1) in PGMEA.

The weight-average molecular weight (Mw) and the dispersity (Mw/Mn) were determined by GPC, and the following results were obtained.
(R1): Mw=9500, Mw/Mn=1.20

### [Comparative Synthesis Example 2] Synthesis of Comparative Polymer (R2)

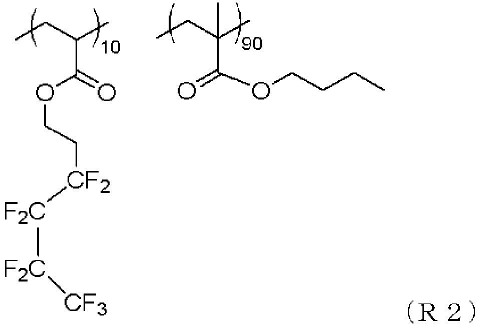

Under a nitrogen atmosphere, 6.0 g of PGMEA was heated and stirred at 80°C. A mixture of 1.43 g (0.005 mol) of the monomer (b1), 5.76 g (0.041 mol) of the monomer (b4), 0.473 g of dimethyl 2,2-azobis(2-methylpropionate), and 34 g of PGMEA was added dropwise thereto over 4 hours. The mixture was further heated and stirred for 16 hours, and then cooled to room temperature to obtain a solution of the target polymer (R2) in PGMEA.

The weight-average molecular weight (Mw) and the dispersity (Mw/Mn) were determined by GPC, and the following results were obtained.
(R2): Mw=5800, Mw/Mn=1.42

### [Comparative Synthesis Example 3] Synthesis of Comparative Polymer (R3)

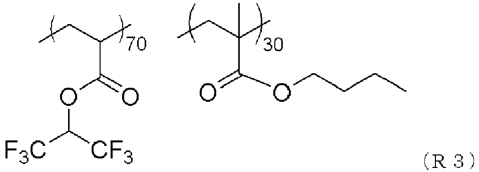

Under a nitrogen atmosphere, 6.0 g of PGMEA was heated and stirred at 80°C. A mixture of 7.00 g (0.032 mol) of the monomer (b2), 1.92 g (0.014 mol) of the monomer (b3), 0.473 g of dimethyl 2,2-azobis(2-methylpropionate), and 34 g of PGMEA was added dropwise thereto over 4 hours. The mixture was further heated and stirred for 16 hours, and then cooled to room temperature to obtain a solution of the target polymer (R3) in PGMEA.

The weight-average molecular weight (Mw) and the dispersity (Mw/Mn) were determined by GPC, and the following results were obtained.
(R3): Mw=6200, Mw/Mn=1.33

### [Comparative Synthesis Example 4] Synthesis of Comparative Polymer (R4)

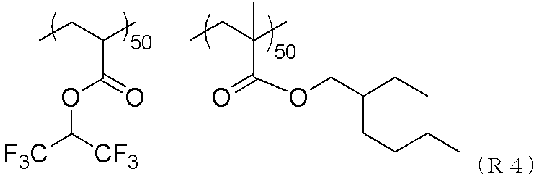

Under a nitrogen atmosphere, 6.0 g of PGMEA was heated and stirred at 80°C. A mixture of 5.00 g (0.023 mol) of (b2), 3.20 g (0.023 mol) of (b5), 0.473 g of dimethyl 2,2-azobis(2-methylpropionate), and 34 g of PGMEA was added dropwise thereto over 4 hours. The mixture was further heated and stirred for 16 hours, and then cooled to room temperature to obtain a solution of the target polymer (R4) in PGMEA.

The weight-average molecular weight (Mw) and the dispersity (Mw/Mn) were determined by GPC, and the following results were obtained.
(R4): Mw=8300, Mw/Mn=1.33

### [Comparative Synthesis Example 5] Synthesis of Comparative Polymer (R5)

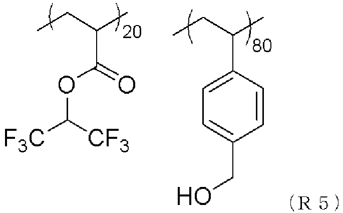

Under a nitrogen atmosphere, 6.0 g of PGMEA was heated and stirred at 80°C. A mixture of 2.00 g (0.009 mol) of (b2), 5.41 g (0.036 mol) of (b6), 0.473 g of dimethyl 2,2-azobis(2-methylpropionate), and 34 g of PGMEA was added dropwise thereto over 4 hours. The mixture was further heated and stirred for 16 hours, and then cooled to room temperature to obtain a solution of the target polymer (R5) in PGMEA.

The weight-average molecular weight (Mw) and the dispersity (Mw/Mn) were determined by GPC, and the following results were obtained.
(R5): Mw=3800, Mw/Mn=1.44

### [Comparative Synthesis Example 6] Synthesis of Comparative Polymer (R6)

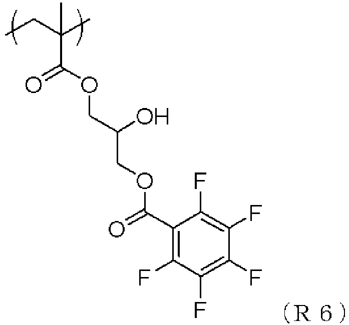

Under a nitrogen atmosphere, 6.0 g of PGMEA was heated and stirred at 80°C. A mixture of 5.31 g (15.0 mmol) of the monomer (b7), 0.173 g (0.75 mmol) of dimethyl 2,2-azobis(2-methylpropionate), and 34 g of PGMEA was added dropwise thereto over 4 hours. The mixture was further heated and stirred for 16 hours, and then cooled to room temperature to obtain a solution of the target polymer (R6) in PGMEA.

The weight-average molecular weight (Mw) and the dispersity (Mw/Mn) were determined by GPC, and the following results were obtained.
(R6): Mw=12000, Mw/Mn=1.48

### [Comparative Synthesis Example 7] Synthesis of Comparative Polymer (R7)

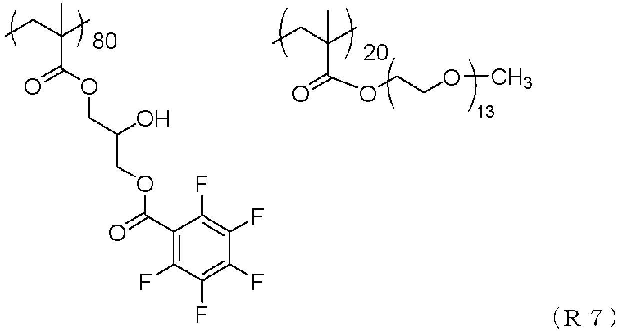

Under a nitrogen atmosphere, 6.0 g of PGMEA was heated and stirred at 80°C. A mixture of 4.25 g (12.0 mmol) of the monomer (b7), 2.02 g (3.0 mmol) of the monomer (b8), 0.173 g (0.75 mmol) of dimethyl 2,2-azobis(2-methylpropionate), and 34 g of PGMEA was added dropwise thereto over 4 hours. The mixture was further heated and stirred for 16 hours, and then cooled to room temperature to obtain a solution of the target polymer (R7) in PGMEA.

The weight-average molecular weight (Mw) and the dispersity (Mw/Mn) were determined by GPC, and the following results were obtained.
(R7): Mw=10400, Mw/Mn=1.50

### [Comparative Synthesis Example 8] Synthesis of Comparative Polymer (R8)

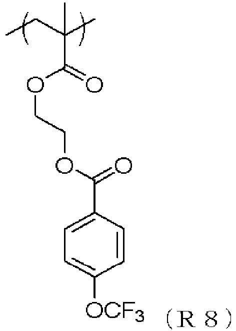

Under a nitrogen atmosphere, 10.0 g of PGMEA was heated and stirred at 80°C. A mixture of 4.77 g (15.0 mmol) of the monomer (b9), 0.173 g (0.75 mmol) of dimethyl 2,2-azobis(2-methylpropionate), and 10.0 g of PGMEA was added dropwise thereto over 4 hours. The mixture was further heated and stirred for 16 hours, and then cooled to room temperature to obtain a solution of the target polymer (R8) in PGMEA.

The weight-average molecular weight (Mw) and the dispersity (Mw/Mn) were determined by GPC, and the following results were obtained.
(R8): Mw=11500, Mw/Mn=1.94

### [Comparative Synthesis Example 9] Synthesis of Comparative Polymer (R9)

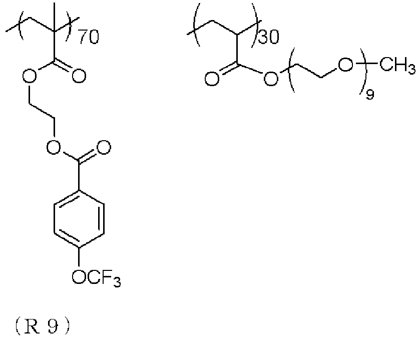

Under a nitrogen atmosphere, 10.0 g of PGMEA was heated and stirred at 80°C. A mixture of 3.34 g (10.5 mmol) of the monomer (b9), 2.17 g (4.5 mmol) of the monomer (b10), 0.173 g (0.75 mmol) of dimethyl 2,2-azobis(2-methylpropionate), and 10.0 g of PGMEA was added dropwise thereto over 4 hours. The mixture was further heated and stirred for 16 hours, and then cooled to room temperature to obtain a solution of the target polymer (R9) in PGMEA.

The weight-average molecular weight (Mw) and the dispersity (Mw/Mn) were determined by GPC, and the following results were obtained.
(R9): Mw=12500, Mw/Mn=1.92

### [Resin or Compound for Forming Organic Film]

C1: a resin represented by the following formula (C1)
C2: a resin represented by the following formula (C2)
C3: a compound represented by the following formula (C3)
C4: a compound represented by the following formula (C4)
C5: a resin represented by the following formula (C5)
C6: a resin represented by the following formula (C6)

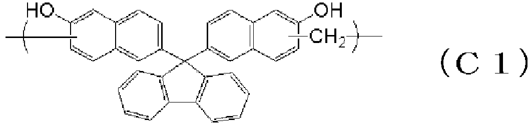

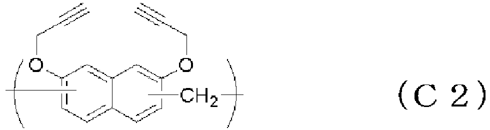

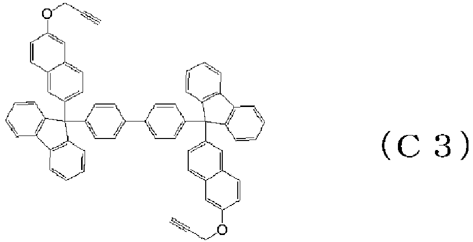

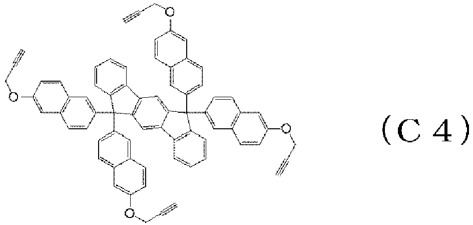

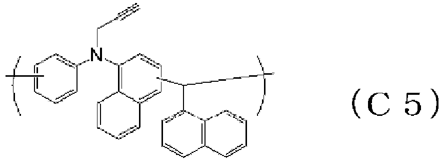

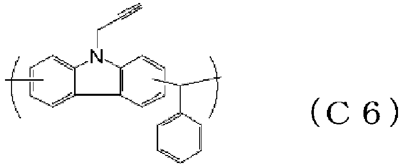

### [Solvent]

(D1): propylene glycol monomethyl ether acetate
(D2): propylene glycol monoethyl ether

### [Preparation of Compositions (UDL-1 to -79 and Comparative UDL-1 to -20) for Forming Organic Film]

One of the polymers (A1) to (A23) and (R1) to (R9), one of the resins or compounds (C1) to (C6) for forming an organic film, and one or both of the solvents were dissolved in the proportions shown in Tables 2 to 5. The mixture was filtered through a 0.1-µm filter made of a fluorinated resin to prepare each of compositions (resist underlayer film materials: UDL-1 to -79 and comparative UDL-1 to -20) for forming an organic film.

**[Table 2]**

| Composition for forming organic film | Resin or compound for forming organic film | | Polymer or comparative polymer | | Solvent 1 | | Solvent 2 | |
|---|---|---|---|---|---|---|---|---|
| | Type | Parts by mass | Type | Parts by mass | Type | Parts by mass | Type | Parts by mass |
| UDL-1 | (C1) | 10.00 | (A1) | 0.10 | (D1) | 89.90 | | |
| UDL-2 | (C1) | 10.00 | (A2) | 0.10 | (D1) | 89.90 | | |
| UDL-3 | (C1) | 10.00 | (A3) | 0.10 | (D1) | 89.90 | | |
| UDL-4 | (C1) | 10.00 | (A4) | 0.10 | (D1) | 89.90 | | |
| UDL-5 | (C1) | 10.00 | (A5) | 0.10 | (D1) | 89.90 | | |
| UDL-6 | (C1) | 10.00 | (A6) | 0.10 | (D1) | 89.90 | | |
| UDL-7 | (C1) | 10.00 | (A7) | 0.10 | (D1) | 89.90 | | |
| UDL-8 | (C1) | 10.00 | (A8) | 0.10 | (D1) | 89.90 | | |
| UDL-9 | (C1) | 10.00 | (A9) | 0.10 | (D1) | 89.90 | | |
| UDL-10 | (C1) | 10.00 | (A10) | 0.10 | (D1) | 89.90 | | |
| UDL-11 | (C1) | 10.00 | (A11) | 0.10 | (D1) | 89.90 | | |
| UDL-12 | (C1) | 10.00 | (A12) | 0.10 | (D1) | 89.90 | | |
| UDL-13 | (C1) | 10.00 | (A13) | 0.10 | (D1) | 89.90 | | |
| UDL-14 | (C1) | 10.00 | (A14) | 0.10 | (D1) | 89.90 | | |
| UDL-15 | (C1) | 10.00 | (A15) | 0.10 | (D1) | 89.90 | | |
| UDL-16 | (C1) | 10.00 | (A16) | 0.10 | (D1) | 89.90 | | |
| UDL-17 | (C1) | 10.00 | (A17) | 0.10 | (D1) | 89.90 | | |
| UDL-18 | (C1) | 10.00 | (A18) | 0.10 | (D1) | 89.90 | | |
| UDL-19 | (C1) | 10.000 | (A19) | 0.10 | (D1) | 89.90 | | |
| UDL-20 | (C1) | 10.00 | (A20) | 0.10 | (D1) | 89.90 | | |
| UDL-21 | (C1) | 10.00 | (A21) | 0.10 | (D1) | 89.90 | | |
| UDL-22 | (C1) | 10.00 | (A22) | 0.10 | (D1) | 89.90 | | |
| UDL-23 | (C1) | 10.00 | (A23) | 0.10 | (D1) | 89.90 | | |
| UDL-24 | (C1) | 10.00 | (A10) | 0.10 | (D1) | 62.93 | (D2) | 26.97 |
| UDL-25 | (C1) | 10.00 | (A11) | 0.10 | (D1) | 62.93 | (D2) | 26.97 |
| UDL-26 | (C1) | 10.00 | (A12) | 0.10 | (D1) | 62.93 | (D2) | 26.97 |
| UDL-27 | (C1) | 10.00 | (A15) | 0.10 | (D1) | 62.93 | (D2) | 26.97 |
| UDL-28 | (C1) | 10.00 | (A18) | 0.10 | (D1) | 62.93 | (D2) | 26.97 |
| UDL-29 | (C1) | 10.00 | (A18) | 1.000 | (D1) | 89.00 | | |

**[Table 3]**

| Composition for forming organic film | Resin or compound for forming organic film | | Polymer or comparative polymer | | Solvent 1 | | Solvent 2 | |
|---|---|---|---|---|---|---|---|---|
| | Type | Parts by mass | Type | Parts by mass | Type | Parts by mass | Type | Parts by mass |
| UDL-30 | (C1) | 10.00 | (A18) | 0.500 | (D1) | 89.50 | | |
| UDL-31 | (C1) | 10.00 | (A18) | 0.001 | (D1) | 90.00 | | |
| UDL-32 | (C2) | 10.00 | (A10) | 0.10 | (D1) | 89.90 | | |
| UDL-33 | (C2) | 10.00 | (A11) | 0.10 | (D1) | 89.90 | | |
| UDL-34 | (C2) | 10.00 | (A12) | 0.10 | (D1) | 89.90 | | |
| UDL-35 | (C2) | 10.00 | (A15) | 0.10 | (D1) | 89.90 | | |
| UDL-36 | (C2) | 10.00 | (A18) | 0.10 | (D1) | 89.90 | | |
| UDL-37 | (C3) | 10.00 | (A10) | 0.10 | (D1) | 89.90 | | |
| UDL-38 | (C3) | 10.00 | (A11) | 0.10 | (D1) | 89.90 | | |
| UDL-39 | (C3) | 10.00 | (A12) | 0.10 | (D1) | 89.90 | | |
| UDL-40 | (C3) | 10.00 | (A15) | 0.10 | (D1) | 89.90 | | |
| UDL-41 | (C3) | 10.00 | (A18) | 0.10 | (D1) | 89.90 | | |
| UDL-42 | (C4) | 10.00 | (A10) | 0.10 | (D1) | 89.90 | | |
| UDL-43 | (C4) | 10.00 | (A11) | 0.10 | (D1) | 89.90 | | |
| UDL-44 | (C4) | 10.00 | (A12) | 0.10 | (D1) | 89.90 | | |
| UDL-45 | (C4) | 10.00 | (A15) | 0.10 | (D1) | 89.90 | | |
| UDL-46 | (C4) | 10.00 | (A18) | 0.10 | (D1) | 89.90 | | |
| UDL-47 | (C5) | 10.00 | (A10) | 0.10 | (D1) | 89.90 | | |
| UDL-48 | (C5) | 10.00 | (A11) | 0.10 | (D1) | 89.90 | | |
| UDL-49 | (C5) | 10.00 | (A12) | 0.10 | (D1) | 89.90 | | |
| UDL-50 | (C5) | 10.00 | (A15) | 0.10 | (D1) | 89.90 | | |
| UDL-51 | (C5) | 10.00 | (A18) | 0.10 | (D1) | 89.90 | | |
| UDL-52 | (C6) | 10.00 | (A10) | 0.10 | (D1) | 89.90 | | |
| UDL-53 | (C6) | 10.00 | (A11) | 0.10 | (D1) | 89.90 | | |
| UDL-54 | (C6) | 10.00 | (A12) | 0.10 | (D1) | 89.90 | | |
| UDL-55 | (C6) | 10.00 | (A15) | 0.10 | (D1) | 89.90 | | |
| UDL-56 | (C6) | 10.00 | (A18) | 0.10 | (D1) | 89.90 | | |

**[Table 4]**

| Composition for forming organic film | Resin or compound for forming organic film | | Polymer or comparative polymer | | Solvent 1 | | Solvent 2 | |
|---|---|---|---|---|---|---|---|---|
| | Type | Parts by mass | Type | Parts by mass | Type | Parts by mass | Type | Parts by mass |
| UDL-57 | (C1) | 25.00 | (A1) | 0.25 | (D1) | 74.75 | | |
| UDL-58 | (C1) | 25.00 | (A2) | 0.25 | (D1) | 74.75 | | |
| UDL-59 | (C1) | 25.00 | (A3) | 0.25 | (D1) | 74.75 | | |
| UDL-60 | (C1) | 25.00 | (A4) | 0.25 | (D1) | 74.75 | | |
| UDL-61 | (C1) | 25.00 | (A5) | 0.25 | (D1) | 74.75 | | |
| UDL-62 | (C1) | 25.00 | (A6) | 0.25 | (D1) | 74.75 | | |
| UDL-63 | (C1) | 25.00 | (A7) | 0.25 | (D1) | 74.75 | | |
| UDL-64 | (C1) | 25.00 | (A8) | 0.25 | (D1) | 74.75 | | |
| UDL-65 | (C1) | 25.00 | (A9) | 0.25 | (D1) | 74.75 | | |
| UDL-66 | (C1) | 25.00 | (A10) | 0.25 | (D1) | 74.75 | | |
| UDL-67 | (C1) | 25.00 | (A11) | 0.25 | (D1) | 74.75 | | |
| UDL-68 | (C1) | 25.00 | (A12) | 0.25 | (D1) | 74.75 | | |
| UDL-69 | (C1) | 25.00 | (A13) | 0.25 | (D1) | 74.75 | | |
| UDL-70 | (C1) | 25.00 | (A14) | 0.25 | (D1) | 74.75 | | |
| UDL-71 | (C1) | 25.00 | (A15) | 0.25 | (D1) | 74.75 | | |
| UDL-72 | (C1) | 25.00 | (A16) | 0.25 | (D1) | 74.75 | | |
| UDL-73 | (C1) | 25.00 | (A17) | 0.25 | (D1) | 74.75 | | |
| UDL-74 | (C1) | 25.00 | (A18) | 0.25 | (D1) | 74.75 | | |
| UDL-75 | (C1) | 25.00 | (A19) | 0.25 | (D1) | 74.75 | | |
| UDL-76 | (C1) | 25.00 | (A20) | 0.25 | (D1) | 74.75 | | |
| UDL-77 | (C1) | 25.00 | (A21) | 0.25 | (D1) | 74.75 | | |
| UDL-78 | (C1) | 25.00 | (A22) | 0.25 | (D1) | 74.75 | | |
| UDL-79 | (C1) | 25.00 | (A23) | 0.25 | (D1) | 74.75 | | |

**[Table 5]**

| Composition for forming organic film | Resin or compound for forming organic film | | Polymer or comparative polymer | | Solvent 1 | | Solvent 2 | |
|---|---|---|---|---|---|---|---|---|
| | Type | Parts by mass | Type | Parts by mass | Type | Parts by mass | Type | Parts by mass |
| Comparative UDL-1 | (C1) | 10.00 | (R1) | 0.10 | (D1) | 89.90 | | |
| Comparative UDL-2 | (C2) | 10.00 | (R1) | 0.10 | (D1) | 89.90 | | |
| Comparative UDL-3 | (C3) | 10.00 | (R1) | 0.10 | (D1) | 89.90 | | |
| Comparative UDL-4 | (C4) | 10.00 | (R1) | 0.10 | (D1) | 89.90 | | |
| Comparative UDL-5 | (C5) | 10.00 | (R1) | 0.10 | (D1) | 89.90 | | |
| Comparative UDL-6 | (C6) | 10.00 | (R1) | 0.10 | (D1) | 89.90 | | |
| Comparative UDL-7 | (C1) | 10.00 | (R2) | 0.10 | (D1) | 89.90 | | |
| Comparative UDL-8 | (C1) | 10.00 | (R3) | 0.10 | (D1) | 89.90 | | |
| Comparative UDL-9 | (C1) | 10.00 | (R4) | 0.10 | (D1) | 89.90 | | |
| Comparative UDL-10 | (C1) | 10.00 | (R5) | 0.10 | (D1) | 89.90 | | |
| Comparative UDL-11 | (C1) | 10.00 | (R6) | 0.10 | (D1) | 89.90 | | |
| Comparative UDL-12 | (C1) | 10.00 | (R7) | 0.10 | (D1) | 89.90 | | |
| Comparative UDL-13 | (C1) | 10.00 | (R8) | 0.10 | (D1) | 89.90 | | |
| Comparative UDL-14 | (C1) | 10.00 | (R9) | 0.10 | (D1) | 89.90 | | |
| Comparative UDL-15 | (C1) | 10.00 | | | (D1) | 90.00 | | |
| Comparative UDL-16 | (C2) | 10.00 | | | (D1) | 90.00 | | |
| Comparative UDL-17 | (C3) | 10.00 | | | (D1) | 90.00 | | |
| Comparative UDL-18 | (C4) | 10.00 | | | (D1) | 90.00 | | |
| Comparative UDL-19 | (C5) | 10.00 | | | (D1) | 90.00 | | |
| Comparative UDL-20 | (C6) | 10.00 | | | (D1) | 90.00 | | |

### [Preparation of Silicon Wafers Having Organic Cured Film Formed Thereon by Using Compositions (UDL-1 to -79 and Comparative UDL-1 to -20) for Forming Organic Film]

Using a coater/developer "CLEAN TRACK LITHIUS Pro AP" of Tokyo Electron Ltd., each of the compositions (UDL-1 to -79 and comparative UDL-1 to -20) for forming an organic film prepared above was respectively discharged in an amount of 2 ml onto a 300-mm silicon wafer at the center. Then, the wafer was rotated at a rotational rate to achieve the average film thickness shown in Tables 6 to 9 after baking to spread the composition. Thus, a film was formed. While rotating the silicon wafer at a rate of 1000 rpm, the remover-discharging nozzle was moved at a speed of 5 mm/s from the periphery of the silicon wafer to a position 3 mm from the center while discharging a remover (mixed solution of propylene glycol monomethyl ether acetate and propylene glycol monomethyl ether (30:70, mass ratio)) at a discharge rate of 2 ml/s, and the remover was further discharged at that position for 5 seconds at a discharge rate of 2 ml/s. After that, the discharging of the discharged liquid was terminated, and the silicon wafer was further rotated at a rate of 1000 rpm for 30 seconds. Next, the silicon wafer on which the film of the composition for forming an organic film had been formed was heated at 350°C for 60 seconds. Thus, a silicon wafer having an organic cured film formed thereon was obtained.

### [Solvent Resistance Evaluation: Examples 1-1 to 1-79 and Comparative Examples 1-1 to 1-20]

A film of each of the compositions (UDL-1 to -79 and comparative UDL-1 to -20) for forming an organic film was respectively formed on a silicon wafer according to the above-described method, and the thickness of the film was measured. Subsequently, a PGMEA solvent was dispensed on the film, left to stand for 30 seconds, spin-dried, and baked at 100°C for 60 seconds to evaporate the PGMEA, and then the film thickness was measured. The absolute value of the value determined by (X₁-X)/X × 100 was obtained as a film thickness change rate (%), where X is the film thickness before dispensing the PGMEA solvent, and X₁ is the film thickness after the PGMEA solvent was dispensed. When the film thickness change rate was lower than 0.5%, the composition was evaluated as "Good", and when 0.5% or higher, "Poor".

### [In-Plane Uniformity Evaluation: Examples 1-1 to 1-79 and Comparative Examples 1-1 to 1-20]

A film of each of the compositions (UDL-1 to -79 and comparative UDL-1 to -20) for forming an organic film was respectively formed on a silicon wafer according to the above-described method, and the film thickness within a radius of 145 mm from the center of the organic cured film was measured. The value determined by (Xₘₐₓ-Xₘᵢₙ)/X_{average} was obtained as in-plane uniformity (%), where Xₘₐₓ is the maximum value of the film thickness, Xₘᵢₙ is the minimum value of the film thickness, and X_{average} is the average film thickness. When the in-plane uniformity was less than 2%, the composition was evaluated as A (good), when 2% or more to less than 3%, B, and when 3% or more, C (poor).

### [Contact Angle Evaluation: Examples 1-1 to 1-79, Comparative Examples 1-1 to 1-10, and Comparative Examples 1-13 and 1-14]

A film of each of the compositions (UDL-1 to -79, comparative UDL-1 to -10, and comparative UDL-13 and - 14) for forming an organic film was respectively formed on a silicon wafer according to the above-described method, and the contact angle with pure water was measured. Regarding comparative UDL-11 and -12 and comparative UDL-15 to -20, the measurement was not carried out since in-plane uniformity was poor.

**[Table 6]**

| | Composition for forming organic film | Average film thickness | Solvent resistance | In-plane uniformity | Contact angle |
|---|---|---|---|---|---|
| Example 1-1 | UDL-1 | 500 nm | Good | B | 67° |
| Example 1-2 | UDL-2 | 500 nm | Good | B | 67° |
| Example 1-3 | UDL-3 | 500 nm | Good | A | 68° |
| Example 1-4 | UDL-4 | 500 nm | Good | B | 67° |
| Example 1-5 | UDL-5 | 500 nm | Good | A | 65° |
| Example 1-6 | UDL-6 | 500 nm | Good | B | 63° |
| Example 1-7 | UDL-7 | 500 nm | Good | B | 63° |
| Example 1-8 | UDL-8 | 500 nm | Good | B | 64° |
| Example 1-9 | UDL-9 | 500 nm | Good | B | 63° |
| Example 1-10 | UDL-10 | 500 nm | Good | A | 65° |
| Example 1-11 | UDL-11 | 500 nm | Good | A | 64° |
| Example 1-12 | UDL-12 | 500 nm | Good | A | 63° |
| Example 1-13 | UDL-13 | 500 nm | Good | A | 65° |
| Example 1-14 | UDL-14 | 500 nm | Good | A | 64° |
| Example 1-15 | UDL-15 | 500 nm | Good | A | 64° |
| Example 1-16 | UDL-16 | 500 nm | Good | A | 64° |
| Example 1-17 | UDL-17 | 500 nm | Good | A | 63° |
| Example 1-18 | UDL-18 | 500 nm | Good | A | 63° |
| Example 1-19 | UDL-19 | 500 nm | Good | A | 64° |
| Example 1-20 | UDL-20 | 500 nm | Good | A | 65° |
| Example 1-21 | UDL-21 | 500 nm | Good | B | 65° |
| Example 1-22 | UDL-22 | 500 nm | Good | A | 64° |
| Example 1-23 | UDL-23 | 500 nm | Good | B | 65° |
| Example 1-24 | UDL-24 | 500 nm | Good | A | 65° |
| Example 1-25 | UDL-25 | 500 nm | Good | A | 64° |
| Example 1-26 | UDL-26 | 500 nm | Good | A | 63° |
| Example 1-27 | UDL-27 | 500 nm | Good | A | 64° |
| Example 1-28 | UDL-28 | 500 nm | Good | A | 63° |
| Example 1-29 | UDL-29 | 500 nm | Good | B | 63° |

**[Table 7]**

| | Composition for forming organic film | Average film thickness | Solvent resistance | In-plane uniformity | Contact angle |
|---|---|---|---|---|---|
| Example 1-30 | UDL-30 | 500 nm | Good | A | 63° |
| Example 1-31 | UDL-31 | 500 nm | Good | B | 65° |
| Example 1-32 | UDL-32 | 500 nm | Good | A | 65° |
| Example 1-33 | UDL-33 | 500 nm | Good | A | 64° |
| Example 1-34 | UDL-34 | 500 nm | Good | A | 65° |
| Example 1-35 | UDL-35 | 500 nm | Good | A | 64° |
| Example 1-36 | UDL-36 | 500 nm | Good | A | 63° |
| Example 1-37 | UDL-37 | 500 nm | Good | A | 65° |
| Example 1-38 | UDL-38 | 500 nm | Good | A | 64° |
| Example 1-39 | UDL-39 | 500 nm | Good | A | 65° |
| Example 1-40 | UDL-40 | 500 nm | Good | A | 64° |
| Example 1-41 | UDL-41 | 500 nm | Good | A | 63° |
| Example 1-42 | UDL-42 | 500 nm | Good | A | 65° |
| Example 1-43 | UDL-43 | 500 nm | Good | A | 64° |
| Example 1-44 | UDL-44 | 500 nm | Good | A | 65° |
| Example 1-45 | UDL-45 | 500 nm | Good | A | 64° |
| Example 1-46 | UDL-46 | 500 nm | Good | A | 63° |
| Example 1-47 | UDL-47 | 500 nm | Good | A | 65° |
| Example 1-48 | UDL-48 | 500 nm | Good | A | 64° |
| Example 1-49 | UDL-49 | 500 nm | Good | A | 65° |
| Example 1-50 | UDL-50 | 500 nm | Good | A | 64° |
| Example 1-51 | UDL-51 | 500 nm | Good | A | 63° |
| Example 1-52 | UDL-52 | 500 nm | Good | A | 65° |
| Example 1-53 | UDL-53 | 500 nm | Good | A | 64° |
| Example 1-54 | UDL-54 | 500 nm | Good | A | 65° |
| Example 1-55 | UDL-55 | 500 nm | Good | A | 64° |
| Example 1-56 | UDL-56 | 500 nm | Good | A | 63° |

**[Table 8]**

| | Composition for forming organic film | Average film thickness | Solvent resistance | In-plane uniformity | Contact angle |
|---|---|---|---|---|---|
| Example 1-57 | UDL-57 | 1000 nm | Good | B | 67° |
| Example 1-58 | UDL-58 | 1000 nm | Good | B | 67° |
| Example 1-59 | UDL-59 | 1000 nm | Good | A | 68° |
| Example 1-60 | UDL-60 | 1000 nm | Good | B | 67° |
| Example 1-61 | UDL-61 | 1000 nm | Good | A | 65° |
| Example 1-62 | UDL-62 | 1000 nm | Good | B | 63° |
| Example 1-63 | UDL-63 | 1000 nm | Good | B | 63° |
| Example 1-64 | UDL-64 | 1000 nm | Good | B | 64° |
| Example 1-65 | UDL-65 | 1000 nm | Good | B | 63° |
| Example 1-66 | UDL-66 | 1000 nm | Good | A | 65° |
| Example 1-67 | UDL-67 | 1000 nm | Good | A | 64° |
| Example 1-68 | UDL-68 | 1000 nm | Good | A | 63° |
| Example 1-69 | UDL-69 | 1000 nm | Good | A | 65° |
| Example 1-70 | UDL-70 | 1000 nm | Good | A | 64° |
| Example 1-71 | UDL-71 | 1000 nm | Good | A | 64° |
| Example 1-72 | UDL-72 | 1000 nm | Good | B | 64° |
| Example 1-73 | UDL-73 | 1000 nm | Good | A | 63° |
| Example 1-74 | UDL-74 | 1000 nm | Good | A | 63° |
| Example 1-75 | UDL-75 | 1000 nm | Good | A | 64° |
| Example 1-76 | UDL-76 | 1000 nm | Good | B | 65° |
| Example 1-77 | UDL-77 | 1000 nm | Good | B | 65° |
| Example 1-78 | UDL-78 | 1000 nm | Good | A | 64° |
| Example 1-79 | UDL-79 | 1000 nm | Good | B | 65° |

**[Table 9]**

| | Composition for forming organic film | Average film thickness | Solvent resistance | In-plane uniformity | Contact angle |
|---|---|---|---|---|---|
| Comparative Example 1-1 | Comparative UDL-1 | 500 nm | Good | A | 75° |
| Comparative Example 1-2 | Comparative UDL-2 | 500 nm | Good | A | 75° |
| Comparative Example 1-3 | Comparative UDL-3 | 500 nm | Good | A | 75° |
| Comparative Example 1-4 | Comparative UDL-4 | 500 nm | Good | A | 75° |
| Comparative Example 1-5 | Comparative UDL-5 | 500 nm | Good | A | 75° |
| Comparative Example 1-6 | Comparative UDL-6 | 500 nm | Good | A | 75° |
| Comparative Example 1-7 | Comparative UDL-7 | 500 nm | Good | A | 90° |
| Comparative Example 1-8 | Comparative UDL-8 | 500 nm | Good | A | 93° |
| Comparative Example 1-9 | Comparative UDL-9 | 500 nm | Good | A | 95° |
| Comparative Example 1-10 | Comparative UDL-10 | 500 nm | Good | A | 74° |
| Comparative Example 1-11 | Comparative UDL-11 | 500 nm | Good | C | - |
| Comparative Example 1-12 | Comparative UDL-12 | 500 nm | Good | C | - |
| Comparative Example 1-13 | Comparative UDL-13 | 500 nm | Good | A | 80° |
| Comparative Example 1-14 | Comparative UDL-14 | 500 nm | Good | A | 76° |
| Comparative Example 1-15 | Comparative UDL-15 | 500 nm | Good | C | - |
| Comparative Example 1-16 | Comparative UDL-16 | 500 nm | Good | C | - |
| Comparative Example 1-17 | Comparative UDL-17 | 500 nm | Good | C | - |
| Comparative Example 1-18 | Comparative UDL-18 | 500 nm | Good | C | - |
| Comparative Example 1-19 | Comparative UDL-19 | 500 nm | Good | C | - |
| Comparative Example 1-20 | Comparative UDL-20 | 500 nm | Good | C | - |

### [Hump Suppression Property Evaluation: Examples 2-1 to 2-79 and Comparative Examples 2-1 to 2-12]

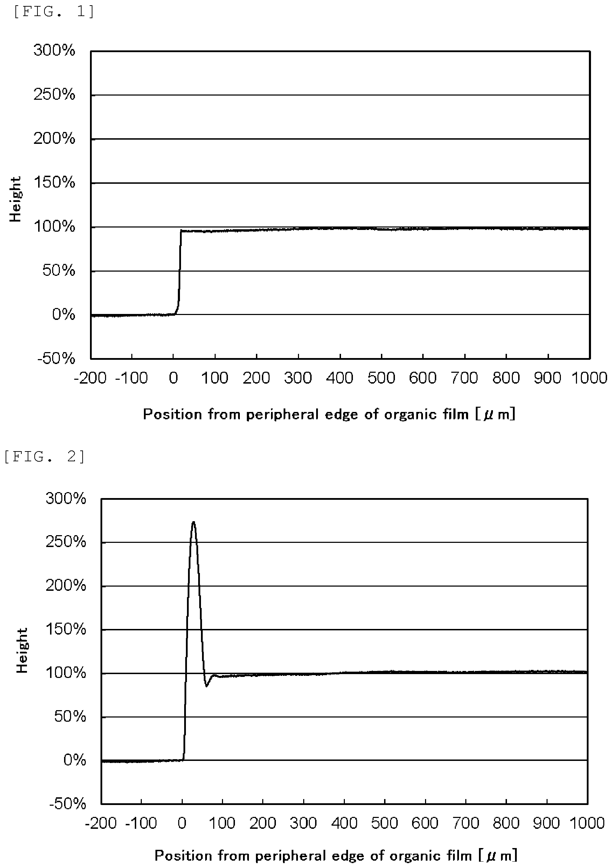

A film of each of the compositions (UDL-1 to -79, comparative UDL-1 to -10, and comparative UDL-13 and - 14) for forming an organic film was respectively formed on a silicon wafer according to the above-described method, and the variation in height from the peripheral edge of the organic film towards the center of the silicon wafer to a position of 1000 µm was measured by using Alpha-Step D-600 (a stylus profiler) manufactured by KLA corporation. The height of the silicon wafer was regarded as 0, and when the maximum height was less than 110% of the film thickness as shown in FIG. 1, the composition was evaluated as A (good), when the maximum height was 110% or more to less than 150%, B, and when a region where the height was 150% or more occurred as shown in FIG. 2, C (poor).

### [Filling Property Evaluation-1: Examples 2-1 to 2-79 and Comparative Examples 2-1 to 2-12]

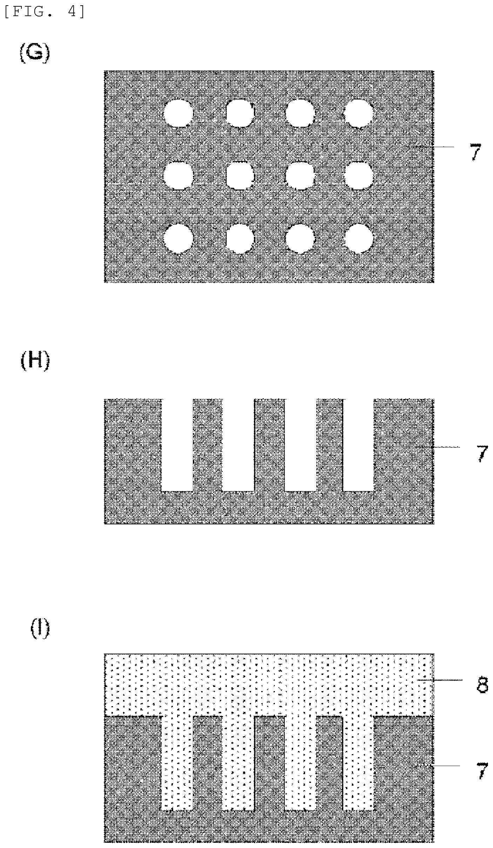

As shown in FIG. 4, a film was formed on an SiO₂ wafer substrate having a dense hole pattern (hole diameter 0.2 µm, hole depth 1.0 µm, distance between the centers of two adjacent holes 0.4 µm) by using each of the compositions (UDL-1 to -79, comparative UDL-1 to -10, and comparative UDL-13 and -14) for forming an organic film respectively according to the above-described method to form a resist underlayer film 8. The substrate used was a base substrate (SiO₂ wafer substrate) 7 having the dense hole pattern shown in FIG. 4 (G) (downward view) and (H) (cross-sectional view). The cross-sectional shape of each of the obtained wafer substrates was observed using a scanning electron microscope (SEM), and it was observed whether or not the inside of the holes was filled with the resist underlayer film without gaps. When a resist underlayer film material having poor filling property is used, gaps are generated inside the holes. When a resist underlayer film material having a desirable filling property is used in the present evaluation, the inside of the holes is filled with the resist underlayer film without gaps, as shown in FIG. 4 (I). The composition was evaluated as "Good" when no gaps were generated, and when gaps were generated, as "Poor".

### [Filling Property Evaluation-2: Examples 2-57 to 2-79]

As shown in FIG. 4, a film was formed on an SiO₂ wafer substrate having a dense hole pattern (hole diameter 0.2 µm, hole depth 2.0 µm, distance between the centers of two adjacent holes 0.4 µm) by using each of the compositions (UDL-57 to -79) for forming an organic film respectively according to the above-described method to form a resist underlayer film 8. The substrate used was a base substrate (SiO₂ wafer substrate) 7 having the dense hole pattern shown in FIG. 4 (G) (downward view) and (H) (cross-sectional view). The cross-sectional shape of each of the obtained wafer substrates was observed using a scanning electron microscope (SEM), and it was observed whether or not the inside of the holes was filled with the resist underlayer film without gaps. When a resist underlayer film material having poor filling property is used, gaps are generated inside the holes. When a resist underlayer film material having a desirable filling property is used in the present evaluation, the inside of the holes is filled with the resist underlayer film without gaps, as shown in FIG. 4 (I). The composition was evaluated as "Good" when no gaps were generated, and when gaps were generated, as "Poor". Note that, in this evaluation, to evaluate whether the filling property is excellent or not, the evaluation conditions are severe, and gaps are more likely to be generated than in the filling property evaluation-1.

### [Silicon-Containing Resist Middle Layer Film Coating Property Evaluation: Examples 2-1 to 2-79 and Comparative Examples 2-1 to 2-12]

An organic cured film was formed respectively on a silicon wafer substrate by using each of the compositions (UDL-1 to -79, comparative UDL-1 to -10, and comparative UDL-13 and -14) for forming an organic film according to the above-described method, the silicon-containing resist middle layer film material (SOG1) described below was applied thereto, and baking was performed at 200°C for 60 seconds to form a silicon-containing resist middle layer film. Then, the condition of the coating film of the silicon-containing resist middle layer film was visually observed and evaluated.

When the condition of the coating film was good, the composition was evaluated as "Good", and when dewetting had occurred, as "Poor".

Note that, in this evaluation, to evaluate whether the coatability of the silicon-containing resist middle layer film is excellent or not, the thickness of the silicon-containing resist middle layer film was set to 5 nm, and this is a special, severe evaluation condition.

The silicon-containing resist middle layer film material (SOG1) was prepared by dissolving a polymer (SP1), a crosslinking catalyst, and an acid in an organic solvent and water in the proportions shown in Table 10, and filtering the solution through a 0.1-µm filter made of a fluororesin.

**[Table 10]**

| | Polymer (parts by mass) | Crosslinking catalyst (parts by mass) | Acid (parts by mass) | Organic solvent (parts by mass) | Water (parts by mass) |
|---|---|---|---|---|---|
| SOG1 | SP1 (100) | TMPANO₃ (1) | Maleic acid (1) | PGEE (44100) | Water (4900) |

The polymer (SP1) is shown below. 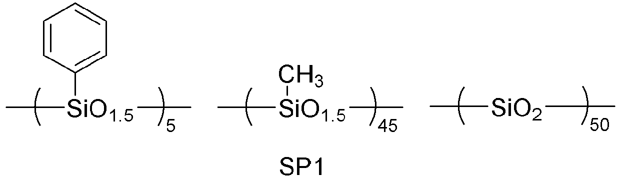
TMPANO₃: trimethylphenylammonium nitrate
PGEE: propylene glycol ethyl ether

**[Table 11]**

| | Composition for forming organic film | Hump suppression property evaluation | Filling property evaluation-1 | Filling property evaluation-2 | Silicon-containing resist middle layer film coating property evaluation |
|---|---|---|---|---|---|
| Example 2-1 | UDL-1 | B | Good | - | Good |
| Example 2-2 | UDL-2 | B | Good | - | Good |
| Example 2-3 | UDL-3 | B | Good | - | Good |
| Example 2-4 | UDL-4 | B | Good | - | Good |
| Example 2-5 | UDL-5 | A | Good | - | Good |
| Example 2-6 | UDL-6 | A | Good | - | Good |
| Example 2-7 | UDL-7 | A | Good | - | Good |
| Example 2-8 | UDL-8 | A | Good | - | Good |
| Example 2-9 | UDL-9 | A | Good | - | Good |
| Example 2-10 | UDL-10 | A | Good | - | Good |
| Example 2-11 | UDL-11 | A | Good | - | Good |
| Example 2-12 | UDL-12 | A | Good | - | Good |
| Example 2-13 | UDL-13 | A | Good | - | Good |
| Example 2-14 | UDL-14 | A | Good | - | Good |
| Example 2-15 | UDL-15 | A | Good | - | Good |
| Example 2-16 | UDL-16 | A | Good | - | Good |
| Example 2-17 | UDL-17 | A | Good | - | Good |
| Example 2-18 | UDL-18 | A | Good | - | Good |
| Example 2-19 | UDL-19 | A | Good | - | Good |
| Example 2-20 | UDL-20 | A | Good | - | Good |
| Example 2-21 | UDL-21 | A | Good | - | Good |
| Example 2-22 | UDL-22 | A | Good | - | Good |
| Example 2-23 | UDL-23 | A | Good | - | Good |
| Example 2-24 | UDL-24 | A | Good | - | Good |
| Example 2-25 | UDL-25 | A | Good | - | Good |
| Example 2-26 | UDL-26 | A | Good | - | Good |
| Example 2-27 | UDL-27 | A | Good | - | Good |
| Example 2-28 | UDL-28 | A | Good | - | Good |
| Example 2-29 | UDL-29 | A | Good | - | Good |

**[Table 12]**

| | Composition for forming organic film | Hump suppression property evaluation | Filling property evaluation-1 | Filling property evaluation-2 | Silicon-containing resist middle layer film coating property evaluation |
|---|---|---|---|---|---|
| Example 2-30 | UDL-30 | A | Good | - | Good |
| Example 2-31 | UDL-31 | A | Good | - | Good |
| Example 2-32 | UDL-32 | A | Good | - | Good |
| Example 2-33 | UDL-33 | A | Good | - | Good |
| Example 2-34 | UDL-34 | A | Good | - | Good |
| Example 2-35 | UDL-35 | A | Good | - | Good |
| Example 2-36 | UDL-36 | A | Good | - | Good |
| Example 2-37 | UDL-37 | A | Good | - | Good |
| Example 2-38 | UDL-38 | A | Good | - | Good |
| Example 2-39 | UDL-39 | A | Good | - | Good |
| Example 2-40 | UDL-40 | A | Good | - | Good |
| Example 2-41 | UDL-41 | A | Good | - | Good |
| Example 2-42 | UDL-42 | A | Good | - | Good |
| Example 2-43 | UDL-43 | A | Good | - | Good |
| Example 2-44 | UDL-44 | A | Good | - | Good |
| Example 2-45 | UDL-45 | A | Good | - | Good |
| Example 2-46 | UDL-46 | A | Good | - | Good |
| Example 2-47 | UDL-47 | A | Good | - | Good |
| Example 2-48 | UDL-48 | A | Good | - | Good |
| Example 2-49 | UDL-49 | A | Good | - | Good |
| Example 2-50 | UDL-50 | A | Good | - | Good |
| Example 2-51 | UDL-51 | A | Good | - | Good |
| Example 2-52 | UDL-52 | A | Good | - | Good |
| Example 2-53 | UDL-53 | A | Good | - | Good |
| Example 2-54 | UDL-54 | A | Good | - | Good |
| Example 2-55 | UDL-55 | A | Good | - | Good |
| Example 2-56 | UDL-56 | A | Good | - | Good |

**[Table 13]**

| | Composition for forming organic film | Hump suppression property evaluation | Filling property evaluation-1 | Filling property evaluation-2 | Silicon-containing resist middle layer film coating property evaluation |
|---|---|---|---|---|---|
| Example 2-57 | UDL-57 | B | Good | Poor | Good |
| Example 2-58 | UDL-58 | B | Good | Poor | Good |
| Example 2-59 | UDL-59 | B | Good | Poor | Good |
| Example 2-60 | UDL-60 | B | Good | Poor | Good |
| Example 2-61 | UDL-61 | A | Good | Poor | Good |
| Example 2-62 | UDL-62 | A | Good | Poor | Good |
| Example 2-63 | UDL-63 | A | Good | Poor | Good |
| Example 2-64 | UDL-64 | A | Good | Poor | Good |
| Example 2-65 | UDL-65 | A | Good | Poor | Good |
| Example 2-66 | UDL-66 | A | Good | Poor | Good |
| Example 2-67 | UDL-67 | A | Good | Poor | Good |
| Example 2-68 | UDL-68 | A | Good | Poor | Good |
| Example 2-69 | UDL-69 | A | Good | Good | Good |
| Example 2-70 | UDL-70 | A | Good | Good | Good |
| Example 2-71 | UDL-71 | A | Good | Good | Good |
| Example 2-72 | UDL-72 | A | Good | Good | Good |
| Example 2-73 | UDL-73 | A | Good | Good | Good |
| Example 2-74 | UDL-74 | A | Good | Good | Good |
| Example 2-75 | UDL-75 | A | Good | Good | Good |
| Example 2-76 | UDL-76 | A | Good | Poor | Good |
| Example 2-77 | UDL-77 | A | Good | Poor | Good |
| Example 2-78 | UDL-78 | A | Good | Poor | Good |
| Example 2-79 | UDL-79 | A | Good | Poor | Good |

**[Table 14]**

| | Composition for forming organic film | Hump suppression property evaluation | Filling property evaluation-1 | Filling property evaluation-2 | Silicon-containing resist middle layer film coating property evaluation |
|---|---|---|---|---|---|
| Comparative Example 2-1 | Comparative UDL-1 | C | Poor | - | Poor |
| Comparative Example 2-2 | Comparative UDL-2 | C | Poor | - | Poor |
| Comparative Example 2-3 | Comparative UDL-3 | C | Poor | - | Poor |
| Comparative Example 2-4 | Comparative UDL-4 | C | Poor | - | Poor |
| Comparative Example 2-5 | Comparative UDL-5 | C | Poor | - | Poor |
| Comparative Example 2-6 | Comparative UDL-6 | C | Poor | - | Poor |
| Comparative Example 2-7 | Comparative UDL-7 | C | Poor | - | Poor |
| Comparative Example 2-8 | Comparative UDL-8 | C | Poor | - | Poor |
| Comparative Example 2-9 | Comparative UDL-9 | C | Poor | - | Poor |
| Comparative Example 2-10 | Comparative UDL-10 | A | Poor | - | Poor |
| Comparative Example 2-11 | Comparative UDL-13 | C | Poor | - | Poor |
| Comparative Example 2-12 | Comparative UDL-14 | B | Poor | - | Poor |

As shown in Tables 6 to 9 and 11 to 14, it was confirmed that the inventive compositions (UDL-1 to -79) for forming an organic film were excellent in solvent resistance, in-plane uniformity, hump suppression property, filling property, and the coating property of the silicon-containing resist middle layer film. In addition, from filling property evaluation-2, it was shown that UDL-69 to -75 were better.

### [Patterning Test: Examples 3-1 to 3-56]

An organic cured film was formed respectively on an SiO₂ wafer substrate by using each of the compositions (UDL-1 to -56) for forming an organic film according to the above-described method, the silicon-containing resist middle layer film material (SOG2) described below was applied thereto, and baking was performed at 200°C for 60 seconds to form a 35-nm thick silicon-containing resist middle layer film. The monolayer resist for ArF described below was applied thereto as a resist upper layer film material and baked at 105°C for 60 seconds to form a 100-nm thick photoresist film. The liquid immersion top coat composition (TC-1) described below was applied to the photoresist film and baked at 90°C for 60 seconds to form a 50-nm thick top coat.

The silicon-containing resist middle layer film material (SOG2) was prepared by dissolving a polymer (SP1), a crosslinking catalyst, and an acid in an organic solvent and water in the proportions shown in Table 15, and filtering the solution through a 0.1-µm filter made of a fluororesin.

**[Table 15]**

| | Polymer (parts by mass) | Crosslinking catalyst (parts by mass) | Acid (parts by mass) | Organic solvent (parts by mass) | Water (parts by mass) |
|---|---|---|---|---|---|
| SOG2 | SP1 (100) | TMPANO₃ (1) | Maleic acid (1) | PGEE (4410) | Water (490) |

The resist upper layer film material (monolayer resist for ArF) was prepared by dissolving a polymer (RP1), an acid generator (PAG1), and a basic compound (Amine1), each in the proportion shown in Table 16, in a solvent containing 0.1% by mass of FC-430 (manufactured by Sumitomo 3M Limited), and filtering the solution through a 0.1-µm filter made of a fluororesin.

**[Table 16]**

| | Polymer (parts by mass) | Acid generator (parts by mass) | Basic compound (parts by mass) | Solvent (parts by mass) |
|---|---|---|---|---|
| Monolayer resist for ArF | RP1 (100) | PAG1 (6.6) | Aminel (0.8) | PGMEA (2500) |

The polymer (RP1), the acid generator (PAG1), and the basic compound (Amine1) are shown below.

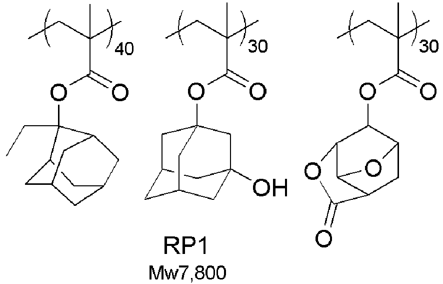

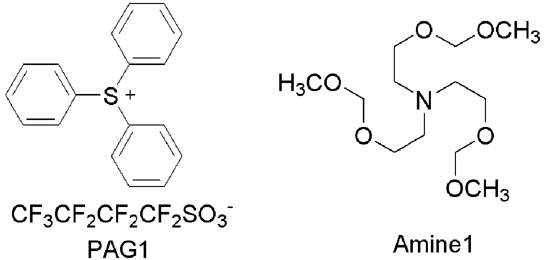

The liquid immersion top coat composition (TC-1) was prepared by dissolving a polymer (PP1) in an organic solvent at the proportion shown in Table 17, and filtering the solution through a 0.1-µm filter made of a fluororesin.

**[Table 17]**

| | Polymer (parts by mass) | Organic solvent (parts by mass) |
|---|---|---|
| TC-1 | PP1 (100) | Diisoamyl ether (2700) |
| | | 2-methyl-1-butanol (270) |

The polymer (PP1) is shown below.

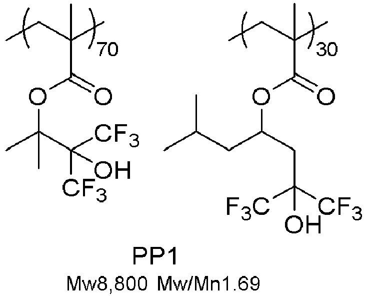

Then, the substrate was exposed to light with an ArF liquid immersion exposure apparatus (NSR-S610C manufactured by Nikon Corporation, NA: 1.30, σ: 0.98/0.65, 35° s-polarized dipole illumination, 6% halftone phase shift mask), baked at 100°C for 60 seconds (PEB), and developed with a 2.38% by mass aqueous solution of tetramethylammonium hydroxide (TMAH) for 30 seconds, thereby obtaining a 55 nm 1:1 positive line-and-space pattern (a resist upper layer film pattern) .

Subsequently, while using the resist upper layer film pattern as a mask, the silicon-containing resist middle layer film was dry-etched (the pattern was transferred) by using an etching apparatus Telius manufactured by Tokyo Electron Ltd.; the organic film was dry-etched (the pattern was transferred) while using the obtained silicon-containing resist middle layer film pattern as a mask; and the SiO₂ wafer substrate (SiO₂ film) was dry-etched (the pattern was transferred) while using the obtained organic film pattern as a mask. The etching conditions were as follows.

### Conditions in transferring resist upper layer film pattern to silicon-containing resist middle layer film

Chamber pressure: 10.0 Pa
RF-power: 1,500 W
CF₄ gas flow rate: 75 mL/min
O₂ gas flow rate: 15 mL/min
Time: 15 sec

### Conditions in transferring silicon-containing resist middle layer film pattern to organic film

Chamber pressure: 2.0 Pa
RF-power: 500 W
Ar gas flow rate: 75 mL/min
O₂ gas flow rate: 45 mL/min
Time: 120 sec

### Conditions in transferring organic film pattern to SiO₂ wafer substrate

Chamber pressure: 2.0 Pa
RF-power: 2,200 W
C₅F₁₂ gas flow rate: 20 mL/min
C₂F₆ gas flow rate: 10 mL/min
Ar gas flow rate: 300 mL/min
O₂ gas flow rate: 60 mL/min
Time: 90 sec

The cross section of the obtained pattern was observed with an electron microscope (S-4700) manufactured by Hitachi, Ltd. Tables 18 and 19 show the results.

**[Table 18]**

| | Composition for forming organic film | Profile after etching for transferring to substrate |
|---|---|---|
| Example 3-1 | UDL-1 | Good |
| Example 3-2 | UDL-2 | Good |
| Example 3-3 | UDL-3 | Good |
| Example 3-4 | UDL-4 | Good |
| Example 3-5 | UDL-5 | Good |
| Example 3-6 | UDL-6 | Good |
| Example 3-7 | UDL-7 | Good |
| Example 3-8 | UDL-8 | Good |
| Example 3-9 | UDL-9 | Good |
| Example 3-10 | UDL-10 | Good |
| Example 3-11 | UDL-11 | Good |
| Example 3-12 | UDL-12 | Good |
| Example 3-13 | UDL-13 | Good |
| Example 3-14 | UDL-14 | Good |
| Example 3-15 | UDL-15 | Good |
| Example 3-16 | UDL-16 | Good |
| Example 3-17 | UDL-17 | Good |
| Example 3-18 | UDL-18 | Good |
| Example 3-19 | UDL-19 | Good |
| Example 3-20 | UDL-20 | Good |
| Example 3-21 | UDL-21 | Good |
| Example 3-22 | UDL-22 | Good |
| Example 3-23 | UDL-23 | Good |
| Example 3-24 | UDL-24 | Good |
| Example 3-25 | UDL-25 | Good |
| Example 3-26 | UDL-26 | Good |
| Example 3-27 | UDL-27 | Good |
| Example 3-28 | UDL-28 | Good |
| Example 3-29 | UDL-29 | Good |

**[Table 19]**

| | Composition for forming organic film | Profile after etching for transferring to substrate |
|---|---|---|
| Example 3-30 | UDL-30 | Good |
| Example 3-31 | UDL-31 | Good |
| Example 3-32 | UDL-32 | Good |
| Example 3-33 | UDL-33 | Good |
| Example 3-34 | UDL-34 | Good |
| Example 3-35 | UDL-35 | Good |
| Example 3-36 | UDL-36 | Good |
| Example 3-37 | UDL-37 | Good |
| Example 3-38 | UDL-38 | Good |
| Example 3-39 | UDL-39 | Good |
| Example 3-40 | UDL-40 | Good |
| Example 3-41 | UDL-41 | Good |
| Example 3-42 | UDL-42 | Good |
| Example 3-43 | UDL-43 | Good |
| Example 3-44 | UDL-44 | Good |
| Example 3-45 | UDL-45 | Good |
| Example 3-46 | UDL-46 | Good |
| Example 3-47 | UDL-47 | Good |
| Example 3-48 | UDL-48 | Good |
| Example 3-49 | UDL-49 | Good |
| Example 3-50 | UDL-50 | Good |
| Example 3-51 | UDL-51 | Good |
| Example 3-52 | UDL-52 | Good |
| Example 3-53 | UDL-53 | Good |
| Example 3-54 | UDL-54 | Good |
| Example 3-55 | UDL-55 | Good |
| Example 3-56 | UDL-56 | Good |

As shown in Tables 18 and 19, in Examples 3-1 to 3-56, where the inventive compositions (UDL-1 to -56) for forming an organic film were used, the resist upper layer film pattern was successfully transferred to the SiO₂ wafer substrate in the end in every case. Thus, it was confirmed that the inventive compositions for forming an organic film can be used suitably for fine processing using a multilayer resist method.

### [Preparation of Resist Upper Layer Film Materials (ArFPR1 to 23 and Comparative ArFPR)]

Each of resist upper layer film materials (ArFPR1 to 23 and comparative ArFPR) were prepared by dissolving a polymer (RP1), an acid generator (PAG1), a basic compound (Amine1), and a compound in a solvent in the proportions shown in Table 20, and filtering the solution through a 0.1-µm filter made of a fluororesin.

**[Table 20]**

| Resist upper layer film material | Polymer (parts by mass) | Acid generator (parts by mass) | Basic compound (parts by mass) | Compound (parts by mass) | Solvent (parts by mass) |
|---|---|---|---|---|---|
| ArFPR1 | RP1 (100) | PAG1 (6.6) | Aminel (0.8) | (A1) (2.5) | PGMEA (2500) |
| ArFPR2 | RP1 (100) | PAG1 (6.6) | Aminel (0.8) | (A2) (2.5) | PGMEA (2500) |
| ArFPR3 | RP1 (100) | PAG1 (6.6) | Aminel (0.8) | (A3) (2.5) | PGMEA (2500) |
| ArFPR4 | RP1 (100) | PAG1 (6.6) | Aminel (0.8) | (A4) (2.5) | PGMEA (2500) |
| ArFPR5 | RP1 (100) | PAG1 (6.6) | Aminel (0.8) | (A5) (2.5) | PGMEA (2500) |
| ArFPR6 | RP1 (100) | PAG1 (6.6) | Aminel (0.8) | (A6) (2.5) | PGMEA (2500) |
| ArFPR7 | RP1 (100) | PAG1 (6.6) | Aminel (0.8) | (A7) (2.5) | PGMEA (2500) |
| ArFPR8 | RP1 (100) | PAG1 (6.6) | Aminel (0.8) | (A8) (2.5) | PGMEA (2500) |
| ArFPR9 | RP1 (100) | PAG1 (6.6) | Aminel (0.8) | (A9) (2.5) | PGMEA (2500) |
| ArFPR10 | RP1 (100) | PAG1 (6.6) | Aminel (0.8) | (A10) (2.5) | PGMEA (2500) |
| ArFPR11 | RP1 (100) | PAG1 (6.6) | Aminel (0.8) | (A11) (2.5) | PGMEA (2500) |
| ArFPR12 | RP1 (100) | PAG1 (6.6) | Aminel (0.8) | (A12) (2.5) | PGMEA (2500) |
| ArFPR13 | RP1 (100) | PAG1 (6.6) | Aminel (0.8) | (A13) (2.5) | PGMEA (2500) |
| ArFPR14 | RP1 (100) | PAG1 (6.6) | Aminel (0.8) | (A14) (2.5) | PGMEA (2500) |
| ArFPR15 | RP1 (100) | PAG1 (6.6) | Aminel (0.8) | (A15) (2.5) | PGMEA (2500) |
| ArFPR16 | RP1 (100) | PAG1 (6.6) | Aminel (0.8) | (A16) (2.5) | PGMEA (2500) |
| ArFPR17 | RP1 (100) | PAG1 (6.6) | Aminel (0.8) | (A17) (2.5) | PGMEA (2500) |
| ArFPR18 | RP1 (100) | PAG1 (6.6) | Aminel (0.8) | (A18) (2.5) | PGMEA (2500) |
| ArFPR19 | RP1 (100) | PAG1 (6.6) | Aminel (0.8) | (A19) (2.5) | PGMEA (2500) |
| ArFPR20 | RP1 (100) | PAG1 (6.6) | Aminel (0.8) | (A20) (2.5) | PGMEA (2500) |
| ArFPR21 | RP1 (100) | PAG1 (6.6) | Aminel (0.8) | (A21) (2.5) | PGMEA (2500) |
| ArFPR22 | RP1 (100) | PAG1 (6.6) | Aminel (0.8) | (A22) (2.5) | PGMEA (2500) |
| ArFPR23 | RP1 (100) | PAG1 (6.6) | Aminel (0.8) | (A23) (2.5) | PGMEA (2500) |
| Comparative ArFPR | RP1 (100) | PAG1 (6.6) | Aminel (0.8) | - | PGMEA (2500) |

### [Preparation of Silicon Wafers Having Resist Upper Layer Film Formed Thereon by Using Resist Upper Layer Film Materials (ArFPR1 to 23 and Comparative ArFPR)]

Using a coater/developer "CLEAN TRACK LITHIUS Pro AP" of Tokyo Electron Ltd., each of the resist upper layer film materials (ArFPR1 to 23 and comparative ArFPR) prepared above was respectively discharged in an amount of 2 ml onto a silicon wafer at the center. Then, the wafer was rotated at a rotational rate to achieve an average film thickness of 100 nm after baking to spread the material. Next, the silicon wafer coated with the resist upper layer film was heated at 105°C for 60 seconds. Thus, a silicon wafer having a resist upper layer film formed thereon was obtained.

### [In-Plane Uniformity Evaluation of Resist Upper Layer Film: Examples 4-1 to 4-23 and Comparative Example 4-1]

A resist upper layer film (ArFPR1 to 23 and comparative ArFPR) was respectively formed on a silicon wafer according to the above-described method, and the film thickness within a radius of 145 mm from the center of the film was measured. The value determined by (Xₘₐₓ-Xₘᵢₙ)/X_{average} was obtained as in-plane uniformity (%), where Xₘₐₓ is the maximum value of the film thickness, Xₘᵢₙ is the minimum value of the film thickness, and X_{average} is the average film thickness. When the in-plane uniformity was less than 3%, the material was evaluated as "Good", when 3% or more, "Poor".

**[Table 21]**

| | Resist upper layer film material | In-plane uniformity |
|---|---|---|
| Example 4-1 | ArFPR1 | Good |
| Example 4-2 | ArFPR2 | Good |
| Example 4-3 | ArFPR3 | Good |
| Example 4-4 | ArFPR4 | Good |
| Example 4-5 | ArFPR5 | Good |
| Example 4-6 | ArFPR6 | Good |
| Example 4-7 | ArFPR7 | Good |
| Example 4-8 | ArFPR8 | Good |
| Example 4-9 | ArFPR9 | Good |
| Example 4-10 | ArFPR10 | Good |
| Example 4-11 | ArFPR11 | Good |
| Example 4-12 | ArFPR12 | Good |
| Example 4-13 | ArFPR13 | Good |
| Example 4-14 | ArFPR14 | Good |
| Example 4-15 | ArFPR15 | Good |
| Example 4-16 | ArFPR16 | Good |
| Example 4-17 | ArFPR17 | Good |
| Example 4-18 | ArFPR18 | Good |
| Example 4-19 | ArFPR19 | Good |
| Example 4-20 | ArFPR20 | Good |
| Example 4-21 | ArFPR21 | Good |
| Example 4-22 | ArFPR22 | Good |
| Example 4-23 | ArFPR23 | Good |
| Comparative Example 4-1 | Comparative ArFPR | Poor |

As shown in Table 21, the resist upper layer film materials (ArFPR1 to 23) of the present invention is excellent in in-plane uniformity, and therefore, it can be seen that the inventive polymer functions as a surfactant that can impart excellent levelling property, and can be used for various compositions for forming an organic film regardless of the type of the resin to be combined with.

### [Preparation of Silicon-Containing Resist Middle Layer Film Materials (SOG3 to 25)]

Each of silicon-containing resist middle layer film materials (SOG3 to 25) was prepared by dissolving a polymer (SP1), a crosslinking catalyst, and an acid in an organic solvent and water in the proportions shown in Table 22, and filtering the solution through a 0.1-µm filter made of a fluororesin.

**[Table 22]**

| Silicon-containing resist middle layer film material | Polymer (parts by mass) | Crosslinking catalyst (parts by mass) | Acid (parts by mass) | Compound (parts by mass) | Organic solvent (parts by mass) | Water (parts by mass) |
|---|---|---|---|---|---|---|
| SOG3 | SP1 (100) | TMPANO₃ (1.0) | Maleic acid (1.0) | (A1) (1.0) | PGEE (44100) | Water (4900) |
| SOG4 | SP1 (100) | TMPANO₃ (1.0) | Maleic acid (1.0) | (A2) (1.0) | PGEE (44100) | Water (4900) |
| SOG5 | SP1 (100) | TMPANO₃ (1.0) | Maleic acid (1.0) | (A3) (1.0) | PGEE (44100) | Water (4900) |
| SOG6 | SP1 (100) | TMPANO₃ (1.0) | Maleic acid (1.0) | (A4) (1.0) | PGEE (44100) | Water (4900) |
| SOG7 | SP1 (100) | TMPANO₃ (1.0) | Maleic acid (1.0) | (A5) (1.0) | PGEE (44100) | Water (4900) |
| SOG8 | SP1 (100) | TMPANO₃ (1.0) | Maleic acid (1.0) | (A6) (1.0) | PGEE (44100) | Water (4900) |
| SOG9 | SP1 (100) | TMPANO₃ (1.0) | Maleic acid (1.0) | (A7) (1.0) | PGEE (44100) | Water (4900) |
| SOG10 | SP1 (100) | TMPANO₃ (1.0) | Maleic acid (1.0) | (A8) (1.0) | PGEE (44100) | Water (4900) |
| SOG11 | SP1 (100) | TMPANO₃ (1.0) | Maleic acid (1.0) | (A9) (1.0) | PGEE (44100) | Water (4900) |
| SOG12 | SP1 (100) | TMPANO₃ (1.0) | Maleic acid (1.0) | (A10) (1.0) | PGEE (44100) | Water (4900) |
| SOG13 | SP1 (100) | TMPANO₃ (1.0) | Maleic acid (1.0) | (A11) (1.0) | PGEE (44100) | Water (4900) |
| SOG14 | SP1 (100) | TMPANO₃ (1.0) | Maleic acid (1.0) | (A12) (1.0) | PGEE (44100) | Water (4900) |
| SOG15 | SP1 (100) | TMPANO₃ (1.0) | Maleic acid (1.0) | (A13) (1.0) | PGEE (44100) | Water (4900) |
| SOG16 | SP1 (100) | TMPANO₃ (1.0) | Maleic acid (1.0) | (A14) (1.0) | PGEE (44100) | Water (4900) |
| SOG17 | SP1 (100) | TMPANO₃ (1.0) | Maleic acid (1.0) | (A15) (1.0) | PGEE (44100) | Water (4900) |
| SOG18 | SP1 (100) | TMPANO₃ (1.0) | Maleic acid (1.0) | (A16) (1.0) | PGEE (44100) | Water (4900) |
| SOG19 | SP1 (100) | TMPANO₃ (1.0) | Maleic acid (1.0) | (A17) (1.0) | PGEE (44100) | Water (4900) |
| SOG20 | SP1 (100) | TMPANO₃ (1.0) | Maleic acid (1.0) | (A18) (1.0) | PGEE (44100) | Water (4900) |
| SOG21 | SP1 (100) | TMPANO₃ (1.0) | Maleic acid (1.0) | (A19) (1.0) | PGEE (44100) | Water (4900) |
| SOG22 | SP1 (100) | TMPANO₃ (1.0) | Maleic acid (1.0) | (A20) (1.0) | PGEE (44100) | Water (4900) |
| SOG23 | SP1 (100) | TMPANO₃ (1.0) | Maleic acid (1.0) | (A21) (1.0) | PGEE (44100) | Water (4900) |
| SOG24 | SP1 (100) | TMPANO₃ (1.0) | Maleic acid (1.0) | (A22) (1.0) | PGEE (44100) | Water (4900) |
| SOG25 | SP1 (100) | TMPANO₃ (1.0) | Maleic acid (1.0) | (A23) (1.0) | PGEE (44100) | Water (4900) |

### [Silicon-Containing Resist Middle Layer Film Coating Property Evaluation: Examples 5-1 to 5-23 and Comparative Example 5-1]

An organic cured film was formed respectively on a silicon wafer substrate by using the composition (comparative UDL-1) for forming an organic film according to the above-described method, each of the silicon-containing resist middle layer film materials (SOG1 and 3 to 25) described below was respectively applied thereto, and baking was performed at 200°C for 60 seconds to form a silicon-containing resist middle layer film. Then, the condition of the coating film of the silicon-containing resist middle layer film was visually observed and evaluated.

When the condition of the coating film was good, the composition was evaluated as "Good", and when dewetting had occurred, as "Poor".

Note that, in this evaluation, to evaluate whether the coatability of the silicon-containing resist middle layer film is excellent or not, the thickness of the silicon-containing resist middle layer film was set to 5 nm, and this is a special, severe evaluation condition.

**[Table 23]**

| | Silicon-containing resist middle layer film material | Silicon-containing resist middle layer film coating property evaluation |
|---|---|---|
| Example 5-1 | SOG3 | Good |
| Example 5-2 | SOG4 | Good |
| Example 5-3 | SOG5 | Good |
| Example 5-4 | SOG6 | Good |
| Example 5-5 | SOG7 | Good |
| Example 5-6 | SOG8 | Good |
| Example 5-7 | SOG9 | Good |
| Example 5-8 | SOG10 | Good |
| Example 5-9 | SOG11 | Good |
| Example 5-10 | SOG12 | Good |
| Example 5-11 | SOG13 | Good |
| Example 5-12 | SOG14 | Good |
| Example 5-13 | SOG15 | Good |
| Example 5-14 | SOG16 | Good |
| Example 5-15 | SOG17 | Good |
| Example 5-16 | SOG18 | Good |
| Example 5-17 | SOG19 | Good |
| Example 5-18 | SOG20 | Good |
| Example 5-19 | SOG21 | Good |
| Example 5-20 | SOG22 | Good |
| Example 5-21 | SOG23 | Good |
| Example 5-22 | SOG24 | Good |
| Example 5-23 | SOG25 | Good |
| Comparative Example 5-1 | SOG1 | Poor |

As shown in Table 23, regarding silicon-containing resist middle layer film materials (SOG3 to 25), dewetting did not occur, and film-formability was excellent. This shows that the inventive polymer can be applied and used as a surfactant for various compositions for forming a film for achieving high film-formability and so forth.

From the above, the inventive composition for forming an organic film has excellent film-formability, high filling property, hump suppression property, and excellent coating property of silicon-containing resist middle layer films, and therefore, is extremely useful as an organic film material used in a multilayer resist process. Furthermore, the inventive patterning processes, using the inventive composition for forming an organic film, make it possible to fill holes and trenches having very high aspect ratios without gaps, form a fine pattern with high precision, and form a hump-suppressed organic film, and therefore, make it possible to manufacture semiconductor devices and the like efficiently.

In addition, the inventive polymer exhibits high film-formability and so forth, and can be used widely as a surfactant.

The present description includes the following embodiments.
[1]: A composition for forming an organic film, comprising: a resin and/or compound (A) for forming an organic film; a polymer (B) having a repeating unit represented by the following general formula (1) and/or (2); and a solvent (C), + 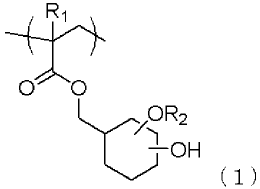 wherein R₁ represents a hydrogen atom or a methyl group, R₂ represents a saturated or unsaturated monovalent organic group having 7 to 50 carbon atoms and at least has at least one fluorine-containing structure represented by the following formula (3), and the substituents OR₂ and OH group on the cyclohexane ring are on adjacent carbon atoms on the cyclohexane ring, 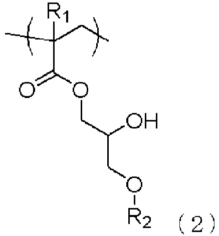 wherein R₁ and R₂ are as defined above, 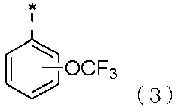 wherein (3) is a partial structure contained in the R₂, "*" represents an attachment point in a structure in the R₂, and two or more kinds of structures represented by (3) or two or more identical structures represented by (3) are optionally contained in the R₂.
[2]: The composition for forming an organic film of [1], wherein the R₂ in the general formulae (1) and (2) contains a partial structure represented by one of the following general formulae (4), 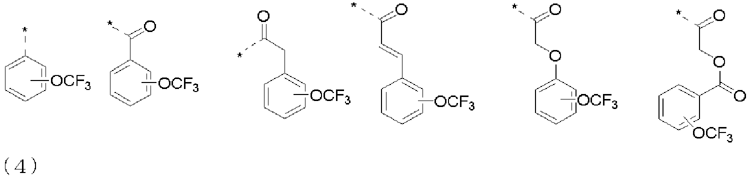 wherein "*" represents an attachment point.
[3]: The composition for forming an organic film of [1] or [2], wherein the polymer (B) is a copolymer having repeating units of one or both of the general formulae (1) and (2) and the following general formula (5), 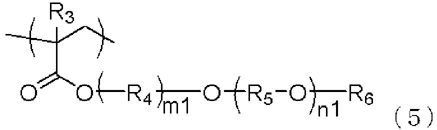 wherein R₃ represents a hydrogen atom or a methyl group; R₄ and R₅ each represent a linear or branched divalent alkylene group having 1 to 4 carbon atoms; R₆ represents a hydrogen atom, an alkyl group having 1 to 4 carbon atoms, or a phenyl group; "m1" represents 0 to 23; "n1" represents 0 to 23; and "m1" and "n1" satisfy 2 ≤ m1+n1 ≤ 23.
[4]: The composition for forming an organic film of any one of [1] to [3], wherein the polymer (B) has a weight-average molecular weight of 1500 to 30000.
[5]: The composition for forming an organic film of any one of [1] to [4], wherein the polymer (B) is contained in an amount of 0.01 parts by mass to 5 parts by mass based on 100 parts by mass of the resin and/or compound (A) for forming an organic film.
[6]: A method for forming an organic film to be used in a manufacturing process of a semiconductor device, the method comprising:
   spin-coating a substrate to be processed with the composition for forming an organic film of any one of [1] to [5]; and
   forming an organic film by heating the substrate to be processed coated with the composition for forming an organic film at a temperature of 100°C or higher and 600°C or lower for 10 to 600 seconds for curing.
[7]: A patterning process comprising:
   forming an organic film on a body to be processed by using the composition for forming an organic film of any one of [1] to [5];
   forming a silicon-containing resist middle layer film on the organic film by using a silicon-containing resist middle layer film material;
   forming a resist upper layer film on the silicon-containing resist middle layer film by using a resist upper layer film material comprising a photoresist composition;
   forming a circuit pattern in the resist upper layer film;
   transferring the pattern to the silicon-containing resist middle layer film by etching while using the resist upper layer film having the formed circuit pattern as a mask;
   transferring the pattern to the organic film by etching while using the silicon-containing resist middle layer film having the transferred pattern as a mask; and
   further transferring the pattern to the body to be processed by etching while using the organic film having the transferred pattern as a mask.
[8]: A patterning process comprising:
   forming an organic film on a body to be processed by using the composition for forming an organic film of any one of [1] to [5];
   forming a silicon-containing resist middle layer film on the organic film by using a silicon-containing resist middle layer film material;
   forming an organic antireflective film or an adhesive film on the silicon-containing resist middle layer film;
   forming a resist upper layer film on the organic antireflective film or the adhesive film by using a resist upper layer film material comprising a photoresist composition;
   forming a circuit pattern in the resist upper layer film;
   transferring the pattern to the organic antireflective film or the adhesive film and to the silicon-containing resist middle layer film by etching while using the resist upper layer film having the formed circuit pattern as a mask;
   transferring the pattern to the organic film by etching while using the silicon-containing resist middle layer film having the transferred pattern as a mask; and
   further transferring the pattern to the body to be processed by etching while using the organic film having the transferred pattern as a mask.
[9]: A patterning process comprising:
   forming an organic film on a body to be processed by using the composition for forming an organic film of any one of [1] to [5];
   forming an inorganic hard mask middle layer film selected from the group consisting of a silicon oxide film, a silicon nitride film, and a silicon oxynitride film on the organic film;
   forming a resist upper layer film on the inorganic hard mask middle layer film by using a resist upper layer film material comprising a photoresist composition;
   forming a circuit pattern in the resist upper layer film;
   transferring the pattern to the inorganic hard mask middle layer film by etching while using the resist upper layer film having the formed circuit pattern as a mask;
   transferring the pattern to the organic film by etching while using the inorganic hard mask middle layer film having the transferred pattern as a mask; and
   further transferring the pattern to the body to be processed by etching while using the organic film having the transferred pattern as a mask.
[10]: A patterning process comprising:
   forming an organic film on a body to be processed by using the composition for forming an organic film of any one of [1] to [5];
   forming an inorganic hard mask middle layer film selected from the group consisting of a silicon oxide film, a silicon nitride film, and a silicon oxynitride film on the organic film;
   forming an organic antireflective film or an adhesive film on the inorganic hard mask middle layer film;
   forming a resist upper layer film on the organic antireflective film or the adhesive film by using a resist upper layer film material comprising a photoresist composition;
   forming a circuit pattern in the resist upper layer film;
   transferring the pattern to the organic antireflective film or the adhesive film and to the inorganic hard mask middle layer film by etching while using the resist upper layer film having the formed circuit pattern as a mask;
   transferring the pattern to the organic film by etching while using the inorganic hard mask middle layer film having the transferred pattern as a mask; and
   further transferring the pattern to the body to be processed by etching while using the organic film having the transferred pattern as a mask.
[11]: The patterning process of [9] or [10], wherein the inorganic hard mask middle layer film is formed by a CVD method or an ALD method.
[12]: The patterning process of any one of [7] to [10], wherein the circuit pattern is formed by a lithography using light having a wavelength of 10 nm or more and 300 nm or less, a direct writing with electron beam, nanoimprinting, or a combination thereof.
[13]: The patterning process of any one of [7] to [10], wherein the circuit pattern is developed with an alkaline development or an organic solvent.
[14]: The patterning process of any one of [7] to [10], wherein the body to be processed is a semiconductor device substrate or the semiconductor device substrate coated with any of a metal film, a metal carbide film, a metal oxide film, a metal nitride film, a metal oxycarbide film, and a metal oxynitride film.
[15]: The patterning process of [14], wherein the metal constituting the body to be processed is silicon, titanium, tungsten, hafnium, zirconium, chromium, germanium, copper, silver, gold, aluminum, indium, gallium, arsenic, palladium, iron, tantalum, iridium, molybdenum, or an alloy thereof.
[16]: A monomer represented by the following general formula (6) or (7), 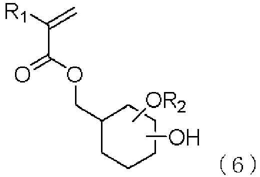 wherein R₁ represents a hydrogen atom or a methyl group, R₂ represents a saturated or unsaturated monovalent organic group having 7 to 50 carbon atoms and at least has at least one fluorine-containing structure represented by the following formula (3), and the substituents OR₂ and OH group on the cyclohexane ring are on adjacent carbon atoms on the cyclohexane ring, 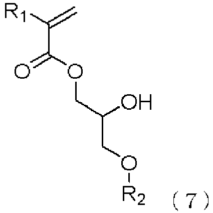 wherein R₁ and R₂ are as defined above, 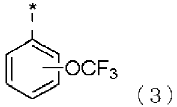 wherein (3) is a partial structure contained in the R₂, "*" represents an attachment point in a structure in the R₂, and two or more kinds of structures represented by (3) or two or more identical structures represented by (3) are optionally contained in the R₂.
[17]: The monomer of [16], wherein the R₂ in the general formulae (6) and (7) contains a partial structure represented by one of the following general formulae (4), 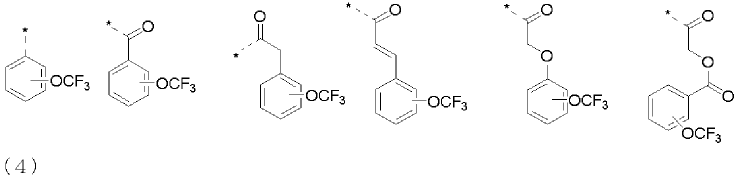 wherein "*" represents an attachment point.
[18]: A polymer having a repeating unit represented by the following general formula (1) and/or (2), 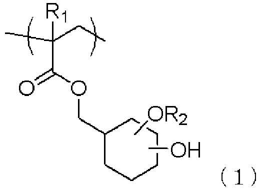 wherein R₁ represents a hydrogen atom or a methyl group, R₂ represents a saturated or unsaturated monovalent organic group having 7 to 50 carbon atoms and at least has at least one fluorine-containing structure represented by the following formula (3), and the substituents OR₂ and OH group on the cyclohexane ring are on adjacent carbon atoms on the cyclohexane ring, 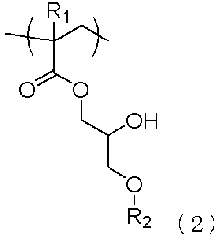 wherein R₁ and R₂ are as defined above, 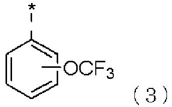 wherein (3) is a partial structure contained in the R₂, "*" represents an attachment point in a structure in the R₂, and two or more kinds of structures represented by (3) or two or more identical structures represented by (3) are optionally contained in the R₂.
[19]: The polymer of [18], wherein the R₂ in the general formulae (1) and (2) contains a partial structure represented by one of the following general formulae (4), 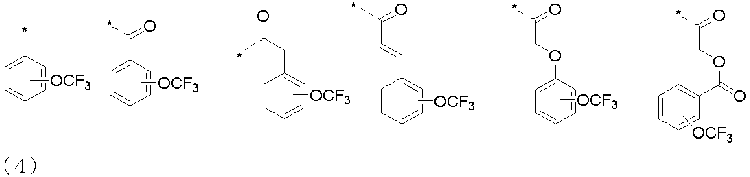 wherein "*" represents an attachment point.
[20]: The polymer of [18] or [19], being a copolymer having repeating units of one or both of the general formulae (1) and (2) and the following general formula (5), 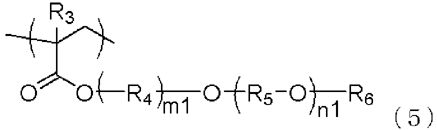 wherein R₃ represents a hydrogen atom or a methyl group; R₄ and R₅ each represent a linear or branched divalent alkylene group having 1 to 4 carbon atoms; R₆ represents a hydrogen atom, an alkyl group having 1 to 4 carbon atoms, or a phenyl group; "m1" represents 0 to 23; "n1" represents 0 to 23; and "m1" and "n1" satisfy 2 ≤ m1+n1 ≤ 23.
[21]: The polymer of any one of [18] to [20], having a weight-average molecular weight of 1500 to 30000.

It should be noted that the present invention is not limited to the above-described embodiments. The embodiments are just examples, and any examples that have substantially the same feature and demonstrate the same functions and effects as those in the technical concept disclosed in claims of the present invention are included in the technical scope of the present invention.

## Claims

1. A composition for forming an organic film, comprising: a resin and/or compound (A) for forming an organic film; a polymer (B) having a repeating unit represented by the following general formula (1) and/or (2); and a solvent (C), 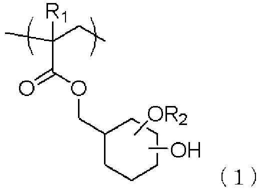 wherein R₁ represents a hydrogen atom or a methyl group, R₂ represents a saturated or unsaturated monovalent organic group having 7 to 50 carbon atoms and at least has at least one fluorine-containing structure represented by the following formula (3), and the substituents OR₂ and OH group on the cyclohexane ring are on adjacent carbon atoms on the cyclohexane ring, 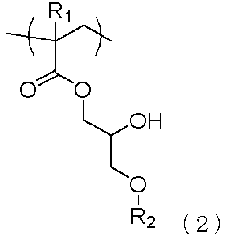 wherein R₁ and R₂ are as defined above, 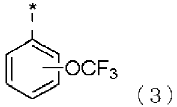 wherein (3) is a partial structure contained in the R₂, "*" represents an attachment point in a structure in the R₂, and two or more kinds of structures represented by (3) or two or more identical structures represented by (3) are optionally contained in the R₂.

2. The composition for forming an organic film according to claim 1, wherein the R₂ in the general formulae (1) and (2) contains a partial structure represented by one of the following general formulae (4), 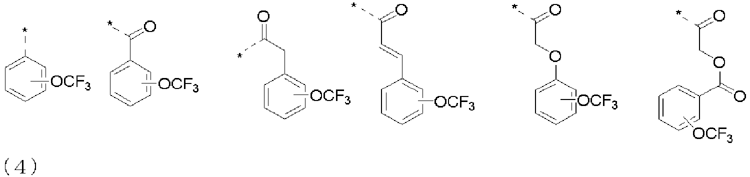
wherein "*" represents an attachment point; and/or
wherein the polymer (B) is a copolymer having repeating units of one or both of the general formulae (1) and (2) and the following general formula (5), 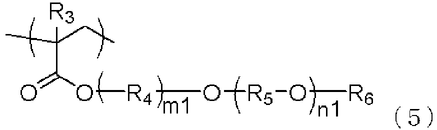
wherein R₃ represents a hydrogen atom or a methyl group;
R₄ and R₅ each represent a linear or branched divalent alkylene group having 1 to 4 carbon atoms; R₆ represents a hydrogen atom, an alkyl group having 1 to 4 carbon atoms, or a phenyl group; "m1" represents 0 to 23; "n1" represents 0 to 23; and "m1" and "n1" satisfy 2 ≤ m1+n1 ≤ 23; and/or
wherein the polymer (B) has a weight-average molecular weight of 1500 to 30000.

3. The composition for forming an organic film according to claim 1 or 2, wherein the polymer (B) is contained in an amount of 0.01 parts by mass to 5 parts by mass based on 100 parts by mass of the resin and/or compound (A) for forming an organic film.

4. A method for forming an organic film to be used in a manufacturing process of a semiconductor device, the method comprising:
spin-coating a substrate to be processed with the composition for forming an organic film according to any one of claims 1 to 3; and
forming an organic film by heating the substrate to be processed coated with the composition for forming an organic film at a temperature of 100°C or higher and 600°C or lower for 10 to 600 seconds for curing.

5. A patterning process comprising:
forming an organic film on a body to be processed by using the composition for forming an organic film according to any one of claims 1 to 3;
forming a silicon-containing resist middle layer film on the organic film by using a silicon-containing resist middle layer film material;
forming a resist upper layer film on the silicon-containing resist middle layer film by using a resist upper layer film material comprising a photoresist composition;
forming a circuit pattern in the resist upper layer film;
transferring the pattern to the silicon-containing resist middle layer film by etching while using the resist upper layer film having the formed circuit pattern as a mask;
transferring the pattern to the organic film by etching while using the silicon-containing resist middle layer film having the transferred pattern as a mask; and
further transferring the pattern to the body to be processed by etching while using the organic film having the transferred pattern as a mask.

6. A patterning process comprising:
forming an organic film on a body to be processed by using the composition for forming an organic film according to any one of claims 1 to 3;
forming a silicon-containing resist middle layer film on the organic film by using a silicon-containing resist middle layer film material;
forming an organic antireflective film or an adhesive film on the silicon-containing resist middle layer film;
forming a resist upper layer film on the organic antireflective film or the adhesive film by using a resist upper layer film material comprising a photoresist composition;
forming a circuit pattern in the resist upper layer film;
transferring the pattern to the organic antireflective film or the adhesive film and to the silicon-containing resist middle layer film by etching while using the resist upper layer film having the formed circuit pattern as a mask;
transferring the pattern to the organic film by etching while using the silicon-containing resist middle layer film having the transferred pattern as a mask; and
further transferring the pattern to the body to be processed by etching while using the organic film having the transferred pattern as a mask.

7. A patterning process comprising:
forming an organic film on a body to be processed by using the composition for forming an organic film according to any one of claims 1 to 3;
forming an inorganic hard mask middle layer film selected from the group consisting of a silicon oxide film, a silicon nitride film, and a silicon oxynitride film on the organic film;
forming a resist upper layer film on the inorganic hard mask middle layer film by using a resist upper layer film material comprising a photoresist composition;
forming a circuit pattern in the resist upper layer film;
transferring the pattern to the inorganic hard mask middle layer film by etching while using the resist upper layer film having the formed circuit pattern as a mask;
transferring the pattern to the organic film by etching while using the inorganic hard mask middle layer film having the transferred pattern as a mask; and
further transferring the pattern to the body to be processed by etching while using the organic film having the transferred pattern as a mask.

8. A patterning process comprising:
forming an organic film on a body to be processed by using the composition for forming an organic film according to any one of claims 1 to 3;
forming an inorganic hard mask middle layer film selected from the group consisting of a silicon oxide film, a silicon nitride film, and a silicon oxynitride film on the organic film;
forming an organic antireflective film or an adhesive film on the inorganic hard mask middle layer film;
forming a resist upper layer film on the organic antireflective film or the adhesive film by using a resist upper layer film material comprising a photoresist composition;
forming a circuit pattern in the resist upper layer film;
transferring the pattern to the organic antireflective film or the adhesive film and to the inorganic hard mask middle layer film by etching while using the resist upper layer film having the formed circuit pattern as a mask;
transferring the pattern to the organic film by etching while using the inorganic hard mask middle layer film having the transferred pattern as a mask; and
further transferring the pattern to the body to be processed by etching while using the organic film having the transferred pattern as a mask.

9. The patterning process according to claim 7 or 8, wherein the inorganic hard mask middle layer film is formed by a CVD method or an ALD method.

10. The patterning process according to any one of claims 5 to 8, wherein the circuit pattern is formed by a lithography using light having a wavelength of 10 nm or more and 300 nm or less, a direct writing with electron beam, nanoimprinting, or a combination thereof; or
wherein the circuit pattern is developed with an alkaline development or an organic solvent; orwherein the body to be processed is a semiconductor device substrate or the semiconductor device substrate coated with any of a metal film, a metal carbide film, a metal oxide film, a metal nitride film, a metal oxycarbide film, and a metal oxynitride film, wherein, as an option, the metal constituting the body to be processed is silicon, titanium, tungsten, hafnium, zirconium, chromium, germanium, copper, silver, gold, aluminum, indium, gallium, arsenic, palladium, iron, tantalum, iridium, molybdenum, or an alloy thereof.

11. A monomer represented by the following general formula (6) or (7), 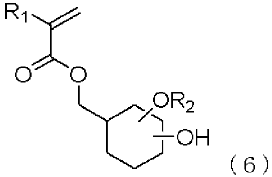
wherein R₁ represents a hydrogen atom or a methyl group,
R₂ represents a saturated or unsaturated monovalent organic group having 7 to 50 carbon atoms and at least has at least one fluorine-containing structure represented by the following formula (3), and the substituents OR₂ and OH group on the cyclohexane ring are on adjacent carbon atoms on the cyclohexane ring, 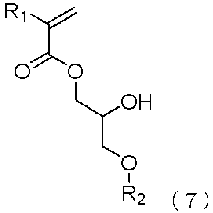
wherein R₁ and R₂ are as defined above, 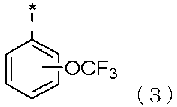
wherein (3) is a partial structure contained in the R₂, "*" represents an attachment point in a structure in the R₂, and two or more kinds of structures represented by (3) or two or more identical structures represented by (3) are optionally contained in the R₂.

12. The monomer according to claim 11, wherein the R₂ in the general formulae (6) and (7) contains a partial structure represented by one of the following general formulae (4), 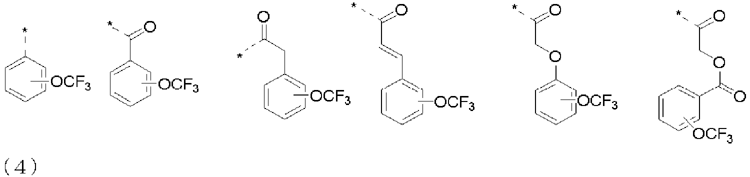 wherein "*" represents an attachment point.

13. A polymer having a repeating unit represented by the following general formula (1) and/or (2), 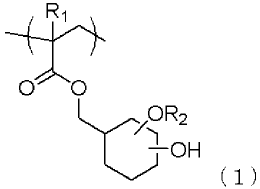 wherein R₁ represents a hydrogen atom or a methyl group, R₂ represents a saturated or unsaturated monovalent organic group having 7 to 50 carbon atoms and at least has at least one fluorine-containing structure represented by the following formula (3), and the substituents OR₂ and OH group on the cyclohexane ring are on adjacent carbon atoms on the cyclohexane ring, 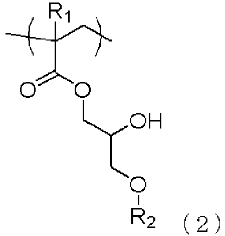 wherein R₁ and R₂ are as defined above, 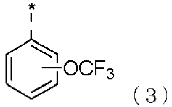 wherein (3) is a partial structure contained in the R₂, "*" represents an attachment point in a structure in the R₂, and two or more kinds of structures represented by (3) or two or more identical structures represented by (3) are optionally contained in the R₂.

14. The polymer according to claim 13, wherein the R₂ in the general formulae (1) and (2) contains a partial structure represented by one of the following general formulae (4), 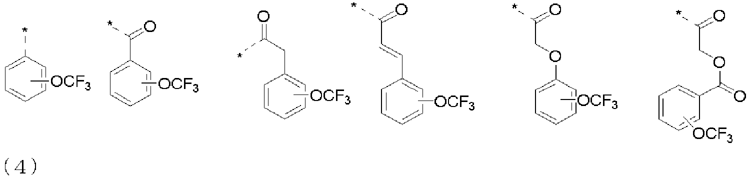
wherein "*" represents an attachment point; and/or
wherein the polymer is a copolymer having repeating units of one or both of the general formulae (1) and (2) and the following general formula (5), 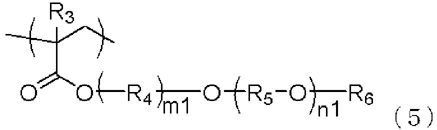
wherein R₃ represents a hydrogen atom or a methyl group;
R₄ and R₅ each represent a linear or branched divalent alkylene group having 1 to 4 carbon atoms; R₆ represents a hydrogen atom, an alkyl group having 1 to 4 carbon atoms, or a phenyl group; "m1" represents 0 to 23; "n1" represents 0 to 23; and "m1" and "n1" satisfy 2 ≤ m1+n1 ≤ 23.

15. The polymer according to claim 13 or 14, having a weight-average molecular weight of 1500 to 30000.
